(19) Europäisches Patentamt
European Patent Office
Office européen des brevets

(11) EP 2 080 768 A1

(12) EUROPEAN PATENT APPLICATION

(43) Date of publication:
**22.07.2009 Bulletin 2009/30**

(51) Int Cl.:
***C07K 14/11*** *(2006.01)* ***C12N 15/62*** *(2006.01)*
***G01N 33/542*** *(2006.01)* ***G01N 33/92*** *(2006.01)*

(21) Application number: **08001073.9**

(22) Date of filing: **21.01.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicants:
- **Freie Universität Berlin**
  **14195 Berlin (DE)**
- **Humboldt Universität zu Berlin**
  **10099 Berlin (DE)**

(72) Inventors:
- **Veit, Michael, Dr.**
  **10627 Berlin (DE)**
- **Herrmann, Andreas, Prof. Dr.**
  **13156 Berlin (DE)**
- **Thaa, Bastian**
  **10119 Berlin (DE)**
- **Engel, Stephanie**
  **10555 Berlin (DE)**
- **Scolari, Silvia**
  **10405 Berlin (DE)**

(74) Representative: **Engelhard, Markus**
**Forrester & Boehmert**
**Pettenkoferstrasse 20-22**
**80336 München (DE)**

# (54) Method for studying virus particle release

(57) The present invention refers to methods and means for studying influenza virus particle release, in particular matrix protein M1 association with the cellular membrane. For this purpose, a matrix protein M1 mutant comprising a nuclear export signal of Rev protein or a myristoylation signal of Lyn kinase, and further comprising a fluorescent protein is provided, as well as nucleic acids related thereto.

EP 2 080 768 A1

## Description

The present invention refers to methods and means for studying influenza virus particle release. In particular, the present invention refers to matrix protein M1 and its association with the cellular membrane. More particularly, the present invention refers to matrix protein M1 mutants comprising a fluorescent protein, and to nucleic acids related thereto. Furthermore, the present invention refers to hemagglutinin HA and its association with lipid-rafts. More particularly, the present invention refers to HA mutants and raft-marker mutants comprising a fluorescent protein, and to nucleic acids related thereto.

## State of the art

Influenza, commonly known as flu, is an infectious disease of birds and mammals caused by RNA viruses of the family *Orthomyxoviridae* (the influenza viruses) characterized by the presence of an envelope and a single-strand genome. In humans, common symptoms of influenza are fever, sore throat, muscle pains, severe headache, coughing, weakness and general discomfort. In more serious cases, influenza causes pneumonia, which can be fatal, particularly in young children and the elderly.

To date, medicaments for the treatment of infections by influenza viruses are limited. Relenza® (GlaxoSmith-Kline) and Tamiflu® (Roche) both target the influenza virus membrane protein neuraminidase (NA). They interfere with the release of newly formed virus particles from infected cells by occupying the active site of the enzyme, thus blocking the enzymatic activity. Although the currently available medicaments show that therapeutic intervention in the release of influenza viruses in principle works, their effectiveness is limited. Moreover, the formation of resistant virus mutants is expected to become a more and more serious problem in the future. Therefore, there is a need for novel medicaments effective in the treatment of infections by influenza viruses, and, in particular, of active agents interfering with virus particle release targeting viral membrane proteins other than NA. However, a basic requirement for the development of such active agents is a better understanding of virus particle release.

Virus particle release is preceded by the assembly and budding of the enveloped viruses. This process requires local enrichment of viral components at the budding site of the host membranes. Two non-excluding mechanisms can account for this: inclusion of membrane proteins into and association of other viral components with subdomains of the plasma membrane (lipid-rafts), and specific interactions between viral proteins. The lipid-raft concept for virus budding is based on two observations: (i) membrane proteins of many viruses are present in rafts and (ii) disintegration of rafts inhibits assembly of virus particles.

Lipid-rafts are dynamic assemblies of cholesterol and sphingolipids that are present in the exoplasmic leaflet of cellular membranes. They are somehow connected to domains of unknown composition in the inner leaflet. Lipid-rafts can selectively incorporate proteins, most notably GPI-anchored proteins into the outer leaflet, double acylated proteins into the inner leaflet and several transmembrane proteins. Thus, rafts concentrate certain membrane proteins, which should favour interactions between the raft components. According to this concept rafts have been proposed to play a role in a multitude of cellular processes, such as numerous signal transduction pathways and vesicular trafficking reactions. Rafts are also considered as point of entry as well as assembly and budding of a wide range of viruses (Simons and Ikonen, 1997; Edidin, 2003; Mayor and Rao, 2004).

One of the first classes of proteins shown to be targeted to lipid-rafts contains a glycosylphospatidylinositol (GPI) moiety, which anchors the protein to the outer leaflet of the membrane bilayer. Many of the peripheral proteins in detergent-resistant membranes (DRMs), the biochemical correlate of lipid-rafts, are dually acylated, either with a myristate and a palmitate or with two palmitates. Some transmembrane proteins also require palmitoylation at cytoplasmic cysteine residues as well as hydrophobic residues in the exoplasmic leaflet of the transmembrane region (TMR) for inclusion into DRMs. The length of the transmembrane segment may also influence partitioning of proteins into rafts, probably because cholesterol-containing rafts are thicker and thus exclude proteins with a short transmembrane segment. Other proteinaceous raft components might not possess an intrinsic raft-localisation signal, but partition into rafts through a protein-protein interaction with an original raft component. The "lipid-shell" hypothesis for targeting of proteins to rafts postulates that raft-proteins have an intrinsic affinity for cholesterol and/or sphingolipids. This should lead to envelopment of each protein molecule by four to five lipid-layers corresponding to approximately 80 molecules. These "lipid-shells" have an affinity for preexisting rafts and thus target the proteins they encase specifically to these membrane domains (Anderson and Jacobson, 2002). Indeed, recent data suggest that apart from lipid-lipid interaction, lipid domain formation can be also driven by protein-lipid interaction. Proteins which preferentially sequester into cholesterol-rich domains will stabilize those domains and, thus, may alter the cholesterol distribution in the membrane. In contrast, cholesterol-rich domains can also be formed by unfavourable interactions between proteins and cholesterol (for a recent review see Epand, 2004). In summary, it appears that a multitude of signals, such as hydrophobic protein modifications, protein-based signals and lipid-shells, work in concert to target proteins to rafts.

In addition to NA, well-characterized membrane proteins of influenza virus are hemagglutinin (HA) and the matrix protein (M1) of influenza virus. Based on Triton-extraction experiments NA and HA are constituents of lipid-rafts, whereas M1 binds to rafts only when either NA or HA are present.

The HA of influenza virus was one of the first transmembrane proteins described as a component of DRMs (Fiedler *et al.* 1993) and its putative raft association has subsequently been characterized. Hydrophobic amino acids in the outer leaflet of the transmembrane region (TMR) and palmitoylation at cytoplasmic and transmembrane cysteine residues are required for partition of HA into DRMs (Melkonian *et al.* 1999; Scheiffele *et al.*, 1997). HA acquires detergent resistance at a late stage during its transport to the cell surface, probably in the trans-Golgi-network (TGN, Skibens *et al.*, 1989). It was postulated that cholesterol-sphingolipid clusters form vesicles in the TGN, which serve as carriers for lipids and entrapped proteins to the apical membrane in epithelial cells (Simons and van Meer, 1988). This model suggests that association with DRMs is a prerequisite for apical transport of HA. Indeed, lowering cholesterol levels blocked transport of HA from the TGN to the cell surface (Keller and Simons, 1998). Although molecular signals for apical transport and DRM-association of HA are both located in the TMR, several mutations have been described, which block association with DRMs, but not apical transport (Tall *et al.,* 2003). Similar results have been obtained for the neuraminidase (NA), the second glycoprotein of influenza virus, which is also DRM-associated and apically transported (Barman *et al.* 2001). It is not known whether the hydrophobic amino acids in the TMR required for DRM-association interact with proteinaceous or lipidic components of rafts. A specific affinity for raft-lipids has only been investigated for the ion-channel protein M2, which binds to cholesterol, but is, however, not associated with DRMs (Zhang *et al.*, 2000, Schroeder *et al.*, 2004).

Rafts are supposed to concentrate proteins to facilitate stimulus-induced interactions between its components. It was shown with electron microscopy that raft-HA is distributed in clusters of 200-280 nm diameter at the surface of virus-infected cells, whereas non-raft HA is distributed randomly (Takeda *et al.* 2003). The large size of the HA-clusters suggests that they were formed by fusion of individual rafts. Interestingly, M2, which does not partition into DRMs, is present in only minute amounts in virus particles, although it is abundantly expressed at the cell surface (Zhang *et al.*, 2000). This suggests that budding of influenza virus occurs primarily at rafts. Furthermore, influenza virus particles contain high amounts of detergent-insoluble lipids, whereas such complexes are absent from viruses (SFV, VSV) which do not utilize rafts for budding (Scheiffle *et al.*, 1999).

The matrix protein M1 forms a proteinaceous layer underneath the envelope in virus particles and is thought to bind to both viral glycoproteins and to ribonucleotide particles (RNP) containing the nucleoprotein NP and the viral genome. The matrix protein is also known to oligomerize, a property which is thought to drive the budding reaction (Zhao *et al.*, 1998). Indeed, overexpression of M1 leads to the formation of virus-like particles, which can incorporate viral glycoproteins when they are coexpressed (Gomez-Puertas *et al.*, 2000; Latham *et al.*, 2001).

The crystal structure of the N-terminal fragment (residues 2 to 158) of the 252-residue M1 protein of influenza virus has been solved (Sha and Luo, 1997). It is folded into two domains, each consisting of four alpha-helices. The N-terminal domain contains a region with potential lipid binding properties and the middle-domain possesses a positively charged region that might interact with the viral RNP. M1 is membrane-bound when expressed from a plasmid, but associates with DRMs when either HA or NA are coexpressed (Ali *et al.*, 2000). Binding of M1 to DRMs is probably mediated by the cytoplasmic tails of HA and/or NA. The expression of tail-less glycoproteins using reverse genetics decreases the amount of M1 binding to DRMs. Viruses containing either tailless HA or NA have a slightly lower budding efficiency and infectivity and aberrant particle shape, but this effect is much more prominent when the cytoplasmic tails are deleted from both glycoproteins. Thus, the cytoplasmic tail signals are dually redundant (Jin *et al.*, 1997).

The described data were combined for the following model of assembly and budding of influenza virus: The two glycoproteins of the virus, HA and NA, accumulate in rafts of the plasma membrane. Binding of the matrix protein M1 to the cytoplasmic tails of the glycoproteins attaches the protein to rafts. The M1 protein then polymerizes, which causes coalescence of rafts. Most cellular raft proteins are excluded from rafts because they do not interact with M1 and are sterically hindered from entering the growing viral envelope. Binding of ribonucleotide particles (containing the viral genome) to the M1-protein induces a curvature of the membrane that will favour the budding process (Briggs *et al.*, 2003, Schmitt and Lamb, 2004; Simons and Vaz, 2004).

Another requirement for the development of active agents targeting influenza virus membrane proteins involved in the particle release are appropriate methods for study.

Detergent extraction is the most popular approach to identify raft components. The procedure involves treatment of intact cells on ice with the detergent Triton-X-100 and subsequent gradient centrifugation. Proteins which are resistant to extraction and float to a low density during centrifugation (and thus behave like cholesterol and sphingolipids) are considered as raft components (Simons and Ikonen, 1997).

Although probably hundreds of proteins have been localized to rafts with this method, it was questioned repeatedly whether partitioning of membrane components into these detergent-resistant membranes (DRMs) reflects their association with rafts inside living cells (e.g. Munro, 2003). Components might be enriched in DRMs simply because they possess common biophysical properties, but this does not necessarily provide evidence that they are clustered inside cells. Furthermore, partition of proteins into detergent-soluble and -insoluble fractions is seldom absolute, sometimes only very few percent of a protein population is present in DRMs. Recent studies with model membranes have shown that Triton-X-100 can even induce subdomain formation in a previously homogeneous membrane. On the other hand, not all proteins that are in rafts are resistant to extraction with nonionic detergents. Hence, "DRMs may differ from rafts in size, composition, structure, and even in existence" (Heerklotz, 2002).

Another approach used to probe raft involvement of a physiological process is the manipulation of cholesterol or sphingolipid levels thereby dissolving the rafts in the membrane. Cholesterol and sphingolipids are typically depleted by inhibitors of their synthesis, and cholesterol can be extracted with cyclodextrins. However, both lipids have functions in the plasma membrane apart from forming lipid rafts and therefore any functional effect caused by their depletion does not necessarily indicate a raft involvement (Munro, 2003).

If molecules are present in rafts, they are not randomly distributed over the whole membrane, but should cluster in certain subdomains of the membrane. Fluorescence microscopy in living cells has failed to reveal laterally segregated structures enriched in major raft components. Thus, preexisting cellular rafts must be smaller than the resolution of the fluorescence microscope (<300 nm) and methods with a higher resolution, such as fluorescence resonance energy transfer (FRET), must be applied. FRET is exceptionally well suited to demonstrate interactions between proteins (or other molecules of interest), because it occurs only if two molecules tagged with a fluorophore are in close proximity (2-6 nm). FRET can be performed in two ways, hetero-FRET and homo-FRET. Hetero-FRET relies on a transfer of energy from a donor to an acceptor fluorophore. For FRET to occur there must be a substantial overlap between the donor's emission spectrum and the acceptor's excitation spectrum. A suitable FRET-pair are cyan and yellow fluorescence (CFP and YFP, respectively), spectral variants of the green fluorescence protein (GFP), which can be attached by genetechnological means to proteins of interest. FRET can be measured as an increase in the acceptor's emission intensity, as an increase in the donor's fluorescence after photobleaching of the acceptor or as reduced lifetime of the excited state of the donor. Fluorescence lifetime imaging microscopy (FLIM) is independent of the concentrations of the fluorescent proteins (and hence their expression levels), but requires sophisticated instrumentation.

Homo-FRET occurs between identical fluorophores and can be measured as a loss of anisotropy (polarization) of the fluorescence emission. The basic idea is that a fluorophore excited by polarized light will also emit polarized light, the emission shows ansiotropy. When energy transfer between molecules occurs it will tend to eliminate the polarization of the light by radiating at different directions from the incident light. The amount of depolarization is calculated as decay in anisotropy. Besides FRET the mobility of the fluorophore also effects the polarized emission. The decay in anisotropy is greatest with fluorophores freely mobile in solution and decreases with decreased rates of mobility, for example by binding to another molecule (Sekar and Periasamy, 2003).

With a hetero-FRET approach Zacharias *et al.* (2002) could demonstrate clustering of proteins in membranes. YFP and CFP were fused to peptide-sequences directing hydrophobic modifications, either dual acylation or geranylgeranylation, which anchor the peptides to the inner leaflet of the membrane. FRET was observed between dually acylated peptides and between prenylated proteins, but not between prenylated and acylated peptides. Only FRET between acylated peptides was sensitive to cholesterol depletion and only acylated peptides cocluster with caveolin, a marker for caveolae-rafts. Thus, both acylated and prenylated peptides are clustered, but only acylated peptides reside in cholesterol and sphingolipid-rich rafts.

Another demonstration of clustering of potential raft-components was obtained with a homo-FRET approach. A small, but significant (20-40%) fraction of GPI-anchored proteins (including YFP or CFP fused to a GPI-moiety) form extremely high density clusters of nanometer size (4-5nm), each consisting of only a few (<4) molecules. The clusters are sensitive to cholesterol levels in living cells. Interestingly, the fraction of monomers and clusters is constant over a wide range of expression levels implicating an active mechanism for their formation and maintenance (Varma and Mayor, 1998, Sharma *et al*, 2004). Thus, the current concept favours very small and unstable (dynamic) rafts, which upon stimulation or crosslinking coalesce to form larger, more stable rafts (Harder *et al.*, 1998).

An object of the present invention was to provide methods and means for studying the assembly of influenza virus proteins in lipid-rafts, preferably the association of HA with lipid-rafts.

M1 can be detected in the nucleus both by biochemical methods (i.e. cellular fractionation), and fluorescence microscopy. However, since M1 is predominantly localized in the nucleus, and since the nuclear fluorescence signal outshines any signals in other compartments, conventional fluorescence microscopy is less suitable for studying the effect of a candidate compound on the membrane binding of M1.

Another object of the present invention was to provide methods and means for studying the association of M1 with the cellular membrane.

Thus, the general object of the present invention was to provide methods and means for studying influenza virus particle release.

## Detailed description of the invention

The object of the present invention is solved by a matrix protein M1 mutant comprising a nuclear export signal of Rev protein and a fluorescent protein or comprising a myristoylation signal of Lyn kinase and a fluorescent protein.

In one embodiment of the mutant, the fluorescent protein is cyan-fluorescent protein CFP.

In one embodiment, the mutant comprises the nuclear export signal of Rev protein at the C-terminus and the fluorescent protein at the N-terminus.

In one embodiment, the mutant comprises a linker.

In one embodiment, the mutant comprises a linker according to SEQ ID NO: 60 between the nuclear export signal of Rev protein and the fluorescent protein.

In one embodiment, the mutant comprises the myristoylation signal of Lyn kinase at the N-terminus and the fluorescent protein at the C-terminus.

The object of the present invention is further solved by a nucleic acid encoding the matrix protein M 1 mutant according to the present invention.

The object of the present invention is further solved by a nucleic acid comprising a nucleic acid sequence according to SEQ ID NO: 1 or SEQ ID NO: 3.

The object of the present invention is further solved by a nucleic acid comprising a nucleic acid sequence according to SEQ ID NO: 59.

The object of the present invention is further solved by an amino acid sequence comprising an amino acid sequence according to SEQ ID NO: 2 or SEQ ID NO: 4.

The object of the present invention is further solved by a protein comprising an amino acid sequence according to SEQ ID NO: 2 or SEQ ID NO: 4.

The object of the present invention is further solved by a matrix protein M1 mutant according to the present invention comprising an amino acid sequence according to SEQ ID NO: 2 or SEQ ID NO: 4.

The object of the present invention is further solved by a vector comprising a nucleic acid according to the present invention, preferably a nucleic acid comprising a nucleic acid sequence according to SEQ ID NO: 1 or SEQ ID NO: 3.

In one embodiment, the vector used to generate the vector according to the present invention is pECFP-N1 or pECFP-C1. In one embodiment, the vector is pECFP-N1 in case of CFP-M1-NES. In another embodiment, the vector is pECFP-C1 in case of Myr-N1-NES.

The object of the present invention is further solved by a cell comprising a mutant according to the present invention and/or a nucleic acid according to the present invention and/or an amino acid sequence according to the present invention and/or a vector and/or a construct according to the present invention.

The object of the present invention is further solved by a use of a mutant according to the present invention and/or of a nucleic acid according to the present invention and/or of an amino acid sequence according to the present invention and/or of a vector and/or a construct according to the present invention and/or of a cell according to the present invention for an evaluation of an association of the matrix protein M1 mutant with a cellular membrane.

The object of the present invention is further solved by a method for an evaluation of an association of a matrix protein M1 mutant according to the present invention with a cellular membrane, preferably using fluorescence microscopy on an intact cell.

The object of the present invention is further solved by a method for an evaluation of an interference of a candidate compound with an association of a matrix protein M1 mutant according to the present invention with a cellular membrane, comprising the steps of:

(a) incubating a cell according to the present invention with the candidate compound;
(b) detecting the association of the matrixprotein M1 mutant with the cellular membrane in the cell.

In one embodiment, the method further comprising the steps of:

(a') incubating a control cell according to the present invention without the candidate compound;
(b') detecting the association of the matrix protein M1 mutant with the cellular membrane in the control cell;
(c) comparing the results obtained in steps (b) and (b').

In one embodiment of the method, steps (b) and (b') involve fluorescence microscopy on the intact cell.

In one embodiment of the method, interference is an inhibition, and the candidate compound is a putative inhibitor of an association of the matrix protein M1 with the cellular membrane.

The object of the present invention is further solved by a primer comprising a nucleic acid sequence according to SEQ ID NO: 64, SEQ ID NO: 65, SEQ ID NO: 70, or SEQ ID NO: 71.

Further considered by the present invention is a high-throughput procedure for an evaluation of an interference of a candidate compound with an association of the matrix protein M1 with the membrane.

Generally considered is a matrix protein M1 mutant from any influenza virus comprising any nuclear export signal (as specified in LaCour *et al.*, 2004 or predicted by the NES-predictor: www.cbs.dtu.dk), or any myristoylation signal (predicted by: http://expasy.org/tools/myristoylator/), and any fluorescent protein for which a cloned gene is available. Correspondingly, respective constructs are considered. Also considered is a any fluorescent protein, e.g. derived from GFP.

The object of the present invention is further solved by a construct selected from the group consisting of ARTIFICAL-HA-YFP (comprising a nucleic acid sequence according to SEQ ID NO: 5 encoding an amino acid sequence according to SEQ ID NO: 6), ARTIFICAL-HA-CFP (comprising a nucleic acid sequence according to SEQ ID NO: 7 encoding an amino acid sequence according to SEQ ID NO: 8), ARTIFICAL-HA-YFPM3 (comprising a nucleic acid sequence according to SEQ ID NO: 9 encoding an amino acid sequence according to SEQ ID NO: 10), and ARTIFICAL-HA-CFPM3 (comprising a nucleic acid sequence according to SEQ ID NO: 11 encoding an amino acid sequence according to SEQ ID NO: 12).

The object of the present invention is further solved by a construct selected from the group consisting of raft-marker GPI(DAF)-CFP (comprising a nucleic acid sequence according to SEQ ID NO: 13 encoding an amino acid sequence according to SEQ ID NO: 14), and GPI(DAF)-YFP (comprising a nucleic acid sequence according to SEQ ID NO: 15 encoding an amino acid sequence according to SEQ ID NO: 16).

The object of the present invention is further solved by a construct selected from the group consisting of HA-mCer (comprising a nucleic acid sequence according to SEQ ID NO: 17 encoding an amino acid sequence according to SEQ ID NO: 18), HA-mYFP (comprising a nucleic acid sequence according to SEQ ID NO: 19 encoding an amino acid sequence according to SEQ ID NO: 20), M3-HA-mCer (comprising a nucleic acid sequence according to SEQ ID NO: 21 encoding an amino acid sequence according to SEQ ID NO: 22), and M3-HA-mYFP (comprising a nucleic acid sequence according to SEQ ID NO: 23 encoding an amino acid sequence according to SEQ ID NO: 24).

The object of the present invention is further solved by a construct selected from the group consisting of raft-marker Lyn-mCer (comprising a nucleic acid sequence according to SEQ ID NO: 25 encoding an amino acid sequence according to SEQ ID NO: 26), Lyn-mYFP (comprising a nucleic acid sequence according to SEQ ID NO: 27 encoding an amino acid sequence according to SEQ ID NO: 28), Mem-mCer (comprising a nucleic acid sequence according to SEQ ID NO: 29 encoding an amino acid sequence according to SEQ ID NO: 30), and Mem-mYFP (comprising a nucleic acid sequence according to SEQ ID NO: 31 encoding an amino acid sequence according to SEQ ID NO: 32).

The object of the present invention is further solved by a mutant protein expressed using a construct according to the present invention, preferably a HA mutant or a raft-marker mutant.

The object of the present invention is further solved by a nucleic acid comprising a nucleic acid sequence selected from the group consisting of SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, and SEQ ID NO: 31.

The object of the present invention is further solved by an amino acid sequence comprising an amino acid sequence selected from the group consisting of SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, and SEQ ID NO: 32.

The object of the present invention is further solved by a protein comprising an amino acid sequence selected from the group consisting of SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, and SEQ ID NO: 32.

The object of the present invention is further solved by a HA mutant comprising an amino acid sequence selected from the group consisting of SEQ ID NO: 6, SEQ ID NO: 8, SEQ ID NO: 10, SEQ ID NO: 12, SEQ ID NO: 18, SEQ ID NO: 20, SEQ ID NO: 22, and SEQ ID NO: 24.

The object of the present invention is further solved by a raft-marker mutant comprising an amino acid sequence selected from the group consisting of SEQ ID NO: 14, SEQ ID NO: 16, SEQ ID NO: 26, SEQ ID NO: 28, SEQ ID NO: 30, and SEQ ID NO: 32.

The object of the present invention is further solved by a vector comprising a nucleic acid according to the present invention, preferably a nucleic acid comprising a nucleic acid sequence selected from the group consisting of SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, and SEQ ID NO: 31.

The object of the present invention is further solved by a cell comprising a mutant protein according to the present invention and/or a nucleic acid according to the present invention and/or an amino acid sequence according to the present invention and/or a vector and/or a construct according to the present invention.

The object of the present invention is further solved by a use of a mutant protein according to the present invention and/or of a nucleic acid according to the present invention and/or of an amino acid sequence according to the present invention and/or of a vector and/or a construct according to the present invention and/or of a cell according to the present invention for an evaluation of an association of a HA mutant according to the present invention and a raft-marker mutant according to the present invention.

The object of the present invention is further solved by a method for an evaluation of an association of a HA mutant according to the present invention and a raft-marker mutant according to the present invention, preferably using FRET on an intact cell.

The object of the present invention is further solved by a method for an evaluation of an interference of a candidate compound with an association of a HA mutant according to the present invention and a raft-marker mutant according to the present invention, comprising the steps of:

(a) incubating a cell expressing a HA mutant according to the present invention and a raft-marker mutant according to the present invention with the candidate compound;
(b) detecting the association of the HA mutant according to the present invention and the raft-marker mutant according to the present invention in the cell.
In one embodiment, the method further comprises the steps of:

(a') incubating a control cell expressing a HA mutant according to the present invention and a raft-marker mutant according to the present invention without the candidate compound;
(b') detecting the association of the HA mutant according to the present invention and the raft-marker mutant according to the present invention in the control cell;
(c) comparing the results obtained in steps (b) and (b').

In one embodiment of the method, steps (b) and (b') involve FRET on the intact cell.

In one embodiment of the method, interference is an inhibition, and the candidate compound is a putative inhibitor of an association of HA with lipid-rafts.

Further considered by the present invention is a high-throughput procedure for an evaluation of an interference of a candidate compound with an association of HA with lipid-rafts.

The term "construct" means a genetically engineered nucleic acid, preferably contained in a vector or plasmid.

The present invention provides methods and means suitable for screening candidate compounds for interference with the binding of M1 to the membrane, and thus, for interference with the assembly of influenza viruses. The present invention further provides methods and means suitable for screening candidate compounds for interference with the accumulation of HA in lipid-rafts, and thus, for interference with the assembly of influenza viruses. Both assay systems can be used independently or in combination.

For carrying out method according to the present invention for an evaluation of an interference of a candidate compound with an association of a HA mutant and a raft-marker with a cellular membrane, any combination of a HA mutant according to the present invention and a raft-marker according to the present invention is considered beyond the explicit combinations described herein.

We constructed HA/raft-marker pairs where the fluorophores are either inside or outside of the cell. HA-mCer, HA-mYFP, M3-HA-mCer, M3-HA-mYFP, Lyn-mCer, Lyn-mYFP, Mem-mCer and Mem-mYFP have the fluorophore on the cytoplasmic side of the plasma membrane. ARTIFICAL-HA-YFP, ARTIFICAL-HA-CFP, ARTIFICAL-HA-YFPM3, ARTIFICAL-HA-CFPM3, GPI(DAF)-CFP and GPI(DAF)-YFP carry the fluorophore on the outside of the cell. To measure FRET, two constructs with the fluorophores on the same side of the membrane must be used.

Use of the described fusion proteins allows to measure FRET on both sides of the plasma membrane. The lipid-composition of rafts differ between the outer and the inner leaflet of the membrane and it is thus possible that certain compounds to be analyzed with the FRET-method described here might interfere differently with raft/HA pairs which carry the fluorophore on the outside or on the inside of the cell. Furthermore, two different fluorophores such as CFP (or its variant Cer) and YFP must be used. All constructs designated M3 have a substitution of the three cyteine residues in the C-terminal part of HA by serines. The cysteine serve as palmitoylation sites, a hydrophobic protein modification which is required for raft localisation of HA. The M3-constructs are intended to be used as a negative control for the FRET-signal, i.e. the signal given by the M3-constructs with a raft-marker is background, which can be substracted from the signal given by the HA constructs containing the palmitoylation site with the raft-marker.

The variants of GFP designated with an m, e.g. mYFP, represent a form of the protein which is less likely to form dimers on its own and thus gives rise to a false positive FRET-signal (Zacharias *et al*., 2002). However, mYFP or mCFP can be probably replaced by YFP and CFP, respectively and *vice versa* without fundamental effects on the assay.

Likewise, we have used the signal peptide of HA for construction of the ARTIFICAL-HA constructs and for GPI (DAF)-CFP. However, every signal peptide derived from the N-terminus of a secreted or transmembrane protein located in the ER, the Golgi or the plasma membrane of an eukaryotic cell should work in the assays. Moreover, the sequence from DAF encoding the attachment of the glykolipid anchor could probably be replaced by any other sequence from a protein attached to a glykolipid-anchor.

In order to investigate M1 binding to membranes using a fluorescently labelled construct, the intrinsic M1 localization to the cell nucleus had to be overcome because the signal would outshine or otherwise interfere with all other M1 signals. The exclusion of M1 from the nucleus was achieved by inclusion of (1) a nuclear export signal derived from Rev or (2) a myristoylation signal derived from Lyn kinase. The usefulness of the M1 constructs is not limited to the CFP-variant described here. In principle any fluorescent protein for which a cloned gene is available, e.g. GFP and spectral variants thereof or the red coral protein or spectral variants thereof, can be fused to M1 and used in the assay.

Moreover, despite fusion to variants of the green-fluorescent protein, our constructs are transported from their site of synthesis to the plasma membrane. To achieve this we had to construct and test several linker regions, but only the one present in the HA-construct described here was not retained inside the cell. Our constructs thus represent useful tools to study the effect of various compounds on the transport of viral proteins to the plasma membrane, the intracellular site of assembly of influenza virus and for studying interactions between HA and M1 with FRET, a further requirement for budding of infectious influenza virions.

SEQ ID NO: 1 to SEQ ID NO: 32 as indicated above are given below.

SEQ ID NO: 1 and SEQ ID NO: 2:

ATGGTGAGCAAGGGCGAGGAGCTGTTCACCGGGGTGGTGCCCATCCTGGTCGAGCTGGACGG
CGACGTAAACGGCCACAAGTTCAGCGTGTCCGGCGAGGGCGAGGGCGATGCCACCTACGGCA
AGCTGACCCTGAAGTTCATCTGCACCACCGGCAAGCTGCCCGTGCCCTGGCCCACCCTCGTG
ACCACCCTGACCTGGGGCGTGCAGTGCTTCAGCCGCTACCCCGACCACATGAAGCAGCACGA
CTTCTTCAAGTCCGCCATGCCCGAAGGCTACGTCCAGGAGCGCACCATCTTCTTCAAGGACG
ACGGCAACTACAAGACCCGCGCCGAGGTGAAGTTCGAGGGCGACACCCTGGTGAACCGCATC
GAGCTGAAGGGCATCGACTTCAAGGAGGACGGCAACATCCTGGGGCACAAGCTGGAGTACAA
CTACATCAGCCACAACGTCTATATCACCGCCGACAAGCAGAAGAACGGCATCAAGGCCAACT
TCAAGATCCGCCACAACATCGAGGACGGCAGCGTGCAGCTCGCCGACCACTACCAGCAGAAC
ACCCCCATCGGCGACGGCCCCGTGCTGCTGCCCGACAACCACTACCTGAGCACCCAGTCCGC
CCTGAGCAAAGACCCCAACGAGAAGCGCGATCACATGGTCCTGCTGGAGTTCGTGACCGCCG
CCGGGATCACTCTCGGCATGGACGAGCTGTACAAGTCCGGACTCAGATCTCGAGCT*ATGAGT*
*CTTCTAACCGAGGTTGAAACGTACGTTCTCTCTATCATCCCATCAGGCCCCCTCAAAGCCGA*
*GATCGCGCAGAGACTTGAAGATGTCTTTGCAGGGAAAAACACAGACCTTGAGGTTCTCATGG*
*AATGGCTAAAGACAAGACCAATCCTGTCACCTCTGACTAAAGGGATTTTGGGTTTGTGTTT*
*ACGCTCACCGTGCCCAGTGAGCAAGGACTGCAGCGTAGACGCTTTGTCCAAAATGCCCTAAA*
*TGGGAATGGGGATCCAAATAACATGGATAAAGCCGTCAAACTATACAGGAAGTTGAAAAGGG*
*AGATAACATTCTATGGAGCTAAGGAAGTGGCACTCAGTTACTCTACTGGAGCACTTGCCAGT*
*TGTATGGGCCTCATATACAACAGAATGGGAACTGTGACCACAGAGGTGGCATTTGGCCTAGT*
*GTGTGCCACTTGTGAGCAGATTGCTGATTCACAGCATCGGTCTCACAGACAGATGGTGGCTA*

*CCACCAATCCACTAATCAGGCATGAGAACAGAATGGTAATGGCCAGCACTACAGCTAAGGCT*
*ATGGAGCAAATGGCTGGGTCAATTGAACAGGCAGCGGAGGCCATGGAGGTTGCTAGCCAGGC*
*TAGGCAGATGGTGCAGGCAATGAGGACAATTGGGACTCATCCTAGCTCCAGTGCTGGTCTGA*
*AAGATGATCTTCTTGAAAATTTGCAGGCCTACCAGAAACGGATGGGAGTGCAGATGCAACGA*
*TTCAAG***CTTCAGCTACCACCGCTTGAGAGACTTACTCTTTGA**

(SEQ ID NO: 1)

MVSKGEELFTGVVPILVELDGDVNGHKFSVSGEGEGDATYGKLTLKFICTTGKLPVPWPTLV
TTLTWGVQCFSRYPDHMKQHDFFKSAMPEGYVQERTIFFKDDGNYKTRAEVKFEGDTLVNRI
ELKGIDFKEDGNILGHKLEYNYISHNVYITADKQKNGIKANFKIRHNIEDGSVQLADHYQQN
TPIGDGPVLLPDNHYLSTQSALSKDPNEKRDHMVLLEFVTAAGITLGMDELYKSGLRSRA*MS*
*LLTEVETYVLSIIPSGPLKAEIAQRLEDVFAGKNTDLEVLMEWLKTRPILSPLTKGILGFVF*
*TLTVPSEQGLQRRRFVQNALNGNGDPNNMDKAVKLYRKLKREITFYGAKEVALSYSTGALAS*
*CMGLIYNRMGTVTTEVAFGLVCATCEQIADSQHRSHRQMVATTNPLIRHENRMVMASTTAKA*
*MEQMAGSIEQAAEAMEVASQARQMVQAMRTIGTHPSSSAGLKDDLLENLQAYQKRMGVQMQR*
*FK***LQLPPLERLTL**

(SEQ ID NO: 2)

(normal typeface: CFP, underlined: linker, italics: M1, **bold:** NES)

SEQ ID NO: 3 and SEQ ID NO: 4:

**ATGGGATCAATCAAATCAAAG***ATGAGTCTTCTAACCGAGGTTGAAACGTACGTTCTCTCTAT*
*CATCCCATCAGGCCCCCTCAAAGCCGAGATCGCGCAGAGACTTGAAGATGTCTTTGCAGGGA*
*AAAACACAGACCTTGAGGTTCTCATGGAATGGCTAAAGACAAGACCAATCCTGTCACCTCTG*
*ACTAAAGGGATTTTGGGGTTTGTGTTTACGCTCACCGTGCCCAGTGAGCAAGGACTGCAGCG*
*TAGACGCTTTGTCCAAAATGCCCTAAATGGGAATGGGGATCCAAATAACATGGATAAAGCCG*
*TCAAACTATACAGGAAGTTGAAAAGGGAGATAACATTCTATGGAGCTAAGGAAGTGGCACTC*
*AGTTACTCTACTGGAGCACTTGCCAGTTGTATGGGCCTCATATACAACAGAATGGGAACTGT*
*GACCACAGAGGTGGCATTTGGCCTAGTGTGTGCCACTTGTGAGCAGATTGCTGATTCACAGC*
*ATCGGTCTCACAGACAGATGGTGGCTACCACCAATCCACTAATCAGGCATGAGAACAGAATG*
*GTAATGGCCAGCACTACAGCTAAGGCTATGGAGCAAATGGCTGGGTCAATTGAACAGGCAGC*
*GGAGGCCATGGAGGTTGCTAGCCAGGCTAGGCAGATGGTGCAGGCAATGAGGACAATTGGGA*
*CTCATCCTAGCTCCAGTGCTGGTCTGAAAGATGATCTTCTTGAAAATTTGCAGGCCTACCAG*
*AAACGGATGGGAGTGCAGATGCAACGATTCAAG*CCGCGGGCCCGGGATCCACCGGTCGCCAC

CATGGTGAGCAAGGGCGAGGAGCTGTTCACCGGGGTGGTGCCCATCCTGGTCGAGCTGGACG
GCGACGTAAACGGCCACAAGTTCAGCGTGTCCGGCGAGGGCGAGGGCGATGCCACCTACGGC
AAGCTGACCCTGAAGTTCATCTGCACCACCGGCAAGCTGCCCGTGCCCTGGCCCACCCTCGT
GACCACCCTGACCTGGGGCGTGCAGTGCTTCAGCCGCTACCCCGACCACATGAAGCAGCACG
ACTTCTTCAAGTCCGCCATGCCCGAAGGCTACGTCCAGGAGCGCACCATCTTCTTCAAGGAC
GACGGCAACTACAAGACCCGCGCCGAGGTGAAGTTCGAGGGCGACACCCTGGTGAACCGCAT
CGAGCTGAAGGGCATCGACTTCAAGGAGGACGGCAACATCCTGGGGCACAAGCTGGAGTACA
ACTACATCAGCCACAACGTCTATATCACCGCCGACAAGCAGAAGAACGGCATCAAGGCCAAC
TTCAAGATCCGCCACAACATCGAGGACGGCAGCGTGCAGCTCGCCGACCACTACCAGCAGAA
CACCCCCATCGGCGACGGCCCCGTGCTGCTGCCCGACAACCACTACCTGAGCACCCAGTCCG
CCCTGAGCAAAGACCCCAACGAGAAGCGCGATCACATGGTCCTGCTGGAGTTCGTGACCGCC
GCCGGGATCACTCTCGGCATGGACGAGCTGTACAAGTAA

(SEQ ID NO: 3)

**MGSIKSK***MSLLTEVETYVLSIIPSGPLKAEIAQRLEDVFAGKNTDLEVLMEWLKTRPILSPL TKGILGFVFTLTVPSEQGLQRRRFVQNALNGNGDPNNMDKAVKLYRKLKREITFYGAKEVAL SYSTGALASCMGLIYNRMGTVTTEVAFGLVCATCEQIADSQHRSHRQMVATTNPLIRHENRM VMASTTAKAMEQMAGSIEQAAEAMEVASQARQMVQAMRTIGTHPSSSAGLKDDLLENLQAYQ KRMGVQMQRFK*<u>PRARDPPVAT</u>MVSKGEELFTGVVPILVELDGDVNGHKFSVSGEGEGDATYG KLTLKFICTTGKLPVPWPTLVTTLTWGVQCFSRYPDHMKQHDFFKSAMPEGYVQERTIFFKD DGNYKTRAEVKFEGDTLVNRIELKGIDFKEDGNILGHKLEYNYISHNVYITADKQKNGIKAN FKIRHNIEDGSVQLADHYQQNTPIGDGPVLLPDNHYLSTQSALSKDPNEKRDHMVLLEFVTA AGITLGMDELYK

(SEQ ID NO: 4)

(**bold:** myristoylation signal, *italics:* M1, <u>underlined</u>: linker, normal typeface: CFP)

SEQ ID NO: 5 and SEQ ID NO: 6:

**ATGAACACTCAAATCCTGGTTTTCGCCCTTGTGGCAGTCATTCCCACAAATGCAGACAAAAT TTGTCTT**<u>CCGCGGGCCCGGGATCCACCGGTCGCCACC</u>*ATGGTGAGCAAGGGCGAGGAGCTGT TCACCGGGGTGGTGCCCATCCTGGTCGAGCTGGACGGCGACGTAAACGGCCACAAGTTCAGC GTGTCCGGCGAGGGCGAGGGCGATGCCACCTACGGCAAGCTGACCCTGAAGTTCATCTGCAC CACCGGCAAGCTGCCCGTGCCCTGGCCCACCCTCGTGACCACCTTCGGCTACGGCCTGCAGT GCTTCGCCCGCTACCCCGACCACATGAAGCAGCACGACTTCTTCAAGTCCGCCATGCCCGAA*

*GGCTACGTCCAGGAGCGCACCATCTTCTTCAAGGACGACGGCAACTACAAGACCCGCGCCGA*
*GGTGAAGTTCGAGGGCGACACCCTGGTGAACCGCATCGAGCTGAAGGGCATCGACTTCAAGG*
*AGGACGGCAACATCCTGGGGCACAAGCTGGAGTACAACTACAACAGCCACAACGTCTATATC*
*ATGGCCGACAAGCAGAAGAACGGCATCAAGGTGAACTTCAAGATCCGCCACAACATCGAGGA*
*CGGCAGCGTGCAGCTCGCCGACCACTACCAGCAGAACACCCCCATCGGCGACGGCCCCGTGC*
*TGCTGCCCGACAACCACTACCTGAGCTACCAGTCCGCCCTGAGCAAAGACCCCAACGAGAAG*
*CGCGATCACATGGTCCTGCTGGAGTTCGTGACCGCCGCCGGGATCACTCTCGGCATGGACGA*
*GCTGTACAAG*GCTAGTATAAGGAACAATACTTATGATCACAGCAAATACAGAGAAGAAGCGA
TGCAAAATAGAATACAAATTGACCCAGTCAAATTGAGTAGTGGCTACAAAGATGTGATACTT
TGGTTTAGCTTCGGGGCATCATGCTTTTTGCTTCTTGCCATTGCAATGGGCCTTGTTTTCAT
ATGTGTGAAGAACGGAAACATGCGGTGCACTATTTGTATATAA

(SEQ ID NO: 5)

**MNTQILVFALVAVIPTNADKICL**PRARDPPVAT*MVSKGEELFTGVVPILVELDGDVNGHKFS*
*VSGEGEGDATYGKLTLKFICTTGKLPVPWPTLVTTFGYGLQCFARYPDHMKQHDFFKSAMPE*
*GYVQERTIFFKDDGNYKTRAEVKFEGDTLVNRIELKGIDFKEDGNILGHKLEYNYNSHNVYI*
*MADKQKNGIKVNFKIRHNIEDGSVQLADHYQQNTPIGDGPVLLPDNHYLSYQSALSKDPNEK*
*RDHMVLLEFVTAAGITLGMDELYK*ASIRNNTYDHSKYREEAMQNRIQIDPVKLSSGYKDVIL
WFSFGASCFLLLAIAMGLVFICVKNGNMRCTICI

(SEQ ID NO: 6)

(**bold:** HA signal sequence, underlined: linker, in *italics:* YFP, in normal typeface: HA-TMD/CT)

SEQ ID NO: 7 and SEQ ID NO: 8:

**ATGAACACTCAAATCCTGGTTTTCGCCCTTGTGGCAGTCATTCCCACAAATGCAGACAAAAT**
**TTGTCTT**CCGCGGCCCGGGATCCACCGGTCGCCACC*ATGGTGAGCAAGGGCGAGGAGCTGT*
*TCACCGGGGTGGTGCCCATCCTGGTCGAGCTGGACGGCGACGTAAACGGCCACAAGTTCAGC*
*GTGTCCGGCGAGGGCGAGGGCGATGCCACCTACGGCAAGCTGACCCTGAAGTTCATCTGCAC*
*CACCGGCAAGCTGCCCGTGCCCTGGCCCACCCTCGTGACCACCCTGACCTGGGGCGTGCAGT*
*GCTTCAGCCGCTACCCCGACCACATGAAGCAGCACGACTTCTTCAAGTCCGCCATGCCCGAA*
*GGCTACGTCCAGGAGCGCACCATCTTCTTCAAGGACGACGGCAACTACAAGACCCGCGCCGA*
*GGTGAAGTTCGAGGGCGACACCCTGGTGAACCGCATCGAGCTGAAGGGCATCGACTTCAAGG*
*AGGACGGCAACATCCTGGGGCACAAGCTGGAGTACAACTACATCAGCCACAACGTCTATATC*

*ACCGCCGACAAGCAGAAGAACGGCATCAAGGCCAACTTCAAGATCCGCCACAACATCGAGGA*
*CGGCAGCGTGCAGCTCGCCGACCACTACCAGCAGAACACCCCCATCGGCGACGGCCCCGTGC*
*TGCTGCCCGACAACCACTACCTGAGCACCCAGTCCGCCCTGAGCAAAGACCCCAACGAGAAG*
*CGCGATCACATGGTCCTGCTGGAGTTCGTGACCGCCGCCGGGATCACTCTCGGCATGGACGA*
*GCTGTACAAG*GCTAGTATAAGGAACAATACTTATGATCACAGCAAATACAGAGAAGAAGCGA
TGCAAAATAGAATACAAATTGACCCAGTCAAATTGAGTAGTGGCTACAAAGATGTGATACTT
TGGTTTAGCTTCGGGGCATCATGCTTTTTGCTTCTTGCCATTGCAATGGGCCTTGTTTTCAT
ATGTGTGAAGAACGGAAACATGCGGTGCACTATTTGTATATAA

(SEQ ID NO: 7)

**MNTQILVFALVAVIPTNADKICL**PRARDPPVAT*MVSKGEELFTGVVPILVELDGDVNGHKFS*
*VSGEGEGDATYGKLTLKFICTTGKLPVPWPTLVTTLTWGVQCFSRYPDHMKQHDFFKSAMPE*
*GYVQERTIFFKDDGNYKTRAEVKFEGDTLVNRIELKGIDFKEDGNILGHKLEYNYISHNVYI*
*TADKQKNGIKANFKIRHNIEDGSVQLADHYQQNTPIGDGPVLLPDNHYLSTQSALSKDPNEK*
*RDHMVLLEFVTAAGITLGMDELYK*ASIRNNTYDHSKYREEAMQNRIQIDPVKLSSGYKDVIL
WFSFGASCFLLLAIAMGLVFICVKNGNMRCTICI

(SEQ ID NO: 8)

(**bold:** HA signal sequence, underlined: linker, in *italics:* CFP, in normal typeface: HA-TMD/CT)

SEQ ID NO: 9 and SEQ ID NO: 10:

**ATGAACACTCAAATCCTGGTTTTCGCCCTTGTGGCAGTCATTCCCACAAATGCAGACAAAAT**
**TTGTCTT**CCGCGGGCCCGGGATCCACCGGTCGCCACC*ATGGTGAGCAAGGGCGAGGAGCTGT*
*TCACCGGGGTGGTGCCCATCCTGGTCGAGCTGGACGGCGACGTAAACGGCCACAAGTTCAGC*
*GTGTCCGGCGAGGGCGAGGGCGATGCCACCTACGGCAAGCTGACCCTGAAGTTCATCTGCAC*
*CACCGGCAAGCTGCCCGTGCCCTGGCCCACCCTCGTGACCACCTTCGGCTACGGCCTGCAGT*
*GCTTCGCCCGCTACCCCGACCACATGAAGCAGCACGACTTCTTCAAGTCCGCCATGCCCGAA*
*GGCTACGTCCAGGAGCGCACCATCTTCTTCAAGGACGACGGCAACTACAAGACCCGCGCCGA*
*GGTGAAGTTCGAGGGCGACACCCTGGTGAACCGCATCGAGCTGAAGGGCATCGACTTCAAGG*
*AGGACGGCAACATCCTGGGGCACAAGCTGGAGTACAACTACAACAGCCACAACGTCTATATC*
*ATGGCCGACAAGCAGAAGAACGGCATCAAGGTGAACTTCAAGATCCGCCACAACATCGAGGA*
*CGGCAGCGTGCAGCTCGCCGACCACTACCAGCAGAACACCCCCATCGGCGACGGCCCCGTGC*
*TGCTGCCCGACAACCACTACCTGAGCTACCAGTCCGCCCTGAGCAAAGACCCCAACGAGAAG*

*CGCGATCACATGGTCCTGCTGGAGTTCGTGACCGCCGCCGGGATCACTCTCGGCATGGACGA*
*GCTGTACAAG*GCTAGTATAAGGAACAATACTTATGATCACAGCAAATACAGAGAAGAAGCGA
TGCAAAATAGAATACAAATTGACCCAGTCAAATTGAGTAGTGGCTACAAAGATGTGATACTT
TGGTTTAGCTTCGGGGCATCATGCTTTTTGCTTCTTGCCATTGCAATGGGCCTTGTTTTCAT
ATCTGTGAAGAACGGAAACATGCGGTCCACTATTTCTATATAA

(SEQ ID NO: 9)

MNTQILVFALVAVIPTNADKICLPRARDPPVAT*MVSKGEELFTGVVPILVELDGDVNGHKFS*
*VSGEGEGDATYGKLTLKFICTTGKLPVPWPTLVTTFGYGLQCFARYPDHMKQHDFFKSAMPE*
*GYVQERTIFFKDDGNYKTRAEVKFEGDTLVNRIELKGIDFKEDGNILGHKLEYNYNSHNVYI*
*MADKQKNGIKVNFKIRHNIEDGSVQLADHYQQNTPIGDGPVLLPDNHYLSYQSALSKDPNEK*
*RDHMVLLEFVTAAGITLGMDELYK*ASIRNNTYDHSKYREEAMQNRIQIDPVKLSSGYKDVIL
WFSFGASCFLLLAIAMGLVFISVKNGNMRSTISI

(SEQ ID NO: 10)

(**bold:** HA signal peptide, underlined: linker, in *italics:* YFP, underlined: residues changed for Cys → Ser mutation)

SEQ ID NO: 11 and SEQ ID NO: 12:

**ATGAACACTCAAATCCTGGTTTTCGCCCTTGTGGCAGTCATTCCCACAAATGCAGACAAAAT**
**TTGTCTT**CCGCGGGCCCGGGATCCACCGGTCGCCACC*ATGGTGAGCAAGGGCGAGGAGCTGT*
*TCACCGGGGTGGTGCCCATCCTGGTCGAGCTGGACGGCGACGTAAACGGCCACAAGTTCAGC*
*GTGTCCGGCGAGGGCGAGGGCGATGCCACCTACGGCAAGCTGACCCTGAAGTTCATCTGCAC*
*CACCGGCAAGCTGCCCGTGCCCTGGCCCACCCTCGTGACCACCCTGACCTGGGGCGTGCAGT*
*GCTTCAGCCGCTACCCCGACCACATGAAGCAGCACGACTTCTTCAAGTCCGCCATGCCCGAA*
*GGCTACGTCCAGGAGCGCACCATCTTCTTCAAGGACGACGGCAACTACAAGACCCGCGCCGA*
*GGTGAAGTTCGAGGGCGACACCCTGGTGAACCGCATCGAGCTGAAGGGCATCGACTTCAAGG*
*AGGACGGCAACATCCTGGGGCACAAGCTGGAGTACAACTACATCAGCCACAACGTCTATATC*
*ACCGCCGACAAGCAGAAGAACGGCATCAAGGCCAACTTCAAGATCCGCCACAACATCGAGGA*
*CGGCAGCGTGCAGCTCGCCGACCACTACCAGCAGAACACCCCCATCGGCGACGGCCCCGTGC*
*TGCTGCCCGACAACCACTACCTGAGCACCCAGTCCGCCCTGAGCAAAGACCCCAACGAGAAG*
*CGCGATCACATGGTCCTGCTGGAGTTCGTGACCGCCGCCGGGATCACTCTCGGCATGGACGA*
*GCTGTACAAG*GCTAGTATAAGGAACAATACTTATGATCACAGCAAATACAGAGAAGAAGCGA
TGCAAAATAGAATACAAATTGACCCAGTCAAATTGAGTAGTGGCTACAAAGATGTGATACTT

TGGTTTAGCTTCGGGGCATCATGCTTTTTGCTTCTTGCCATTGCAATGGGCCTTGTTTTCAT
ATCTGTGAAGAACGGAAACATGCGGTCCACTATTTCTATATAA

(SEQ ID NO: 11)

**MNTQILVFALVAVIPTNADKICL**PRARDPPVAT*MVSKGEELFTGVVPILVELDGDVNGHKFS*
*VSGEGEGDATYGKLTLKFICTTGKLPVPWPTLVTTLTWGVQCFSRYPDHMKQHDFFKSAMPE*
*GYVQERTIFFKDDGNYKTRAEVKFEGDTLVNRIELKGIDFKEDGNILGHKLEYNYISHNVYI*
*TADKQKNGIKANFKIRHNIEDGSVQLADHYQQNTPIGDGPVLLPDNHYLSTQSALSKDPNEK*
*RDHMVLLEFVTAAGITLGMDELYK*ASIRNNTYDHSKYREEAMQNRIQIDPVKLSSGYKDVIL
WFSFGASCFLLLAIAMGLVFISVKNGNMRSTISI

(SEQ ID NO: 12)

(**bold:** HA signal peptide, underlined: linker, in *italics:* CFP, underlined: residues changed for Cys → Ser mutation)

SEQ ID NO: 13 and SEQ ID NO: 14:

**ATGAACACTCAAATCCTGGTTTTCGCCCTTGTGGCAGTCATTCCCACAAATGCAGACAAAAT**
**TTGTCTT**<u>CCGCGGGCCCGGGATCCACCGGTCGCCACC</u>*ATGGTGAGCAAGGGCGAGGAGCTGT*
*TCACCGGGGTGGTGCCCATCCTGGTCGAGCTGGACGGCGACGTAAACGGCCACAAGTTCAGC*
*GTGTCCGGCGAGGGCGAGGGCGATGCCACCTACGGCAAGCTGACCCTGAAGTTCATCTGCAC*
*CACCGGCAAGCTGCCCGTGCCCTGGCCCACCCTCGTGACCACCCTGACCTGGGGCGTGCAGT*
*GCTTCAGCCGCTACCCCGACCACATGAAGCAGCACGACTTCTTCAAGTCCGCCATGCCCGAA*
*GGCTACGTCCAGGAGCGCACCATCTTCTTCAAGGACGACGGCAACTACAAGACCCGCGCCGA*
*GGTGAAGTTCGAGGGCGACACCCTGGTGAACCGCATCGAGCTGAAGGGCATCGACTTCAAGG*
*AGGACGGCAACATCCTGGGGCACAAGCTGGAGTACAACTACATCAGCCACAACGTCTATATC*
*ACCGCCGACAAGCAGAAGAACGGCATCAAGGCCAACTTCAAGATCCGCCACAACATCGAGGA*
*CGGCAGCGTGCAGCTCGCCGACCACTACCAGCAGAACACCCCCATCGGCGACGGCCCCGTGC*
*TGCTGCCCGACAACCACTACCTGAGCACCCAGTCCGCCCTGAGCAAAGACCCCAACGAGAAG*
*CGCGATCACATGGTCCTGCTGGAGTTCGTGACCGCCGCCGGGATCACTCTCGGCATGGACGA*
*GCTGTACAAG*CCAAATAAAGGAAGTGGAACCACTTCAGGTACTACCCGTCTTCTATCTGGGC
ACACGTGTTTCACGTTGACAGGTTTGCTTGGGACGCTAGTAACCATGGGCTTGCTGACTTA

(SEQ ID NO: 13)

**MNTQILVFALVAVIPTNADKICL**<u>PRARDPPVAT</u>*MVSKGEELFTGVVPILVELDGDVNGHKFS*
*VSGEGEGDATYGKLTLKFICTTGKLPVPWPTLVTTLTWGVQCFSRYPDHMKQHDFFKSAMPE*
*GYVQERTIFFKDDGNYKTRAEVKFEGDTLVNRIELKGIDFKEDGNILGHKLEYNYISHNVYI*
*TADKQKNGIKANFKIRHNIEDGSVQLADHYQQNTPIGDGPVLLPDNHYLSTQSALSKDPNEK*
*RDHMVLLEFVTAAGITLGMDELYK*PNKGSGTTSGTTRLLSGHTCFTLTGLLGTLVTMGLLT

(SEQ ID NO: 14)

(**bold:** HA signal peptide, <u>underlined</u>: linker, *italics:* CFP, normal typeface: GPI anchor sequence derived from DAF)

SEQ ID NO: 15 and SEQ ID NO: 16:

**ATGAACACTCAAATCCTGGTTTTCGCCCTTGTGGCAGTCATTCCCACAAATGCAGACAAAAT**
**TTGTCTT**CCGCGGGCCCGGGATCCACCGGTCGCCACC*ATGGTGAGCAAGGGCGAGGAGCTGT*
*TCACCGGGGTGGTGCCCATCCTGGTCGAGCTGGACGGCGACGTAAACGGCCACAAGTTCAGC*
*GTGTCCGGCGAGGGCGAGGGCGATGCCACCTACGGCAAGCTGACCCTGAAGTTCATCTGCAC*
*CACCGGCAAGCTGCCCGTGCCCTGGCCCACCCTCGTGACCACCTTCGGCTACGGCCTGCAGT*
*GCTTCGCCCGCTACCCCGACCACATGAAGCAGCACGACTTCTTCAAGTCCGCCATGCCCGAA*
*GGCTACGTCCAGGAGCGCACCATCTTCTTCAAGGACGACGGCAACTACAAGACCCGCGCCGA*
*GGTGAAGTTCGAGGGCGACACCCTGGTGAACCGCATCGAGCTGAAGGGCATCGACTTCAAGG*
*AGGACGGCAACATCCTGGGGCACAAGCTGGAGTACAACTACAACAGCCACAACGTCTATATC*
*ATGGCCGACAAGCAGAAGAACGGCATCAAGGTGAACTTCAAGATCCGCCACAACATCGAGGA*
*CGGCAGCGTGCAGCTCGCCGACCACTACCAGCAGAACACCCCCATCGGCGACGGCCCCGTGC*
*TGCTGCCCGACAACCACTACCTGAGCTACCAGTCCGCCCTGAGCAAAGACCCCAACGAGAAG*
*CGCGATCACATGGTCCTGCTGGAGTTCGTGACCGCCGCCGGGATCACTCTCGGCATGGACGA*
*GCTGTACAAG*CCAAATAAAGGAAGTGGAACCACTTCAGGTACTACCCGTCTTCTATCTGGGC
ACACGTGTTTCACGTTGACAGGTTTGCTTGGGACGCTAGTAACCATGGGCTTGCTGACTTA

(SEQ ID NO: 15)

**MNTQILVFALVAVIPTNADKICL**PRARDPPVAT*MVSKGEELFTGVVPILVELDGDVNGHKFS*
*VSGEGEGDATYGKLTLKFICTTGKLPVPWPTLVTTFGYGLQCFARYPDHMKQHDFFKSAMPE*
*GYVQERTIFFKDDGNYKTRAEVKFEGDTLVNRIELKGIDFKEDGNILGHKLEYNYNSHNVYI*
*MADKQKNGIKVNFKIRHNIEDGSVQLADHYQQNTPIGDGPVLLPDNHYLSYQSALSKDPNEK*
*RDHMVLLEFVTAAGITLGMDELYK*PNKGSGTTSGTTRLLSGHTCFTLTGLLGTLVTMGLLT

(SEQ ID NO: 16)

(**bold:** HA signal peptide, underlined: linker, *italics:* YFP, normal typeface: GPI anchor sequence derived from DAF)

SEQ ID NO: 17 and SEQ ID NO: 18:

*ATGAACACTCAAATCCTGGTTTTCGCCCTTGTGGCAGTCATTCCCACAAATGCAGACAAAAT*
*TTGTCTTGGACATCATGCTGTATCAAATGGCACCAAAGTAAACACACTCACTGAGAGAGGAG*
*TAGAAGTTGTCAATGCAACGGAAACAGTGGAGCGGACAAACATCCCCAAAATTTGCTCAAAA*
*GGGAAAAGAACCACTGATCTTGGCCAATGCGGACTGTTAGGGACCATTACCGGACCACCTCA*
*ATGCGACCAATTTCTAGAATTTTCAGCTGATCTAATAATCGAGAGACGAGAAGGAAATGATG*
*TTTGTTACCCGGGGAAGTTTGTTAATGAAGAGGCATTGCGACAAATCCTCAGAGGATCAGGT*
*GGGATTGACAAAGAAACAATGGGATTCACATATAGTGGAATAAGGACCAACGGAACAACTAG*
*TGCATGTAGAAGATCAGGGTCTTCATTCTATGCAGAAATGGAGTGGCTCCTGTCAAATACAG*
*ACAATGCTTCTTTCCCACAAATGACAAAATCATACAAAAACACAAGGAGAGAATCAGCTCTG*
*ATAGTATGGGGAATCCACCATTCAGGATCAACCACCGAACAGACCAAACTATATGGGAGTGG*
*AAATAAACTGATAACAGTCGGGAGTTCCAAATATCATCAATCTTTTGTGCCGAGTCCAGGAA*
*CACGACCGCAGATAAATGGCCAGTCCGGACGGATTGATTTTCATTGGTTGATCTTGGATCCC*
*AATGATACAGTTACTTTTAGTTTCAATGGGGCTTTCATAGCTCCAAATCGTGCCAGCTTCTT*
*GAGGGGAAAGTCCATGGGGATCCAGAGCGATGTGCAGGTTGATGCCAATTGCGAAGGGGAAT*
*GCTACCACAGTGGAGGGACTATAACAAGCAGATTGCCTTTTCAAAACATCAATAGCAGAGCA*
*GTTGGCAAATGCCCAAGATATGTAAAACAGGAAAGTTTATTATTGGCAACTGGGATGAAGAA*
*CGTTCCCGAACCTTCCAAAAAAAGGAAAAAAAGAGGCCTGTTTGGCGCTATAGCAGGGTTTA*
*TTGAAAATGGTTGGGAAGGTCTGGTCGACGGGTGGTACGGTTTCAGGCATCAGAATGCACAA*
*GGAGAAGGAACTGCAGCAGACTACAAAAGCACCCAATCGGCAATTGATCAGATAACCGGAAA*
*GTTAAATAGACTCATTGAGAAAACCAACCAGCAATTTGAGCTAATAGATAATGAATTCACTG*
*AGGTGGAAAAGCAGATTGGCAATTTAATTAACTGGACCAAAGACTCCATCACAGAAGTATGG*
*TCTTACAATGCTGAACTTATTGTGGCAATGGAAAACCAGCACACTATTGATTTGGCTGATTC*
*AGAGATGAACAGGCTGTATGAGCGAGTGAGGAAACAATTAAGGGAAAATGCTGAAGAGGATG*
*GTACTGGTTGCTTTGAAATTTTTCATAAATGTGACGATGATTGTATGGCTAGTATAAGGAAC*
*AATACTTATGATCACAGCAAATACAGAGAAGAAGCGATGCAAAATAGAATACAAATTGACCC*
*AGTCAAATTGAGTAGTGGCTACAAAGATGTGATACTTTGGTTTAGCTTCGGGGCATCATGCT*
*TTTTGCTTCTTGCCATTGCAATGGGCCTTGTTTTCATATGTGTGAAGAACGGAAACATGCGG*
*TGCACTATTTGTATA***CTCCGGCCTGAAGCTCCGCGGGCCCGGGATCCACCGGTCGCCACC**AT
GGTGAGCAAGGGCGAGGAGCTGTTCACCGGGGTGGTGCCCATCCTGGTCGAGCTGGACGGCG

ACGTAAACGGCCACAAGTTCAGCGTGTCCGGCGAGGGCGAGGGCGATGCCACCTACGGCAAG
CTGACCCTGAAGTTCATCTGCACCACCGGCAAGCTGCCCGTGCCCTGGCCCACCCTCGTGAC
CACCCTGACCTGGGGCGTGCAGTGCTTCGCCCGCTACCCCGACCACATGAAGCAGCACGACT
TCTTCAAGTCCGCCATGCCCGAAGGCTACGTCCAGGAGCGCACCATCTTCTTCAAGGACGAC
GGCAACTACAAGACCCGCGCCGAGGTGAAGTTCGAGGGCGACACCCTGGTGAACCGCATCGA
GCTGAAGGGCATCGACTTCAAGGAGGACGGCAACATCCTGGGGCACAAGCTGGAGTACAACG
CCATCAGCGACAACGTCTATATCACCGCCGACAAGCAGAAGAACGGCATCAAGGCCAACTTC
AAGATCCGCCACAACATCGAGGACGGCAGCGTGCAGCTCGCCGACCACTACCAGCAGAACAC
CCCCATCGGCGACGGCCCCGTGCTGCTGCCCGACAACCACTACCTGAGCACCCAGTCCAAAC
TGAGCAAAGACCCCAACGAGAAGCGCGATCACATGGTCCTGCTGGAGTTCGTGACCGCCGCC
GGGATCACTCTCGGCATGGACGAGCTGTACAAGTAA

(SEQ ID NO: 17)

*MNTQILVFALVAVIPTNADKICLGHHAVSNGTKVNTLTERGVEVVNATETVERTNIPKICSK*
*GKRTTDLGQCGLLGTITGPPQCDQFLEFSADLIIERREGNDVCYPGKFVNEEALRQILRGSG*
*GIDKETMGFTYSGIRTNGTTSACRRSGSSFYAEMEWLLSNTDNASFPQMTKSYKNTRRESAL*
*IVWGIHHSGSTTEQTKLYGSGNKLITVGSSKYHQSFVPSPGTRPQINGQSGRIDFHWLILDP*
*NDTVTFSFNGAFIAPNRASFLRGKSMGIQSDVQVDANCEGECYHSGGTITSRLPFQNINSRA*
*VGKCPRYVKQESLLLATGMKNVPEPSKKRKKRGLFGAIAGFIENGWEGLVDGWYGFRHQNAQ*
*GEGTAADYKSTQSAIDQITGKLNRLIEKTNQQFELIDNEFTEVEKQIGNLINWTKDSITEVW*
*SYNAELIVAMENQHTIDLADSEMNRLYERVRKQLRENAEEDGTGCFEIFHKCDDDCMASIRN*
*NTYDHSKYREEAMQNRIQIDPVKLSSGYKDVILWFSFGASCFLLLAIAMGLVFICVKNGNMR*
*CTICI***LRPEAPRARDPPVAT**MVSKGEELFTGVVPILVELDGDVNGHKFSVSGEGEGDATYGK
LTLKFICTTGKLPVPWPTLVTTLTWGVQCFARYPDHMKQHDFFKSAMPEGYVQERTIFFKDD
GNYKTRAEVKFEGDTLVNRIELKGIDFKEDGNILGHKLEYNAISDNVYITADKQKNGIKANF
KIRHNIEDGSVQLADHYQQNTPIGDGPVLLPDNHYLSTQSALSKDPNEKRDHMVLLEFVTAA
GITLGMDELYK

(SEQ ID NO: 18)

(*italics*: HA, **bold:** linker, normal typeface: mCer)

SEQ ID NO: 19 and SEQ ID NO: 20:

*ATGAACACTCAAATCCTGGTTTTCGCCCTTGTGGCAGTCATTCCCACAAATGCAGACAAAAT*

*TTGTCTTGGACATCATGCTGTATCAAATGGCACCAAAGTAAACACACTCACTGAGAGAGGAG*

*TAGAAGTTGTCAATGCAACGGAAACAGTGGAGCGGACAAACATCCCCAAAATTTGCTCAAAA*
*GGGAAAAGAACCACTGATCTTGGCCAATGCGGACTGTTAGGGACCATTACCGGACCACCTCA*
*ATGCGACCAATTTCTAGAATTTTCAGCTGATCTAATAATCGAGAGACGAGAAGGAAATGATG*
*TTTGTTACCCGGGGAAGTTTGTTAATGAAGAGGCATTGCGACAAATCCTCAGAGGATCAGGT*
*GGGATTGACAAAGAAACAATGGGATTCACATATAGTGGAATAAGGACCAACGGAACAACTAG*
*TGCATGTAGAAGATCAGGGTCTTCATTCTATGCAGAAATGGAGTGGCTCCTGTCAAATACAG*
*ACAATGCTTCTTTCCCACAAATGACAAAATCATACAAAAACACAAGGAGAGAATCAGCTCTG*
*ATAGTATGGGGAATCCACCATTCAGGATCAACCACCGAACAGACCAAACTATATGGGAGTGG*
*AAATAAACTGATAACAGTCGGGAGTTCCAAATATCATCAATCTTTTGTGCCGAGTCCAGGAA*
*CACGACCGCAGATAAATGGCCAGTCCGGACGGATTGATTTTCATTGGTTGATCTTGGATCCC*
*AATGATACAGTTACTTTTAGTTTCAATGGGGCTTTCATAGCTCCAAATCGTGCCAGCTTCTT*
*GAGGGGAAAGTCCATGGGGATCCAGAGCGATGTGCAGGTTGATGCCAATTGCGAAGGGGAAT*
*GCTACCACAGTGGAGGGACTATAACAAGCAGATTGCCTTTTCAAAACATCAATAGCAGAGCA*
*GTTGGCAAATGCCCAAGATATGTAAAACAGGAAAGTTTATTATTGGCAACTGGGATGAAGAA*
*CGTTCCCGAACCTTCCAAAAAAAGGAAAAAAAGAGGCCTGTTTGGCGCTATAGCAGGGTTTA*
*TTGAAAATGGTTGGGAAGGTCTGGTCGACGGGTGGTACGGTTTCAGGCATCAGAATGCACAA*
*GGAGAAGGAACTGCAGCAGACTACAAAAGCACCCAATCGGCAATTGATCAGATAACCGGAAA*
*GTTAAATAGACTCATTGAGAAAACCAACCAGCAATTTGAGCTAATAGATAATGAATTCACTG*
*AGGTGGAAAAGCAGATTGGCAATTTAATTAACTGGACCAAAGACTCCATCACAGAAGTATGG*
*TCTTACAATGCTGAACTTATTGTGGCAATGGAAAACCAGCACACTATTGATTTGGCTGATTC*
*AGAGATGAACAGGCTGTATGAGCGAGTGAGGAAACAATTAAGGGAAAATGCTGAAGAGGATG*
*GTACTGGTTGCTTTGAAATTTTTCATAAATGTGACGATGATTGTATGGCTAGTATAAGGAAC*
*AATACTTATGATCACAGCAAATACAGAGAAGAAGCGATGCAAAATAGAATACAAATTGACCC*
*AGTCAAATTGAGTAGTGGCTACAAAGATGTGATACTTTGGTTTAGCTTCGGGGCATCATGCT*
*TTTTGCTTCTTGCCATTGCAATGGGCCTTGTTTTCATATGTGTGAAGAACGGAAACATGCGG*
*TGCACTATTTGTATA***CTCCGGCCTGAAGCTCCGCGGCCCGGGATCCACCGGTCGCCACC**AT
GGTGAGCAAGGGCGAGGAGCTGTTCACCGGGGTGGTGCCCATCCTGGTCGAGCTGGACGGCG
ACGTAAACGGCCACAAGTTCAGCGTGTCCGGCGAGGGCGAGGGCGATGCCACCTACGGCAAG
CTGACCCTGAAGTTCATCTGCACCACCGGCAAGCTGCCCGTGCCCTGGCCCACCCTCGTGAC
CACCTTCGGCTACGGCCTGCAGTGCTTCGCCCGCTACCCCGACCACATGAAGCAGCACGACT
TCTTCAAGTCCGCCATGCCCGAAGGCTACGTCCAGGAGCGCACCATCTTCTTCAAGGACGAC
GGCAACTACAAGACCCGCGCCGAGGTGAAGTTCGAGGGCGACACCCTGGTGAACCGCATCGA
GCTGAAGGGCATCGACTTCAAGGAGGACGGCAACATCCTGGGGCACAAGCTGGAGTACAACT
ACAACAGCCACAACGTCTATATCATGGCCGACAAGCAGAAGAACGGCATCAAGGTGAACTTC
AAGATCCGCCACAACATCGAGGACGGCAGCGTGCAGCTCGCCGACCACTACCAGCAGAACAC

CCCCATCGGCGACGGCCCCGTGCTGCTGCCCGACAACCACTACCTGAGCTACCAGTCCAAAC
TGAGCAAAGACCCCAACGAGAAGCGCGATCACATGGTCCTGCTGGAGTTCGTGACCGCCGCC
GGGATCACTCTCGGCATGGACGAGCTGTACAAGTAA

(SEQ ID NO: 19)

*MNTQILVFALVAVIPTNADKICLGHHAVSNGTKVNTLTERGVEVVNATETVERTNIPKICSK*
*GKRTTDLGQCGLLGTITGPPQCDQFLEFSADLIIERREGNDVCYPGKFVNEEALRQILRGSG*
*GIDKETMGFTYSGIRTNGTTSACRRSGSSFYAEMEWLLSNTDNASFPQMTKSYKNTRRESAL*
*IVWGIHHSGSTTEQTKLYGSGNKLITVGSSKYHQSFVPSPGTRPQINGQSGRIDFHWLILDP*
*NDTVTFSFNGAFIAPNRASFLRGKSMGIQSDVQVDANCEGECYHSGGTITSRLPFQNINSRA*
*VGKCPRYVKQESLLLATGMKNVPEPSKKRKKRGLFGAIAGFIENGWEGLVDGWYGFRHQNAQ*
*GEGTAADYKSTQSAIDQITGKLNRLIEKTNQQFELIDNEFTEVEKQIGNLINWTKDSITEVW*
*SYNAELIVAMENQHTIDLADSEMNRLYERVRKQLRENAEEDGTGCFEIFHKCDDDCMASIRN*
*NTYDHSKYREEAMQNRIQIDPVKLSSGYKDVILWFSFGASCFLLLAIAMGLVFICVKNGNMR*
*CTICI***LRPEAPRARDPPVAT**MVSKGEELFTGVVPILVELDGDVNGHKFSVSGEGEGDATYGK
LTLKFICTTGKLPVPWPTLVTTFGYGLQCFARYPDHMKQHDFFKSAMPEGYVQERTIFFKDD
GNYKTRAEVKFEGDTLVNRIELKGIDFKEDGNILGHKLEYNYNSHNVYIMADKQKNGIKVNF
KIRHNIEDGSVQLADHYQQNTPIGDGPVLLPDNHYLSYQSALSKDPNEKRDHMVLLEFVTAA
GITLGMDELYK

(SEQ ID NO: 20)

(*italics:* HA, **bold:** linker, normal typeface: mYFP)

SEQ ID NO: 21 and SEQ ID NO: 22:

*ATGAACACTCAAATCCTGGTTTTCGCCCTTGTGGCAGTCATTCCCACAAATGCAGACAAAAT*

*TTGTCTTGGACATCATGCTGTATCAAATGGCACCAAAGTAAACACACTCACTGAGAGAGGAG*

*TAGAAGTTGTCAATGCAACGGAAACAGTGGAGCGGACAAACATCCCCAAAATTTGCTCAAAA*

*GGGAAAAGAACCACTGATCTTGGCCAATGCGGACTGTTAGGGACCATTACCGGACCACCTCA*

*ATGCGACCAATTTCTAGAATTTTCAGCTGATCTAATAATCGAGAGACGAGAAGGAAATGATG*

*TTTGTTACCCGGGGAAGTTTGTTAATGAAGAGGCATTGCGACAAATCCTCAGAGGATCAGGT*

*GGGATTGACAAAGAAACAATGGGATTCACATATAGTGGAATAAGGACCAACGGAACAACTAG*

*TGCATGTAGAAGATCAGGGTCTTCATTCTATGCAGAAATGGAGTGGCTCCTGTCAAATACAG*

*ACAATGCTTCTTTCCCACAAATGACAAAATCATACAAAAACACAAGGAGAGAATCAGCTCTG*

*ATAGTATGGGGAATCCACCATTCAGGATCAACCACCGAACAGACCAAACTATATGGGAGTGG*

*AAATAAACTGATAACAGTCGGGAGTTCCAAATATCATCAATCTTTTGTGCCGAGTCCAGGAA*
*CACGACCGCAGATAAATGGCCAGTCCGGACGGATTGATTTTCATTGGTTGATCTTGGATCCC*
*AATGATACAGTTACTTTTAGTTTCAATGGGGCTTTCATAGCTCCAAATCGTGCCAGCTTCTT*
*GAGGGGAAAGTCCATGGGGATCCAGAGCGATGTGCAGGTTGATGCCAATTGCGAAGGGGAAT*
*GCTACCACAGTGGAGGGACTATAACAAGCAGATTGCCTTTTCAAAACATCAATAGCAGAGCA*
*GTTGGCAAATGCCCAAGATATGTAAAACAGGAAAGTTTATTATTGGCAACTGGGATGAAGAA*
*CGTTCCCGAACCTTCCAAAAAAAGGAAAAAAAGAGGCCTGTTTGGCGCTATAGCAGGGTTTA*
*TTGAAAATGGTTGGGAAGGTCTGGTCGACGGGTGGTACGGTTTCAGGCATCAGAATGCACAA*
*GGAGAAGGAACTGCAGCAGACTACAAAAGCACCCAATCGGCAATTGATCAGATAACCGGAAA*
*GTTAAATAGACTCATTGAGAAAACCAACCAGCAATTTGAGCTAATAGATAATGAATTCACTG*
*AGGTGGAAAAGCAGATTGGCAATTTAATTAACTGGACCAAAGACTCCATCACAGAAGTATGG*
*TCTTACAATGCTGAACTTATTGTGGCAATGGAAAACCAGCACACTATTGATTTGGCTGATTC*
*AGAGATGAACAGGCTGTATGAGCGAGTGAGGAAACAATTAAGGGAAAATGCTGAAGAGGATG*
*GTACTGGTTGCTTTGAAATTTTTCATAAATGTGACGATGATTGTATGGCTAGTATAAGGAAC*
*AATACTTATGATCACAGCAAATACAGAGAAGAAGCGATGCAAAATAGAATACAAATTGACCC*
*AGTCAAATTGAGTAGTGGCTACAAAGATGTGATACTTTGGTTTAGCTTCGGGGCATCATGCT*
*TTTTGCTTCTTGCCATTGCAATGGGCCTTGTTTTCATATCTGTGAAGAACGGAAACATGCGG*
*TCCACTATTTCTATA***CTCCGGCCTGAAGCTCCGCGGCCCGGGATCCACCGGTCGCCACC**AT
GGTGAGCAAGGGCGAGGAGCTGTTCACCGGGGTGGTGCCCATCCTGGTCGAGCTGGACGGCG
ACGTAAACGGCCACAAGTTCAGCGTGTCCGGCGAGGGCGAGGGCGATGCCACCTACGGCAAG
CTGACCCTGAAGTTCATCTGCACCACCGGCAAGCTGCCCGTGCCCTGGCCCACCCTCGTGAC
CACCCTGACCTGGGGCGTGCAGTGCTTCGCCCGCTACCCCGACCACATGAAGCAGCACGACT
TCTTCAAGTCCGCCATGCCCGAAGGCTACGTCCAGGAGCGCACCATCTTCTTCAAGGACGAC
GGCAACTACAAGACCCGCGCCGAGGTGAAGTTCGAGGGCGACACCCTGGTGAACCGCATCGA
GCTGAAGGGCATCGACTTCAAGGAGGACGGCAACATCCTGGGGCACAAGCTGGAGTACAACG
CCATCAGCGACAACGTCTATATCACCGCCGACAAGCAGAAGAACGGCATCAAGGCCAACTTC
AAGATCCGCCACAACATCGAGGACGGCAGCGTGCAGCTCGCCGACCACTACCAGCAGAACAC
CCCCATCGGCGACGGCCCCGTGCTGCTGCCCGACAACCACTACCTGAGCACCCAGTCCAAAC
TGAGCAAAGACCCCAACGAGAAGCGCGATCACATGGTCCTGCTGGAGTTCGTGACCGCCGCC
GGGATCACTCTCGGCATGGACGAGCTGTACAAGTAA

(SEQ ID NO: 21)

*MNTQILVFALVAVIPTNADKICLGHHAVSNGTKVNTLTERGVEVVNATETVERTNIPKICSK*
*GKRTTDLGQCGLLGTITGPPQCDQFLEFSADLIIERREGNDVCYPGKFVNEEALRQILRGSG*
*GIDKETMGFTYSGIRTNGTTSACRRSGSSFYAEMEWLLSNTDNASFPQMTKSYKNTRRESAL*

*IVWGIHHSGSTTEQTKLYGSGNKLITVGSSKYHQSFVPSPGTRPQINGQSGRIDFHWLILDP*
*NDTVTFSFNGAFIAPNRASFLRGKSMGIQSDVQVDANCEGECYHSGGTITSRLPFQNINSRA*
*VGKCPRYVKQESLLLATGMKNVPEPSKKRKKRGLFGAIAGFIENGWEGLVDGWYGFRHQNAQ*
*GEGTAADYKSTQSAIDQITGKLNRLIEKTNQQFELIDNEFTEVEKQIGNLINWTKDSITEVW*
*SYNAELIVAMENQHTIDLADSEMNRLYERVRKQLRENAEEDGTGCFEIFHKCDDDCMASIRN*
*NTYDHSKYREEAMQNRIQIDPVKLSSGYKDVILWFSFGASCFLLLAIAMGLVFISVKNGNMR*
*STISI***LRPEAPRARDPPVAT**MVSKGEELFTGVVPILVELDGDVNGHKFSVSGEGEGDATYGK
LTLKFICTTGKLPVPWPTLVTTLTWGVQCFARYPDHMKQHDFFKSAMPEGYVQERTIFFKDD
GNYKTRAEVKFEGDTLVNRIELKGIDFKEDGNILGHKLEYNAISDNVYITADKQKNGIKANF
KIRHNIEDGSVQLADHYQQNTPIGDGPVLLPDNHYLSTQSALSKDPNEKRDHMVLLEFVTAA
GITLGMDELYK

(SEQ ID NO: 22)

(*italics:* HA, underlined: mutations to obtain Cys → Ser exchange (no palmitoylation), **bold:** linker, normal typeface: mCer)

SEQ ID NO: 23 and SEQ ID NO: 24:

ATGAACACTCAAATCCTGGTTTTCGCCCTTGTGGCAGTCATTCCCACAAATGCAGACAAAAT
TTGTCTTGGACATCATGCTGTATCAAATGGCACCAAAGTAAACACACTCACTGAGAGAGGAG
TAGAAGTTGTCAATGCAACGGAAACAGTGGAGCGGACAAACATCCCCAAAATTTGCTCAAAA
GGGAAAAGAACCACTGATCTTGGCCAATGCGGACTGTTAGGGACCATTACCGGACCACCTCA
ATGCGACCAATTTCTAGAATTTTCAGCTGATCTAATAATCGAGAGACGAGAAGGAAATGATG
TTTGTTACCCGGGGAAGTTTGTTAATGAAGAGGCATTGCGACAAATCCTCAGAGGATCAGGT
GGGATTGACAAAGAAACAATGGGATTCACATATAGTGGAATAAGGACCAACGGAACAACTAG
TGCATGTAGAAGATCAGGGTCTTCATTCTATGCAGAAATGGAGTGGCTCCTGTCAAATACAG
ACAATGCTTCTTTCCCACAAATGACAAAATCATACAAAAACACAAGGAGAGAATCAGCTCTG
ATAGTATGGGGAATCCACCATTCAGGATCAACCACCGAACAGACCAAACTATATGGGAGTGG
AAATAAACTGATAACAGTCGGGAGTTCCAAATATCATCAATCTTTTGTGCCGAGTCCAGGAA
CACGACCGCAGATAAATGGCCAGTCCGGACGGATTGATTTTCATTGGTTGATCTTGGATCCC
AATGATACAGTTACTTTTAGTTTCAATGGGGCTTTCATAGCTCCAAATCGTGCCAGCTTCTT
GAGGGGAAAGTCCATGGGGATCCAGAGCGATGTGCAGGTTGATGCCAATTGCGAAGGGGAAT
GCTACCACAGTGGAGGGACTATAACAAGCAGATTGCCTTTTCAAAACATCAATAGCAGAGCA
GTTGGCAAATGCCCAAGATATGTAAAACAGGAAAGTTTATTATTGGCAACTGGGATGAAGAA
CGTTCCCGAACCTTCCAAAAAAAGGAAAAAAAGAGGCCTGTTTGGCGCTATAGCAGGGTTTA

*TTGAAAATGGTTGGGAAGGTCTGGTCGACGGGTGGTACGGTTTCAGGCATCAGAATGCACAA*
*GGAGAAGGAACTGCAGCAGACTACAAAAGCACCCAATCGGCAATTGATCAGATAACCGGAAA*
*GTTAAATAGACTCATTGAGAAAACCAACCAGCAATTTGAGCTAATAGATAATGAATTCACTG*
*AGGTGGAAAAGCAGATTGGCAATTTAATTAACTGGACCAAAGACTCCATCACAGAAGTATGG*
*TCTTACAATGCTGAACTTATTGTGGCAATGGAAAACCAGCACACTATTGATTTGGCTGATTC*
*AGAGATGAACAGGCTGTATGAGCGAGTGAGGAAACAATTAAGGGAAAATGCTGAAGAGGATG*
*GTACTGGTTGCTTTGAAATTTTTCATAAATGTGACGATGATTGTATGGCTAGTATAAGGAAC*
*AATACTTATGATCACAGCAAATACAGAGAAGAAGCGATGCAAAATAGAATACAAATTGACCC*
*AGTCAAATTGAGTAGTGGCTACAAAGATGTGATACTTTGGTTTAGCTTCGGGGCATCATGCT*
*TTTTGCTTCTTGCCATTGCAATGGGCCTTGTTTTCATATCTGTGAAGAACGGAAACATGCGG*
*TCCACTATTTCTATA***CTCCGGCCTGAAGCTCCGCGGGCCCGGGATCCACCGGTCGCCACC**AT
GGTGAGCAAGGGCGAGGAGCTGTTCACCGGGGTGGTGCCCATCCTGGTCGAGCTGGACGGCG
ACGTAAACGGCCACAAGTTCAGCGTGTCCGGCGAGGGCGAGGGCGATGCCACCTACGGCAAG
CTGACCCTGAAGTTCATCTGCACCACCGGCAAGCTGCCCGTGCCCTGGCCCACCCTCGTGAC
CACCTTCGGCTACGGCCTGCAGTGCTTCGCCCGCTACCCCGACCACATGAAGCAGCACGACT
TCTTCAAGTCCGCCATGCCCGAAGGCTACGTCCAGGAGCGCACCATCTTCTTCAAGGACGAC
GGCAACTACAAGACCCGCGCCGAGGTGAAGTTCGAGGGCGACACCCTGGTGAACCGCATCGA
GCTGAAGGGCATCGACTTCAAGGAGGACGGCAACATCCTGGGGCACAAGCTGGAGTACAACT
ACAACAGCCACAACGTCTATATCATGGCCGACAAGCAGAAGAACGGCATCAAGGTGAACTTC
AAGATCCGCCACAACATCGAGGACGGCAGCGTGCAGCTCGCCGACCACTACCAGCAGAACAC
CCCCATCGGCGACGGCCCCGTGCTGCTGCCCGACAACCACTACCTGAGCTACCAGTCCAAAC
TGAGCAAAGACCCCAACGAGAAGCGCGATCACATGGTCCTGCTGGAGTTCGTGACCGCCGCC
GGGATCACTCTCGGCATGGACGAGCTGTACAAGTAA

(SEQ ID NO: 23)

*MNTQILVFALVAVIPTNADKICLGHHAVSNGTKVNTLTERGVEVVNATETVERTNIPKICSK*
*GKRTTDLGQCGLLGTITGPPQCDQFLEFSADLIIERREGNDVCYPGKFVNEEALRQILRGSG*
*GIDKETMGFTYSGIRTNGTTSACRRSGSSFYAEMEWLLSNTDNASFPQMTKSYKNTRRESAL*
*IVWGIHHSGSTTEQTKLYGSGNKLITVGSSKYHQSFVPSPGTRPQINGQSGRIDFHWLILDP*
*NDTVTFSFNGAFIAPNRASFLRGKSMGIQSDVQVDANCEGECYHSGGTITSRLPFQNINSRA*
*VGKCPRYVKQESLLLATGMKNVPEPSKKRKKRGLFGAIAGFIENGWEGLVDGWYGFRHQNAQ*
*GEGTAADYKSTQSAIDQITGKLNRLIEKTNQQFELIDNEFTEVEKQIGNLINWTKDSITEVW*
*SYNAELIVAMENQHTIDLADSEMNRLYERVRKQLRENAEEDGTGCFEIFHKCDDDCMASIRN*
*NTYDHSKYREEAMQNRIQIDPVKLSSGYKDVILWFSFGASCFLLLAIAMGLVFISVKNGNMR*
*STISI***LRPEAPRARDPPVAT**MVSKGEELFTGVVPILVELDGDVNGHKFSVSGEGEGDATYGK

LTLKFICTTGKLPVPWPTLVTTFGYGLQCFARYPDHMKQHDFFKSAMPEGYVQERTIFFKDD
GNYKTRAEVKFEGDTLVNRIELKGIDFKEDGNILGHKLEYNYNSHNVYIMADKQKNGIKVNF
KIRHNIEDGSVQLADHYQQNTPIGDGPVLLPDNHYLSYQSALSKDPNEKRDHMVLLEFVTAA
GITLGMDELYK

(SEQ ID NO: 24)

(*italics:* HA, underlined: mutations to obtain Cys → Ser exchange (no palmitoylation), **bold:** linker, normal typeface: mYFP)

SEQ ID NO: 25 and SEQ ID NO: 26:

**ATGGGATGTATTAAATCAAAAAGGAAAGACAATCTCAATGACGATGAACCGGTCGCCACC**AT
GGTGAGCAAGGGCGAGGAGCTGTTCACCGGGGTGGTGCCCATCCTGGTCGAGCTGGACGGCG
ACGTAAACGGCCACAAGTTCAGCGTGTCCGGCGAGGGCGAGGGCGATGCCACCTACGGCAAG
CTGACCCTGAAGTTCATCTGCACCACCGGCAAGCTGCCCGTGCCCTGGCCCACCCTCGTGAC
CACCCTGACCTGGGGCGTGCAGTGCTTCGCCCGCTACCCCGACCACATGAAGCAGCACGACT
TCTTCAAGTCCGCCATGCCCGAAGGCTACGTCCAGGAGCGCACCATCTTCTTCAAGGACGAC
GGCAACTACAAGACCCGCGCCGAGGTGAAGTTCGAGGGCGACACCCTGGTGAACCGCATCGA
GCTGAAGGGCATCGACTTCAAGGAGGACGGCAACATCCTGGGGCACAAGCTGGAGTACAACG
CCATCAGCGACAACGTCTATATCACCGCCGACAAGCAGAAGAACGGCATCAAGGCCAACTTC
AAGATCCGCCACAACATCGAGGACGGCAGCGTGCAGCTCGCCGACCACTACCAGCAGAACAC
CCCCATCGGCGACGGCCCCGTGCTGCTGCCCGACAACCACTACCTGAGCACCCAGTCCAAAC
TGAGCAAAGACCCCAACGAGAAGCGCGATCACATGGTCCTGCTGGAGTTCGTGACCGCCGCC
GGGATCACTCTCGGCATGGACGAGCTGTACAAGTAA

(SEQ ID NO: 25)

**MGCIKSKRKDNLNDDEPVAT**MVSKGEELFTGVVPILVELDGDVNGHKFSVSGEGEGDATYGK
LTLKFICTTGKLPVPWPTLVTTLTWGVQCFARYPDHMKQHDFFKSAMPEGYVQERTIFFKDD
GNYKTRAEVKFEGDTLVNRIELKGIDFKEDGNILGHKLEYNAISDNVYITADKQKNGIKANF
KIRHNIEDGSVQLADHYQQNTPIGDGPVLLPDNHYLSTQSALSKDPNEKRDHMVLLEFVTAA
GITLGMDELYK

(SEQ ID NO: 26)

(**bold:** Lyn kinase sequence, **bold** and underlined: linker, normal typeface: mCer)

SEQ ID NO: 27 and SEQ ID NO: 28:

**ATGGGATGTATTAAATCAAAAAGGAAAGACAATCTCAATGACGATGAACCGGTCGCCACC**AT
GGTGAGCAAGGGCGAGGAGCTGTTCACCGGGGTGGTGCCCATCCTGGTCGAGCTGGACGGCG
ACGTAAACGGCCACAAGTTCAGCGTGTCCGGCGAGGGCGAGGGCGATGCCACCTACGGCAAG
CTGACCCTGAAGTTCATCTGCACCACCGGCAAGCTGCCCGTGCCCTGGCCCACCCTCGTGAC
CACCTTCGGCTACGGCCTGCAGTGCTTCGCCCGCTACCCCGACCACATGAAGCAGCACGACT
TCTTCAAGTCCGCCATGCCCGAAGGCTACGTCCAGGAGCGCACCATCTTCTTCAAGGACGAC
GGCAACTACAAGACCCGCGCCGAGGTGAAGTTCGAGGGCGACACCCTGGTGAACCGCATCGA
GCTGAAGGGCATCGACTTCAAGGAGGACGGCAACATCCTGGGGCACAAGCTGGAGTACAACT
ACAACAGCCACAACGTCTATATCATGGCCGACAAGCAGAAGAACGGCATCAAGGTGAACTTC
AAGATCCGCCACAACATCGAGGACGGCAGCGTGCAGCTCGCCGACCACTACCAGCAGAACAC
CCCCATCGGCGACGGCCCCGTGCTGCTGCCCGACAACCACTACCTGAGCTACCAGTCCAAAC
TGAGCAAAGACCCCAACGAGAAGCGCGATCACATGGTCCTGCTGGAGTTCGTGACCGCCGCC
GGGATCACTCTCGGCATGGACGAGCTGTACAAGTAA

(SEQ ID NO: 27)

**MGCIKSKRKDNLNDDEPVAT**MVSKGEELFTGVVPILVELDGDVNGHKFSVSGEGEGDATYGK
LTLKFICTTGKLPVPWPTLVTTFGYGLQCFARYPDHMKQHDFFKSAMPEGYVQERTIFFKDD
GNYKTRAEVKFEGDTLVNRIELKGIDFKEDGNILGHKLEYNYNSHNVYIMADKQKNGIKVNF
KIRHNIEDGSVQLADHYQQNTPIGDGPVLLPDNHYLSYQSALSKDPNEKRDHMVLLEFVTAA
GITLGMDELYK

(SEQ ID NO: 28)

(**bold:** Lyn kinase sequence, bold and underlined: linker, normal typeface: mYFP)

SEQ ID NO: 29 and SEQ ID NO: 30:

**ATGCTGTGCTGTATGAGAAGAACCAAACAGGTTGAAAAGAATGATGAGGACCAACCGGTCGC
CACC**ATGGTGAGCAAGGGCGAGGAGCTGTTCACCGGGGTGGTGCCCATCCTGGTCGAGCTGG
ACGGCGACGTAAACGGCCACAAGTTCAGCGTGTCCGGCGAGGGCGAGGGCGATGCCACCTAC
GGCAAGCTGACCCTGAAGTTCATCTGCACCACCGGCAAGCTGCCCGTGCCCTGGCCCACCCT
CGTGACCACCCTGACCTGGGGCGTGCAGTGCTTCGCCCGCTACCCCGACCACATGAAGCAGC
ACGACTTCTTCAAGTCCGCCATGCCCGAAGGCTACGTCCAGGAGCGCACCATCTTCTTCAAG
GACGACGGCAACTACAAGACCCGCGCCGAGGTGAAGTTCGAGGGCGACACCCTGGTGAACCG

CATCGAGCTGAAGGGCATCGACTTCAAGGAGGACGGCAACATCCTGGGGCACAAGCTGGAGT
ACAACGCCATCAGCGACAACGTCTATATCACCGCCGACAAGCAGAAGAACGGCATCAAGGCC
AACTTCAAGATCCGCCACAACATCGAGGACGGCAGCGTGCAGCTCGCCGACCACTACCAGCA
GAACACCCCCATCGGCGACGGCCCCGTGCTGCTGCCCGACAACCACTACCTGAGCACCCAGT
CCAAACTGAGCAAAGACCCCAACGAGAAGCGCGATCACATGGTCCTGCTGGAGTTCGTGACC
GCCGCCGGGATCACTCTCGGCATGGACGAGCTGTACAAGTAA

(SEQ ID NO: 29)

**MLCCMRRTKQVEKNDEDQ**<u>**PVAT**</u>MVSKGEELFTGVVPILVELDGDVNGHKFSVSGEGEGDATY
GKLTLKFICTTGKLPVPWPTLVTTLTWGVQCFARYPDHMKQHDFFKSAMPEGYVQERTIFFK
DDGNYKTRAEVKFEGDTLVNRIELKGIDFKEDGNILGHKLEYNAISDNVYITADKQKNGIKA
NFKIRHNIEDGSVQLADHYQQNTPIGDGPVLLPDNHYLSTQSALSKDPNEKRDHMVLLEFVT
AAGITLGMDELYK

(SEQ ID NO: 30)

(**bold:** GAP43 sequence, **bold** and <u>underlined</u>: linker, normal typeface: mCer)

SEQ ID NO: 31 and SEQ ID NO: 32:

**ATGCTGTGCTGTATGAGAAGAACCAAACAGGTTGAAAAGAATGATGAGGACCAA**<u>**CCGGTCGC**</u>
<u>**CACC**</u>ATGGTGAGCAAGGGCGAGGAGCTGTTCACCGGGGTGGTGCCCATCCTGGTCGAGCTGG
ACGGCGACGTAAACGGCCACAAGTTCAGCGTGTCCGGCGAGGGCGAGGGCGATGCCACCTAC
GGCAAGCTGACCCTGAAGTTCATCTGCACCACCGGCAAGCTGCCCGTGCCCTGGCCCACCCT
CGTGACCACCTTCGGCTACGGCCTGCAGTGCTTCGCCCGCTACCCCGACCACATGAAGCAGC
ACGACTTCTTCAAGTCCGCCATGCCCGAAGGCTACGTCCAGGAGCGCACCATCTTCTTCAAG
GACGACGGCAACTACAAGACCCGCGCCGAGGTGAAGTTCGAGGGCGACACCCTGGTGAACCG
CATCGAGCTGAAGGGCATCGACTTCAAGGAGGACGGCAACATCCTGGGGCACAAGCTGGAGT
ACAACTACAACAGCCACAACGTCTATATCATGGCCGACAAGCAGAAGAACGGCATCAAGGTG
AACTTCAAGATCCGCCACAACATCGAGGACGGCAGCGTGCAGCTCGCCGACCACTACCAGCA
GAACACCCCCATCGGCGACGGCCCCGTGCTGCTGCCCGACAACCACTACCTGAGCTACCAGT
CCAAACTGAGCAAAGACCCCAACGAGAAGCGCGATCACATGGTCCTGCTGGAGTTCGTGACC
GCCGCCGGGATCACTCTCGGCATGGACGAGCTGTACAAGTAA

(SEQ ID NO: 31)

**MLCCMRRTKQVEKNDEDQ*****PVAT***MVSKGEELFTGVVPILVELDGDVNGHKFSVSGEGEGDATY
GKLTLKFICTTGKLPVPWPTLVTTFGYGLQCFARYPDHMKQHDFFKSAMPEGYVQERTIFFK
DDGNYKTRAEVKFEGDTLVNRIELKGIDFKEDGNILGHKLEYNYNSHNVYIMADKQKNGIKV
NFKIRHNIEDGSVQLADHYQQNTPIGDGPVLLPDNHYLSYQSALSKDPNEKRDHMVLLEFVT
AAGITLGMDELYK

(SEQ ID NO: 32)

(**bold:** GAP43 sequence, **bold** and underlined: linker, normal typeface: mYF)

The invention shall be now further illustrated considering the accompanying figures and the following examples.

## Figures

Figure 1 shows the assembly of viral components at lipid-rafts. Upper panel: scanning electron microscopy picture. Lower panel: schematic diagram. HA, hemagglutinin; NA, neuraminidase; M1, matrix protein M1; vRNP, viral ribonucleoprotein.

Figure 2 is a schematic diagram showing the interactions of HA with raft-markers in transfected cells as studied with FRET. Left panel: Native HA-CFP (left) and Lyn-YFP (right). Right panel: GPI(DAF)-CFP (left) and ARTIFICAL-HA-YFP protein (right) containing the raft-targeting signal as well as the trans-membrane and cytoplasmic domain of HA. CFP, cyan fluorescence protein; YFP, yellow fluorescence protein; SP, signal peptide.

Figure 3 shows the ARTIFICAL-HA-CFP construct (Fig. 3a) and the raft-marker GPI(DAF)-YFP (Fig. 3b). L, linker; TMD, transmembrane region; CT, cytoplasmatic domain; GPI, glycolipid anchor; DAF, decay-accelaration factor.
SP(HA), signal peptide from HA.

Figure 4 shows CHO cells transiently expressing ARTIFICAL-HA-YFP (Fig. 4a), GPI(DAF)-CFP (Fig. 4b), GPI (DAF)-YFP (Fig. 4c). Confocal fluorescence microscopy.

Figure 5 shows a fluorography picture of a SDS-PAGE gel of immunoprecipitated proteins of [$^{35}$S]methionin labelled CHO cells transfected with ARTIFICAL-HAYFP construct after digestion with endoglycosidase H (H) or PNGase F (F).

Figure 6 shows the life time of raft-marker GPI(DAF)-CFP fluorescence in the presence and absence of ARTIFICAL-HA-YFP (Fig. 6a) and the FRET efficiency (%) of GPI(DAF)-CFP/HA-YFP prior to and after depletion of cholesterol (Fig. 6b).

Figure 7 shows the HA-mCer construct (Fig. 7a), the raft-marker Lyn-YFP construct (Fig. 7b) and the raft-marker Mem-mYFP construct (Fig. 7c). mCer, mCerulean; m, monomerized form of YFP with the A206K mutation; GAP, GAP43.

Figure 8 shows CHO cells transiently expressing HA-mCerulean (Fig. 8a), stably expressing Mem-mYFP (Fig. 8b) or Lyn-mYFP (Fig. 8c) and MDCK cells stably expressing Mem-mYFP (Fig. 8d) and Lyn-mYFP (Fig. 8e). Confocal fluorescence microscopy.

Figure 9 shows SDS-PAGE under reducing (red) or non-reducing (non-red) conditions and fluorography of proteins precipitated from [$^{35}$S]methionine labelled CHO cells transfected with HA-mCer. Proteolytic cleavage (Fig. 9a), glycosylation (Fig. 9b) and palmitoylation (Fig. 9c).

Figure 10 shows CHO cells transfected with HA-mCerulean (left panel in Fig. 10a and Fig. 10b each) and Lyn-YFP (right panel in Fig. 10a and Fig 10b each). FRET analysis prior to bleaching (Fig. 10a) and after bleaching (Fig. 10b).

Figure 11 shows the life time of HA-mCerulean in the presence and absence of raftmarker Lyn.

Figure 12 shows the CFP-M1-NES construct (Fig. 12a) and the Myr-M1-CFP construct (Fig. 12b). M1, matrix protein; NES, nuclear export signal; Myr, myristoylation signal.

Figure 13 shows CHO cells transfected with CFP-M1-wild type (a), CFP-M1-nuclear export signal (b) and Myr-M1-CFP (c). Fluorescence microscopy.

Figure 14 shows the membrane association of CFP-M1-wt and CFP-M1-NES as assessed by biochemical fractionation. CHO cells were transfected with the indicated constructs, lysed, cleared of nuclei by centrifugation and then ultracentrifuged at 100,000x g for 60 min. (a) represents the total cell lysate, (b) the 100,000× g pellet (membranes and membrane-bound proteins) and (c) the 100,000× g supernatant (soluble proteins). Western Blot probed with an anti-M1 antibody, the position of M1 is indicated by an arrow. A negative control (untransfected cells) is included (right lanes).

## EXAMPLES

EXAMPLE 1: Studies on the interaction of HA and raft-marker on the outside of the plasma membrane using Artifical-HA-YFP (or -CFP) and raft-marker-CFP (or -YFP) -GPI constructs

### 1.1 ARTIFICAL-HA-YFP

The ARTIFICAL-HA-YFP construct includes the "yellow-fluorescent protein" (YFP) fused to the signal peptide of HA via a linker region. Located at the C-terminus of YFP are 35 amino acids of the extracytoplasmatic domain of HA as well as the complete transmembrane region and the cytoplasmatic domain (Fig. 3a).

Also considered is the replacement of YFP by CFP (or by its variant Cer).

The linker between the signal peptide and the fluorophore (either CFP or YFP) is as follows:

**ATGAACACTCAAATCCTGGTTTTCGCCCTTGTGGCAGTCATTCCCACAAATGCAGACAAAAT**

**TTGTCTT**CCGCGGGCCCGGGATCCACCGGTCGCCACC*ATGGTG*

(SEQ ID NO: 33)

(in **bold**: HA signal peptide, underlined: linker region, in *italics:* start of CFP or YFP sequence). Translation into protein: **MNTQILVFALVAVIPTNADKICL**PRARDPPVAT*MV* (SEQ ID NO: 34)

The linker between the end of the fluorophore and TMD+CT of HA is as follows:

*GACGAGCTGTACAAG*GCTAGTATAAGGAACAATACTTATGATCACAGCAAATACAGAGAAGA

AGCGATGCAAAATAGAATACAAATTGACCCAGTCAAATTGAGTAGTGGCTACAAAGATGTGA

TACTTTGGTTTAGCTTCGGGGCATCATGCTTTTTGCTTCTTGCCATTGCAATGGGCCTTGTT

TTCATATGTGTGAAGAACGGAAACATGCGGTGCACTATTTGTATATAA

(SEQ ID NO: 35)

(in *italics:* end of CFP/YFP, in normal typeface: sequence for TMD and CT of HA). Translation into protein: *DELYK*ASIR**NNT**YDHSKYREEAMQNRIQIDPVKLSSGYKDVILWFSFGASCFLLLAIAMGLV FIC*VKNGNMRC*TIC*I.

(SEQ ID NO: 36)
(in **bold:** glycosylation sites, underlined: transmembrane region, underlined and **bold:** C-terminal domain, C*: palmitoylated cysteine residues).

Also considered is a mutant of HA in which the three palmitoylated cysteine residues important for raft localization are replaced by serines. The cysteine mutants are called ARTIFICAL-HA-YFPM3, ARTIFICAL-HA-CFPM3, respectively.

1.2 Raft-marker GPI(DAF)-YFP

The raft-marker GPI(DAF)-YFP includes the signal peptide of HA fused to the YFP via a linker region. Located at the C-terminus of YFP are amino acids comprising the signals for attachment of the glycolipid anchor (GPI) from the cellular protein "decay-acceleration factor" (DAF) (Legler *et al.* 2005) (Fig. 3b).

Also considered is the replacement of YFP by CFP.

The DNA sequence of the GPI(DAF)-YFP construct is as follows:

**ATGAACACTCAAATCCTGGTTTTCGCCCTTGTGGCAGTCATTCCCACAAATGCAGACAAAAT**
**TTGTCTT**CCGCGGGCCCGGGATCCACCGGTCGCCACC*ATGGTGAGCAAGGGCGAGGAGCTGT*
*TCACCGGGGTGGTGCCCATCCTGGTCGAGCTGGACGGCGACGTAAACGGCCACAAGTTCAGC*
*GTGTCCGGCGAGGGCGAGGGCGATGCCACCTACGGCAAGCTGACCCTGAAGTTCATCTGCAC*
*CACCGGCAAGCTGCCCGTGCCCTGGCCCACCCTCGTGACCACCTTCGGCTACGGCCTGCAGT*
*GCTTCGCCCGCTACCCCGACCACATGAAGCAGCACGACTTCTTCAAGTCCGCCATGCCCGAA*
*GGCTACGTCCAGGAGCGCACCATCTTCTTCAAGGACGACGGCAACTACAAGACCCGCGCCGA*
*GGTGAAGTTCGAGGGCGACACCCTGGTGAACCGCATCGAGCTGAAGGGCATCGACTTCAAGG*
*AGGACGGCAACATCCTGGGGCACAAGCTGGAGTACAACTACAACAGCCACAACGTCTATATC*
*ATGGCCGACAAGCAGAAGAACGGCATCAAGGTGAACTTCAAGATCCGCCACAACATCGAGGA*
*CGGCAGCGTGCAGCTCGCCGACCACTACCAGCAGAACACCCCCATCGGCGACGGCCCCGTGC*
*TGCTGCCCGACAACCACTACCTGAGCTACCAGTCCGCCCTGAGCAAAGACCCCAACGAGAAG*
*CGCGATCACATGGTCCTGCTGGAGTTCGTGACCGCCGCCGGGATCACTCTCGGCATGGACGA*
*GCTGTACAAG*CCAAATAAAGGAAGTGGAACCACTTCAGGTACTACCCGTCTTCTATCTGGGC
ACACGTGTTTCACGTTGACAGGTTTGCTTGGGACGCTAGTAACCATGGGCTTGCTGACTTA

(SEQ ID NO: 15)

(in **bold:** HA signal peptide, underlined: linker, *italics*: YFP, normal typeface: GPI anchor sequence derived from DAF).

The protein sequence of the GPI(DAF)-YFP construct is as follows:

**MNTQILVFALVAVIPTNADKICL**PRARDPPVAT*MVSKGEELFTGVVPILVELDGDVNGHKFS*
*VSGEGEGDATYGKLTLKFICTTGKLPVPWPTLVTTFGYGLQCFARYPDHMKQHDFFKSAMPE*
*GYVQERTIFFKDDGNYKTRAEVKFEGDTLVNRIELKGIDFKEDGNILGHKLEYNYNSHNVYI*
*MADKQKNGIKVNFKIRHNIEDGSVQLADHYQQNTPIGDGPVLLPDNHYLSYQSALSKDPNEK*
*RDHMVLLEFVTAAGITLGMDELYK*PNKGSGTTSGTTRLLSGHTCFTLTGLLGTLVTMGLLT

(SEQ ID NO: 16)

1.3 ARTIFICAL-HA-YFP ARTIFICAL-HA-CFP, ARTIFICAL-HA-YFPM3, ARTIFICAL-HA-CFPM3, GPI(DAF)-CFP, and GPI(DAF)-YFP

ARTIFICAL-HA-YFP, ARTIFICAL-HA-CFP, ARTIFICAL-HA-YFPM3, ARTIFICAL-HA-CFPM3, GPI (DAF)-CFP, GPI(DAF)-YFP constructs were generated using the plasmids pEYFP-N1 and pECFP-N1 (Clontech). The HA signal peptide was designed as oligonucleotide having the following sequences:
GATCTCATGAACACTCAAATCCTGGTTTTCGCCCTTGTGGCAGTCATTCCCACAAATGCAGA CAAAATTTGTCTTCCGC (SEQ ID NO: 37)
sense oligonucleotide
GGAAGACAAATTTTGTCTGCATTTGTGGGAATGACTGCCACAAGGGCGAAAACCAGGATTTG AGTGTTCATGA (SEQ ID NO: 38)
antisense oligonucleotide

The oligonucleotide was ligated into the plasmids using the *Bgl*II and *Sac*II restrictions sites within the multiple cloning site (MCS) (both for the HA and GPI constructs). For the HA constructs, the HA transmembrane domain and cytoplasmic tail was obtained as a single polymerase chain reaction (PCR) fragment using as a template the cDNA encoding the HA gene from influenza virus strain A/FPV/Rostock/34 (H7N1) and the cDNA encoding the HA gene from influenza virus strain A/FPV/Rostock/34 (H7N1) mutated at postions 551, 559, 562 (C→S).

The PCR fragment was generated using the following primers:
GGCTGTACAAGGCTAGTATAAGGAACAATACT (SEQ ID NO: 39)
sense primer
GCGCGGCCGCTTATATACAAATAGTGCACCGC (SEQ ID NO: 40)
HA wt antisense primer
GCGCGGCCGCTTATATAGAAATAGTGGACCGC (SEQ ID NO: 41)
HA M3 antisense primer

The PCR fragment was ligated into the plasmid using the *BsrG*I and *Not*I restrictions sites. For GPI(DAF) constructs (Legler *et al.* 2004), the GPI signal of the DAF was designed as oligonucleotide having the following sequences:

GTACAAGCCAAATAAAGGAAGTGGAACCACTTCAGGTACTACCCGTCTTCTATCTGGGCACA
CGTGTTTCACGTTGACAGGTTTGCTTGGGACGCTAGTAACCATGGGCTTGCTGACTTAGC
antisense oligonucleotide (SEQ ID NO: 42)

GGCCGCTAAGTCAGCAAGCCCATGGTTACTAGCGTCCCAAGCAAACCTGTCAACGTGAAACA
CGTGTGCCCAGATAGAAGACGGGTAGTACCTGAAGTGGTTCCACTTCCTTTATTTGGCTT
sense oligonucleotide (SEQ ID NO: 43)

The oligonucleotide was cloned into the *BsrG*I/*Not*I restriction sites of the vectors. Amplification of the plasmids was carried out in *E. coli* (chemically competent, DH-5α).

1.4 Microscopic and biochemical studies

Confocal fluorescence microscopy showed that in CHO cells transiently expressing ARTIFICAL-HA-YFP (Fig. 4a), GPI(DAF)-CFP (Fig. 4b) or GPI(DAF)-YFP (Fig. 4c) the proteins localized at the plasma membrane and in the Golgi apparatus, i.e. at intracellular sites where also the native non-labelled proteins are located.

Figure 5 shows that an ARTIFICAL-HA-YFP construct is glycosylated and able to pass the Golgi apparatus. After transfection and labeling of CHO cells with [$^{35}$S]methionine, the immunoprecipitated proteins were digested with endoglycosidase H or PNGase F or remained undigested (-) and were subjected to SDS-PAGE and fluorography.

1.5 FRET and FLIM analysis

Intensity measurements as well as FRET measurements were carried out with transfected cells expressing donor alone (CFP or mCerulean) or both, donor (CFP or mCerulean) and acceptor (YFP or mYFP). Emission of CFP or mCerulean and YFP or mYFP was obtained by sequential excitation at 440 nm and 515 nm. FRET-induced emission of the acceptor was obtained by excitation at 440 nm.

Table 1: Laser and wavelength for the detection of the fluorophores

| Fluorophore | Laser | Wavelength |
|---|---|---|
| CFP | Laser diode | 440 nm |
| YFP | Argon laser | 514 nm |
| YFP-photobleaching | Argon laser | 514 nm |

Table 2: Filters and beam splitter for the detection of the fluorophores

| Flurophores | Excitation dichroic mirror | Emission dichroic mirror | Detection range |
|---|---|---|---|
| CFP | DM 458/515 | BS 490 | 461 nm-486 nm |
| YFP | DM 458/515 | BS 490 | 535 nm-575 nm |

Tested bleaching conditions:

- 100% laser power, 1 s
- 33% laser power, 30 s
- 50% laser power, 30 s, 45 s, 60 s

Microscope: Olympus FluoView 1000
Objective: UPLSAPO 60x Oil, NA 1.35 (Olympus)
Fluorescence intensity based FRET efficiencies were calculated according to:

$$E=1-I_{DA}/I_D \qquad (1)$$

wherein $I_D$ is the fluorescence intensity of a donor in the absence of an acceptor, and $I_{DA}$ is the intensity of a donor in the presence of an acceptor.
FRET efficiencies measured by acceptor photobleaching were calculated according to:

$$E=1-I_{D(prebleaching)}/I_{D(postbleaching)} \qquad (2)$$

wherein $I_{D(prebleaching)}$ is the fluorescence intensity of a donor in the presence of an acceptor (prebleaching), and $I_{D(postbleaching)}$ is the intensity of a donor after complete bleaching of an acceptor (postbleaching).
FLIM measurements were carried out with tranfected cells expressing donor (CFP or mCerulean) and with transfected cells expressing donor (CFP or mCerulean) and acceptor (YFP or mYFP).

Table 3: Laser and wavelength for the detection of the fluorophores

| Fluorophore | Laser | Wavelength |
|---|---|---|
| CFP | Pulsed laser diode | 440 nm |

Microscope: Olympus FluoView 1000 with LSMupgrade Kit (FLIM) from PicoQuant
Objective: UPLSAPO 60x Oil, NA 1.35 (Olympus)
For lifetime measurement a pulsed diode laser was used with a frequency of 20 MHz. FLIM pictures were accumulated for 90 seconds.
For lifetime measurement the DM 458/515 dichroic mirror was used. The fluorescence was detected by a single photon avalanche photodiode (PicoQuant) via a 470 ± 15 nm filter. The lifetimes were measured by the method of time-correlated single photon counting (TCSPC). The data were collected by the TimeHarp 200 PC card (PicoQuant). In general, the calculation of a fluorescence lifetime image is done by sorting all photons that correspond to one pixel into a histogram, which is then fitted to an exponential decay function to extract the lifetime information. The analysis was performed by the SymPhoTime (PicoQuant) software by deconvoluting the measured curve with the instrument response function (IRF). For single cell lifetime analysis only the fluorescence photons originated from the plasma membrane were taken into account.

Analysis of lifetimes can be done according to different models. In the first model we considered that the donor has an intrinsic complex decay with two components, but we considered that the donor is a single population.

Equation 3 describes the donor decay:

$$I(t) = \sum_i \alpha_i e^{-t/\tau_i} \qquad (3)$$

In this way FRET efficiency has been calculated using the average lifetime after normalizing the amplitudes ($E\alpha_i=1$, where $\alpha_i$ represents the fraction of the emitting molecules having a particular lifetime, $\tau_i$).

$$\tau_{av} = \sum_i \alpha_i \tau_i \qquad (4)$$

FRET efficiency can be obtained by

$$E_{FRET} = 1 - \tau_{DA}/\tau_D \qquad (5)$$

where $\tau_D$ and $\tau_{DA}$ are the donor excited state lifetime in the absence and presence of the acceptor, respectively.

In the second model the complex decay of the donor is due to the existence of two populations of donors and it is described as:

$$I_D(t) = \alpha_1 e^{-t/\tau_1} + \alpha_2 e^{-t/\tau_2} \qquad (6)$$

(donor in the absence of acceptor)

$$I_{DA}(t) = \alpha_1 e^{-t/\tau_1} + \alpha_2 e^{-t/\tau_2} + \alpha_{q1} e^{-t/\tau_{q1}} + \alpha_{q2} e^{-t/\tau_{q2}} \qquad (7)$$

(donor in the presence of acceptor)

The FRET efficiency is then calculated using equation 5, with $\tau_1$, $\tau_2$ and $\tau_{q1}$, $\tau_{q2}$ as the unquenched and quenched donor lifetimes, respectively.

Figure 6a shows the lifetime of fluorescence of the raft-marker GPI(DAF)-CFP in the presence and absence of ARTIFICAL-HA-YFP.

Figure 6b shows the FRET efficiency (%) of GPI(DAF)-CFP/ARTIFICAL-HA-YFP before and after depletion of cholesterol. In this study, cells were treated with 5 mM β-methylcyclodextrin for 1 min at 4 °C. FRET efficiency after cholesterol extraction is reduced due to resolution of the lipid-rafts.

EXAMPLE 2: Studies on the interaction of HA and raft-marker on the inner side of the plasma membrane using HA-mCer and raft-marker Lyn-mYFP or GAP43-mYFP constructs

2.1 HA-mCer

The HA-mCer construct includes mCerulean (mCer), an improved CFP (Rizzo *et al.* 2004), fused to the C-terminus of HA via a linker region (Fig. 7a). Since some of the HA-mCer constructs created are not transported to the cell surface, the construction of the linker region is of particular significance.

The wt-HA-mCer construct is as follows:

*TGCACTATTTGTATA***CTCCGGCCTGAAGCTCCGCGGGCCCGGGATCCACCGGTCGCCACC**AT GGTGAGCAAG (SEQ ID NO: 44)

(in *italics:* end of HA sequence, palmitoylated cysteines underlined, in **bold:** linker, in normal typeface: start of mCerulean).

Translation into protein:

*CTIC***LRPEAPRARDPPVAT**MVSK) (SEQ ID NO: 45)

Also considered is a mutant of HA (M3), in which the three palmitoylation sites important for raft localization are replaced.

2.2 Raft-marker Lyn-mYFP

The raft-marker Lyn-mYFP construct includes a myristoylation signal (i.e. a signal for the attachment of myristic acid) at the N-terminus and an attachement site for the long-chain fatty acid palmitic acid from the cellular Lyn kinase fused to the monomerized (m) YFP via a short linker (Fig. 7b).

The Lyn-mYFP construct is as follows:

**ccgcggaccATGGGATGTATTAAATCAAAAAGGAAAGACAATCTCAATGACGATGAACCGGT**
**CGCCACC**ATGGTG (SEQ ID NO: 46)

(small letters: Kozak sequence, ATG: start codon of Lyn kinase, in bold: myristoylation and palmitoylation signal from Lyn kinase, in **bold** and underlined: linker, normal typeface: start of mYFP/mCer). Translation into protein:

**MG*C*IKSKRKDNLNDDEPVAT**MV (SEQ ID NO: 47)

(myristoylated glycine and palmitoylated cysteine residue marked with asterisk).

2.3 Raft-marker Mem-mYFP

The raft-marker Mem-mYFP construct includes two attachment sites for the long-chain fatty acid palmitic acid from the cellular protein GAP43 at the N-terminus, fused to the monomerized YFP via a short linker (Fig. 7c).

The raft-marker Mem-mYFP construct is as follows:

**accATGCTGTGCTGTATGAGAAGAACCAAACAGGTTGAAAAGAATGATGAGGACCAACCGGT**
**CGCCACC**ATGGTG (SEQ ID NO: 48)

(small letters: Kozak sequence, ATG: start codon of Lyn kinase, in **bold:** palmitoylation signal from GAP43, in **bold** and underlined: linker, normal typeface: start of mYFP/mCer). Translation into protein:

**MLC*C*MRRTKQVEKNDEDQPVAT**MV (SEQ ID NO: 49)

(palmitoylated cysteine residues marked with asterisk).

2.4 HA-mCer, HA-mYFP, M3-HA-mCer, M3-HA-mYFP, Lyn-mCer, Lyn-mYFP, Mem-mCer, and Mem-mYFP

The construction of HA-mCer, HA-mYFP, M3-HA-mCer, M3-HA-mYFP, Lyn-mCer, Lyn-mYFP, Mem-mCer, and Mem-mYFP was carried out using the plasmids pEYFP-N1 and pECFP-N1 (Clontech). mCerulean-C1 (mCer) was provided by D.W. Piston (Rizzo *et al.* 2004). mCerulean is an improved cyan-fluorescent protein variant and was ligated into the pECFP-N1 vector using the *Age*I and *BsrG*I restriction sites, which are located directly before the start codon and near the stop codon of mCer and CFP.

The A206K monomeric mutation (Zacharias *et al.* 2002) in mYFP was made using the primers given below following the manufactures protocol of Quickchange site-directed mutagenesis kit (Stratagene):
GGGGTCTTTGCTCAGTTTGGACTGGTAGCTCAG (SEQ ID NO: 50)
sense primer
CTGAGCTACCAGTCCAAACTGAGCAAAGACCCC (SEQ ID NO: 51)
antisense primer

The HA constructs wt-HA and M3-HA were obtained as single PCR fragments using as a template the cDNA encoding the HA gene from influenza virus strain A/FPV/Rostock/34 (H7N1) and the cDNA encoding the HA gene from influenza virus strain A/FPV/Rostock/34 (H7N1) mutated at postions 551, 559, 562 (C→S).

For the PCR amplification the following primers were used for both constructs:
CTCAGATCTCATGAACACTCAAATCCTGGTTTTC (SEQ ID NO: 52)
sense primer
GACCCGCGGAGCTTCAGGCCGGAGTATACAAATAGTGCACCG (SEQ ID NO: 53)
antisense primer for wt HA
GACCCGCGGAGCTTCAGGCCGGAGTATAGAAATAGTGGACCG (SEQ ID NO: 54)
antisense primer for M3 HA

The PCR fragments were ligated into the plasmid using the *Bgl*II and *Sac*II restriction sites within the MCS. The Lyn- and the Mem-peptides were designed as oligonucleotides. For Mem the following oligonucleotides were used:
GGGCCGCGGATGCTGTGCTGTATGAGAAGAACCAAACAGGTTGAAAAGAATGATGAGGACCA ACCGGTCAAA (SEQ ID NO: 55)
sense oligonucleotide
TTTGACCGGTTGGTCCTCATCATTCTTTTCAACCTGTTTGGTTCTTCTCATACAGCACAGCA TCCGCGGCCC (SEQ ID NO: 56)
antisense oligonucleotide

For Lyn the following oligonucleotides were used:
GGGCCGCGGACCATGGGATGTATTAAATCAAAAAGGAAAGACAATCTCAATGACGATGAACC GGTCCCC (SEQ ID NO: 57)
sense oligonucleotide
GGGGACCGGTTCATCGTCATTGAGATTGTCTTTCCTTTTTGATTTAATACATCCCATGGTCC GCGGCCC (SEQ ID NO: 58)
antisense oligonucleotide

These oligonucleotides were ligated into the vectors mCerulean-N1 and mYFP-N1 using the *Sac*II and *Age*I restriction sites within the MCS. Amplification of the plasmids was carried out in *E.coli* (chemically competent, XL-1 blue).

Transfection of CHO-K1 cells was performed with Lipofectamine 2000 transfection reagent (Invitrogen). The cells were analyzed for expression of the fluorescent protein 20-24 h after transfection using a confocal microscope Olympus FluoView 1000.

2.5 Microscopic and biochemical studies

Confocal fluorescence microscopy showed that in CHO cells transiently expressing HA-mCer (Fig. 8a) or stably expressing Mem-mYFP (Fig. 8b) or Lyn-mYFP (Fig. 8c), as well as MDCK cells stably expressing Mem-mYFP (Fig. 8d) or Lyn-mYFP (Fig. 8e) the proteins localized at the plasma membrane and in the Golgi apparatus, i.e. at intracellular sites where also the endogenous non-labelled proteins are located.

After transfection of HA-mCer and M3-HA-mCer and labelling of CHO cells with [$^{35}$S]methionine, the immunoprecipitated proteins were subjected to SDS-PAGE under reduced and non-reduced conditions and to fluorography. Figure 9a shows that both the wild type HA-mCer and the M3-HA-mCer mutant are proteolytically cleaved into $HA_1$ and $HA_2$-mCer.

In the experiment underlying Figure 9b CHO cells were transfected with HA-mCer and M3-HA-mCer and labelled with [$^{35}$S]methionine. Prior to SDS-PAGE and fluorography the immunoprecipitated proteins were digested with endoglycosidase H or PNGase F or remained undigested (-) and were subjected to SDS-PAGE and fluorography.

In the experiment underlying Figure 9c CHO cells were transfected with HA-mCer and M3-HA-mCer and labelled with [$^{35}$S]methionine or [$^{3}$H]palmitic acid. Proteins were immunoprecipitated and analysed for an incorporation of labelled fatty acid by SDS-PAGE and fluorography. As shown in Figure 9c, [$^{3}$H]palmitic acid is incorporated into wild-type HA-mCer, but not into the mutant M3-HA-mCer.

The results shown in Figures 9a to 9c demonstrate that with regard to proteolytic cleavage, glycosylation and palmitoylation, i.e. activities related to infectivity, HA-mCer behaves like the native HA.

2.6 FRET and FLIM analysis

FRET and FLIM analysis was carried out as described above (see section 1.5 of Example 1 above).

Figures 10a and 10b shows the interaction between HA-mCer and Lyn-mYFP making use of an intensity enhancement of the donor signal after bleaching of the acceptor. Emission of HA-mCer (Fig. 10a, left panel) and Lyn-mYFP (Fig. 10a, right panel) in CHO cells transiently expressing these proteins was detected prior to bleaching. It is shown that both HA-mCer and Lyn-mYFP was expressed in three different cells. After bleaching, the cell marked by a circle did not show any YFP emission, but mCer emission was increased. In contrast, non-bleached cells did not show any change in mCer or YFP emission.

Figure 11 shows the lifetime of fluorescence of HA-mCer in the presence and absence of raft-marker Lyn-mYFP.

EXAMPLE 3: Studies on matrix protein M 1 membrane association

3.1 CFP-M1-NES

The CFP-M1-NES construct includes at the C-terminus an amino acid sequence from Rev protein of the human immunodeficiency virus (HIV-1) responsible for the export from the nucleus (nuclear export signal, NES) and, in addition, the CFP fused to the N-terminus of M1 (Fig. 12a).

To generate the CFP-M1-NES construct, M1 including the NES from Rev (HIV-1) was amplified using PCR and cloned into the MCS of the vector pECFP-N1 making use of the *Xho*I and *Kpn*I restriction sites. As a result, a linker was formed located between the C-terminus of CFP and the N-terminus of M1:
*TACAAG*TCCGGACTCAGATCTCGAGCT**ATGAGT** (SEQ ID NO: 59)
(in *italics*: end of CFP sequence, in **bold:** start of M1 sequence, normal typeface: linker sequence). Translation into protein: YKSGLRSRA**MS**) (SEQ ID NO: 60)

The C-terminus of M 1 is followed by the NES consisting of eleven amino acids having the following sequence: LQLPPLERLTL (SEQ ID NO: 61)

In more detail, the linkage between the C-terminus of M1 and the NES is as follows:
*TTCAAG***CTTCAGCTACCACCGCTTGAGAGACTTACTCTTTGA** (SEQ ID NO: 62)
(in *italics:* M1 sequence, in **bold:** NES). Translation into protein:
*FK***LQLPPLERLTL** (SEQ ID NO: 63)

For construction, the open reading frame (ORF) coding for M1 of influenza A/FPV/Rostock/34 was amplified by polymerase chain reaction (PCR) from plasmid pHH-M (Wagner *et al.* 2005) using the following primers:
GCTTACCTCGAGCTATGAGTCTTCTAACCGAGG (SEQ ID NO: 64)
GTCTCAGGTACCTCA*AAGAGTAAGTCTCTCAAGCGGTGGTAGCTGAAG*CTTGAATCGTTGC (SEQ ID NO: 65)
(underlined: recognition sequences for the restriction enzymes *Xho*I and *Kpn*I, respectively; in *italics:* nucleotide sequence encoding the NES derived from HIV-1 Rev, amino acid sequence LQLPPLERLTL as in SEQ ID NO: 61).

PCR was carried out using AccuTaq LA polymerase (Sigma-Aldrich). The PCR product and the vector pECFP-C1 (Clontech) were digested with *Xho*I and *Kpn*I (New England BioLabs) and ligated. After transformation of chemocompetent *E. coli* XL-1 blue (Stratagene), colonies were selected on YT-agar plates supplemented with 50 μg/ml kanamycin. The correctness of the obtained plasmid was checked by sequencing.

To check for expression, CHO-K1 cells were grown on coverslips and transfected with the construct using Lipofectamine 2000 (Invitrogen) according to the manufacturer's instructions. After 24 hours of expression, cells were washed with phosphate-buffered saline (PBS) and fixed with paraformaldehyde (3 % in PBS) for 10 min. Microscopy was conducted with a Leica SP2 confocal laser-scanning microscope.

3 .2 Myr-M1-NES

The Myr-M1-NES construct includes at the N-terminus a myristoylation signal (i.e. a signal for the attachment of myristic acid) from the cellular Lyn kinase, and, in addition, the CFP fused to the C-terminus of M1 (Fig. 12b).

To generate the Myr-M1-NES construct, M1 comprising the myristoylation signal from the Lyn kinase was amplified using PCR and cloned into the MCS of vector pECFP-C1 making use of the *Xho*I and *Kpn*I restriction sites.

The linkage between the N-terminus of CFP and M1 is as follows:
**ATGGGATCAATCAAATCAAAG**ATGAGTCTT (SEQ ID NO: 66)
(in **bold:** myristoylation signal derived from Lyn kinase, in normal typeface: M1 sequence). Translation into protein:
**MGSIKSK**MSL (SEQ ID NO: 67)

The linkage between the C-terminus of M1 and CFP is as follows:
TTCAAG**CCGCGGGCCCGGGATCCACCGGTCGCCACC***ATGGTGAGC* (SEQ ID NO: 68)
(normal typeface: M1 sequence, in bold: linker, in *italics:* CFP sequence). Translation into protein:

FK**PRARDPPVAT***MVS* (SEQ ID NO: 69)

For construction, the open reading frame (ORF) of M1 was amplified by PCR from plasmid pHH-M using the following primers:

GCTTACCTCGAG*ATGGGATCAATCAAATCAAAG*ATGAGTCTTCTAACC (SEQ ID NO: 70)

GTCTTACCGCGGCTTGAATCGTTGCATCTGCAC (SEQ ID NO: 71)

(underlined: recognition sequences for the restriction enzymes *Xho*I and *Sac*II, respectively; in *italics:* myristoylation sequence derived from Lyn kinase, amino acid sequence MGSIKSK as in SEQ ID NO: 67).

The PCR products were purified, digested with *Xho*I and *Kpn*I (New England BioLabs), repurified and ligated with pECFP-N1 (Clontech) digested with the same enzymes. All other procedures were the same as for CFP-M1-NES (see section 3.1 above).

3.3 Microscopic and biochemical studies

CHO-K1 cells were transfected with (a) CFP-M1 wildtype, (b) CFP-M1-NES and (c) Myr-M1-CFP, and localization of the protein was analyzed using fluorescence microscopy (Fig. 13). In contrast to M1-wildtype, predominantly found in the nucleus, CFP-M1-NES is found at intracellular membranes, and Myr-M1-CFP is found in the Golgi apparatus as well as at the plasma membrane.

CHO-K1 cells were transfected with CFP-M1 wildtype and CFP-M1-NES; non-transfected cells served as a negative control. The cells were fractionated into membrane and soluble compounds by differential centrifugation and subjected to Western blot using anti-M1 antibody (Virostat). In Fig. 14, lane (a) shows complete cell lysate, lane (b) shows the membrane fraction (100,000× g fraction), and lane (c) shows the soluble fraction (100,000× g supernatant). In contrast to largely soluble CFP-M1 wildtype, CFP-M1-NES is detected nearly exclusively in the membrane fraction.

**References**

Ali A, Avalos RT, Ponimaskin E, Nayak DP. (2000) Influenza virus assembly: effect of influenza virus glycoproteins on the membrane association of M1 protein. J Virol. 74:8709-8719.

Anderson, RG and Jacobson, K (2002) A role for lipid shells in targeting proteins to caveolae, rafts and other lipid domains. Science 296, 1821-1825.

Bacia K, Schuette CG, Kahya N, Jahn R, Schwille P. (2004) SNAREs prefer liquid-disordered over "raft" (liquid-ordered) domains when reconstituted into giant unilamellar vesicles. J Biol Chem. 279:37951-5.

Barmann, S, Ali, A, Hui, EKW, Adhikary, L and Nayak, DP (2001) Transport of viral proteins to the apical membranes and interaction of matrix protein with glycoproteins in the assembly of influenza viruses. Virus Res. 77, 61-69.

Baumgart, T, Hess, ST, Webb, WW. (2003) Imaging coexisting fluid domains in biomembrane models coupling curvature and line tension. Nature 425, 821-825.

Briggs, JAG, Wilk, T and Fuller, SD (2003) Do lipid rafts mediate virus assembly and pseudotyping? J. gen. Virol. 84, 757-768.

Carrasco, M, Amorim, MJ and Digard, P (2004) Lipid-raft dependent targeting of the influenza A virus nucleoprotein to the apical plasma membrane. Traffic 5 979-992.

Dietrich, C, Bagatolli, LA, Volovyk, ZN, Thompson, NL, Levi, M., Jacobson, K, Gratton, E. (2001) Lipid rafts reconstituted in model membranes. Biophys. J. 80,1417-1428.

Edidin, M (2003) The state of lipid rafts: from model membranes to cells. Annu Rev Biophys Biomol Struct. 32:257-83.

Epand, RM (2004) Do proteins facilitate formation of cholesterol-rich domains? Biochim. Biophys. Acta 1666, 227-238.

Fiedler, K, Kobayashi, T, Kurzchalia, TV and Simons K (1993) Glycosphingolipid-enriched, detergent-insoluble complexes in protein sorting in epithelial cells. Biochemistry 32, 6365-6373.

Gomez-Puertas P, Albo C, Perez-Pastrana E, Vivo A, Portela A. (2000) Influenza virus matrix protein is the major driving force in virus budding. J Virol. 74, 11538-11547.

Harder T, Scheiffele P, Verkade P, Simons K. (1998) Lipid domain structure of the plasma membrane revealed by patching of membrane components. J Cell Biol. 141:929-942.

Heerklotz, H (2002) Triton promotes domain formation in lipid raft mixtures. Biophys J. 83, 2693-2701.

Jin, H, Leser, GP, Zhang, J and Lamb, RA (1997) Influenza virus hemagglutinin and neuraminidase cytoplasmic tails control particle shape. EMBO. J 16, 1236-1247.

Keller, P and Simons, K (1998) Cholesterol is required for surface transport of influenza virus hemagglutinin. J. Cell Biol. 140, 1357-1367.

LaCour T, Kiemer L, Mølgaard A,Gupta R, Skriver K and Brunak S (2004) Analysis and prediction of leucine-rich nuclear export signals. Protein Engineering, Design & Selection 17 527-536.
Latham T, Galarza JM. (2001) Formation of wild-type and chimeric influenza virus-like particles following simultaneous expression of only four structural proteins. J Virol. 75:6154-6165.
Legler, D. F., Doucey, M., et al. (2005) Differential insertion of GPI-anchored GFPs into lipid rafts of live cells. The FASEB Journal 19: 73-75.
Mayor, S and Rao, M (2004) Rafts: Scale dependent, active lipid organization at the cell surface. Traffic 5, 231-240.
Melkonian KA, Ostermeyer AG, Chen JZ, Roth MG, Brown DA. (1999) Role of lipid modifications in targeting proteins to detergent-resistant membrane rafts. Many raft proteins are acylated, while few are prenylated. J Biol Chem. 274: 3910-3917.
Munro, S: (2003) Lipid rafts: elusive or illusive? Cell. 115, 377-388. Rizzo, M. A., G. H. Springer, et al. (2004) An improved cyan fluorescent protein variant useful for FRET. Nat Biotechnol 22(4): 445-9.
Scheiffele, P, Roth, MG and Simons, K (1997) Interaction of influenza virus hemagglutinin with sphingolipid-cholesterol membrane domains via its transmembrane domain. EMBO-J. 16, 5501-5508.
Scheiffele, P, Rietveld, A, Wilk, T and Simons, K (1999) Influenza virus select ordered lipid domains during budding from the plasma membrane. J. Biol. Chem. 274, 2038-2044.
Schmitt, AP and Lamb, RA (2004) Escaping from the cell: assembly and budding of negative-strand RNA viruses. Curr Top Microbiol Immunol. 283:145-196.
Schroeder C, Heider H, Moncke-Buchner E, Lin TI. (2004) The influenza virus ion channel and maturation cofactor M2 is a cholesterol-binding protein. Eur Biophys J Jun 25; [Epub ahead of print]
Sekar, RB and Periasamy, A (2003) Fluorescence resonance energy transfer (FRET) microscopy imaging of live cell protein localizations. J. Cell Biol. 160, 629-633.
Sha, B. and Luo, M (1997) Structure of a bifunctional membrane-RNA binding protein, influenza virus matrix protein M1. Nat. Struct. Biol. 4, 239-244.
Sharma, P., Varma, R., Sarasij, RC, Ira, Gousset, K., Krishnamoorthy, G., Rao, M. and Mayor, S. (2004) Nanoscale organization of multiple GPI-anchored proteins in living cell membranes. Cell 116, 577-589.
Simons, K and van Meer, G (1988) Lipid sorting in epithelial cells. Biochemistry 27, 6197-6202.
Simons K, Ikonen E. (1997) Functional rafts in cell membranes. Nature. 387:569-72.
Simons, K. and Vaz, WLC (2004) Model systems, lipid rafts and cell membranes. Annu Rev Biophys Biomol Struct 33: 269-295.
Skibens, JE; Roth, MG and Matlin, KS (1989) Differential extractability of influenza virus hemagglutinin during intracellular transport in polarized epithelial cells and nonpolar fibroblasts. J. Cell. Biol. 108, 821-832.
Tall, RD, Alonso, MA and Roth, MG (2003) Features of influenza HA required for apical sorting differ from those required for assocation with DRMs or MAL. Traffic 4, 838-849.
Takeda M, Leser GP, Russell CJ, Lamb RA. (2003) Influenza virus hemagglutinin concentrates in lipid raft microdomains for efficient viral fusion.Proc Natl Acad Sci U S A. 100:14610-7.
Varma., R and Mayor, S. (1998) GPI-anchored proteins are organized in submicron domains at the cell surface. Nature 394, 798-801.
Wagner R, Herwig A, Azzouz N, Klenk HD: Acylation-Mediated Membrane Anchoring of Avian Influenza Virus Hemagglutinin Is Essential for Fusion Pore Formation and Virus Infectivity. J Virol 79 (2005), 6449-6458.
Zacharias, DA, Violin, JD, Newton, AC and Tsien, RY (2002) Partitioning of lipid-modified monomeric GFPs into membrane microdomains of live cells. Science 296, 913-916.
Zhang, J, Pekosz, A and Lamb, RA (2000) Influenza virus assembly and lipid raft microdomains: a role for the cytoplsmic tails of the spike glycoproteins. J. Virol 74, 4634-4644
Zhao H, Ekstrom M, Garoff H. (1998) The M1 and NP proteins of influenza A virus form homo- but not heterooligomeric complexes when coexpressed in BHK-21 cells. J Gen Virol. 79 2435-46.

Annex:

| No: | DNA sequence | Amino acid sequence |
|---|---|---|
| 1 (CFP-M1-NES): | ATGGTGAGCAAGGGCGAGGAGCTGTTCACCGGGGTGGT<br>GCCCATCCTGGTCGAGCTGGACGGCGACGTAAACGGCC<br>ACAAGTTCAGCGTGTCCGGCGAGGGCGAGGGCGATGCC<br>ACCTACGGCAAGCTGACCCTGAAGTTCATCTGCACCAC<br>CGGCAAGCTGCCCGTGCCCTGGCCCACCCTCGTGACCA<br>CCCTGACCTGGGGCGTGCAGTGCTTCAGCCGCTACCCC<br>GACCACATGAAGCAGCACGACTTCTTCAAGTCCGCCAT<br>GCCCGAAGGCTACGTCCAGGAGCGCACCATCTTCTTCA<br>AGGACGACGGCAACTACAAGACCCGCGCCGAGGTGAAG<br>TTCGAGGGCGACACCCTGGTGAACCGCATCGAGCTGAA<br>GGGCATCGACTTCAAGGAGGACGGCAACATCCTGGGGC<br>ACAAGCTGGAGTACAACTACATCAGCCACAACGTCTAT<br>ATCACCGCCGACAAGCAGAAGAACGGCATCAAGGCCAA<br>CTTCAAGATCCGCCACAACATCGAGGACGGCAGCGTGC<br>AGCTCGCCGACCACTACCAGCAGAACACCCCCATCGGC<br>GACGGCCCCGTGCTGCTGCCCGACAACCACTACCTGAG<br>CACCCAGTCCGCCCTGAGCAAAGACCCCAACGAGAAGC<br>GCGATCACATGGTCCTGCTGGAGTTCGTGACCGCCGCC<br>GGGATCACTCTCGGCATGGACGAGCTGTACAAGTCCGG<br>ACTCAGATCTCGAGCT*ATGAGTCTTCTAACCGAGGTTG*<br>*AAACGTACGTTCTCTCTATCATCCCATCAGGCCCCCTC*<br>*AAAGCCGAGATCGCGCAGAGACTTGAAGATGTCTTTGC*<br>*AGGGAAAAACACAGACCTTGAGGTTCTCATGGAATGGC*<br>*TAAAGACAAGACCAATCCTGTCACCTCTGACTAAAGGG*<br>*ATTTTGGGGTTTGTGTTTACGCTCACCGTGCCCAGTGA*<br>*GCAAGGACTGCAGCGTAGACGCTTTGTCCAAAATGCCC*<br>*TAAATGGGAATGGGGATCCAAATAACATGGATAAAGCC*<br>*GTCAAACTATACAGGAAGTTGAAAAGGGAGATAACATT*<br>*CTATGGAGCTAAGGAAGTGGCACTCAGTTACTCTACTG*<br>*GAGCACTTGCCAGTTGTATGGGCCTCATATACAACAGA*<br>*ATGGGAACTGTGACCACAGAGGTGGCATTTGGCCTAGT*<br>*GTGTGCCACTTGTGAGCAGATTGCTGATTCACAGCATC*<br>*GGTCTCACAGACAGATGGTGGCTACCACCAATCCACTA*<br>*ATCAGGCATGAGAACAGAATGGTAATGGCCAGCACTAC*<br>*AGCTAAGGCTATGGAGCAAATGGCTGGGTCAATTGAAC*<br>*AGGCAGCGGAGGCCATGGAGGTTGCTAGCCAGGCTAGG*<br>*CAGATGGTGCAGGCAATGAGGACAATTGGGACTCATCC*<br>*TAGCTCCAGTGCTGGTCTGAAAGATGATCTTCTTGAAA*<br>*ATTTGCAGGCCTACCAGAAACGGATGGGAGTGCAGATG*<br>*CAACGATTCAAG***CTTCAGCTACCACCGCTTGAGAGACT**<br>**TACTCTTTGA**<br>Normal typeface: CFP, underlined: linker, italics: M1, **bold**: NES. | MVSKGEELFTGVV<br>PILVELDGDVNGH<br>KFSVSGEGEGDAT<br>YGKLTLKFICTTG<br>KLPVPWPTLVTTL<br>TWGVQCFSRYPDH<br>MKQHDFFKSAMPE<br>GYVQERTIFFKDD<br>GNYKTRAEVKFEG<br>DTLVNRIELKGID<br>FKEDGNILGHKLE<br>YNYISHNVYITAD<br>KQKNGIKANFKIR<br>HNIEDGSVQLADH<br>YQQNTPIGDGPVL<br>LPDNHYLSTQSAL<br>SKDPNEKRDHMVL<br>LEFVTAAGITLGM<br>DELYKSGLRSRA*M*<br>*SLLTEVETYVLSI*<br>*IPSGPLKAEIAQR*<br>*LEDVFAGKNTDLE*<br>*VLMEWLKTRPILS*<br>*PLTKGILGFVFTL*<br>*TVPSEQGLQRRRF*<br>*VQNALNGNGDPNN*<br>*MDKAVKLYRKLKR*<br>*EITFYGAKEVALS*<br>*YSTGALASCMGLI*<br>*YNRMGTVTTEVAF*<br>*GLVCATCEQIADS*<br>*QHRSHRQMVATTN*<br>*PLIRHENRMVMAS*<br>*TTAKAMEQMAGSI*<br>*EQAAEAMEVASQA*<br>*RQMVQAMRTIGTH*<br>*PSSSAGLKDDLLE*<br>*NLQAYQKRMGVQM*<br>*QRFK***LQLPPLERL**<br>**TL** |
| 2 (Myr-M1-CFP): | **ATGGGATCAATCAAATCAAAG***ATGAGTCTTCTAACCGA*<br>*GGTTGAAACGTACGTTCTCTCTATCATCCCATCAGGCC*<br>*CCCTCAAAGCCGAGATCGCGCAGAGACTTGAAGATGTC*<br>*TTTGCAGGGAAAAACACAGACCTTGAGGTTCTCATGGA* | **MGSIKSK***MSLLTE*<br>*VETYVLSIIPSGP*<br>*LKAEIAQRLEDVF*<br>*AGKNTDLEVLMEW* |

| | | |
|---|---|---|
| | *ATGGCTAAAGACAAGACCAATCCTGTCACCTCTGACTA*<br>*AAGGGATTTTGGGGTTTGTGTTTACGCTCACCGTGCCC*<br>*AGTGAGCAAGGACTGCAGCGTAGACGCTTTGTCCAAAA*<br>*TGCCCTAAATGGGAATGGGGATCCAAATAACATGGATA*<br>*AAGCCGTCAAACTATACAGGAAGTTGAAAAGGGAGATA*<br>*ACATTCTATGGAGCTAAGGAAGTGGCACTCAGTTACTC*<br>*TACTGGAGCACTTGCCAGTTGTATGGGCCTCATATACA*<br>*ACAGAATGGGAACTGTGACCACAGAGGTGGCATTTGGC*<br>*CTAGTGTGTGCCACTTGTGAGCAGATTGCTGATTCACA*<br>*GCATCGGTCTCACAGACAGATGGTGGCTACCACCAATC*<br>*CACTAATCAGGCATGAGAACAGAATGGTAATGGCCAGC*<br>*ACTACAGCTAAGGCTATGGAGCAAATGGCTGGGTCAAT*<br>*TGAACAGGCAGCGGAGGCCATGGAGGTTGCTAGCCAGG*<br>*CTAGGCAGATGGTGCAGGCAATGAGGACAATTGGGACT*<br>*CATCCTAGCTCCAGTGCTGGTCTGAAAGATGATCTTCT*<br>*TGAAAATTTGCAGGCCTACCAGAAACGGATGGGAGTGC*<br>*AGATGCAACGATTCAAG*CCGCGGGCCCGGGATCCACCG<br>GTCGCCACCATGGTGAGCAAGGGCGAGGAGCTGTTCAC<br>CGGGGTGGTGCCCATCCTGGTCGAGCTGGACGGCGACG<br>TAAACGGCCACAAGTTCAGCGTGTCCGGCGAGGGCGAG<br>GGCGATGCCACCTACGGCAAGCTGACCCTGAAGTTCAT<br>CTGCACCACCGGCAAGCTGCCCGTGCCCTGGCCCACCC<br>TCGTGACCACCCTGACCTGGGGCGTGCAGTGCTTCAGC<br>CGCTACCCCGACCACATGAAGCAGCACGACTTCTTCAA<br>GTCCGCCATGCCCGAAGGCTACGTCCAGGAGCGCACCA<br>TCTTCTTCAAGGACGACGGCAACTACAAGACCCGCGCC<br>GAGGTGAAGTTCGAGGGCGACACCCTGGTGAACCGCAT<br>CGAGCTGAAGGGCATCGACTTCAAGGAGGACGGCAACA<br>TCCTGGGGCACAAGCTGGAGTACAACTACATCAGCCAC<br>AACGTCTATATCACCGCCGACAAGCAGAAGAACGGCAT<br>CAAGGCCAACTTCAAGATCCGCCACAACATCGAGGACG<br>GCAGCGTGCAGCTCGCCGACCACTACCAGCAGAACACC<br>CCCATCGGCGACGGCCCCGTGCTGCTGCCCGACAACCA<br>CTACCTGAGCACCCAGTCCGCCCTGAGCAAAGACCCCA<br>ACGAGAAGCGCGATCACATGGTCCTGCTGGAGTTCGTG<br>ACCGCCGCCGGGATCACTCTCGGCATGGACGAGCTGTA<br>CAAGTAA<br>**Bold**: Myristoylation signal, *italics*: M1, underlined: linker, normal typeface: CFP | *LKTRPILSPLTKG*<br>*ILGFVFTLTVPSE*<br>*QGLQRRRFVQNAL*<br>*NGNGDPNNMDKAV*<br>*KLYRKLKREITFY*<br>*GAKEVALSYSTGA*<br>*LASCMGLIYNRMG*<br>*TVTTEVAFGLVCA*<br>*TCEQIADSQHRSH*<br>*RQMVATTNPLIRH*<br>*ENRMVMASTTAKA*<br>*MEQMAGSIEQAAE*<br>*AMEVASQARQMVQ*<br>*AMRTIGTHPSSSA*<br>*GLKDDLLENLQAY*<br>*QKRMGVQMQRFK*P<br>RARDPPVATMVSK<br>GEELFTGVVPILV<br>ELDGDVNGHKFSV<br>SGEGEGDATYGKL<br>TLKFICTTGKLPV<br>PWPTLVTTLTWGV<br>QCFSRYPDHMKQH<br>DFFKSAMPEGYVQ<br>ERTIFFKDDGNYK<br>TRAEVKFEGDTLV<br>NRIELKGIDFKED<br>GNILGHKLEYNYI<br>SHNVYITADKQKN<br>GIKANFKIRHNIE<br>DGSVQLADHYQQN<br>TPIGDGPVLLPDN<br>HYLSTQSALSKDP<br>NEKRDHMVLLEFV<br>TAAGITLGMDELY<br>K |
| 3 (Artifical-HA-YFP): | **ATGAACACTCAAATCCTGGTTTTCGCCCTTGTGGCAGT**<br>**CATTCCCACAAATGCAGACAAAATTTGTCTT**CCGCGGG<br>CCCGGGATCCACCGGTCGCCACC*ATGGTGAGCAAGGGC*<br>*GAGGAGCTGTTCACCGGGGTGGTGCCCATCCTGGTCGA*<br>*GCTGGACGGCGACGTAAACGGCCACAAGTTCAGCGTGT*<br>*CCGGCGAGGGCGAGGGCGATGCCACCTACGGCAAGCTG*<br>*ACCCTGAAGTTCATCTGCACCACCGGCAAGCTGCCCGT*<br>*GCCCTGGCCCACCCTCGTGACCACCTTCGGCTACGGCC*<br>*TGCAGTGCTTCGCCCGCTACCCCGACCACATGAAGCAG*<br>*CACGACTTCTTCAAGTCCGCCATGCCCGAAGGCTACGT*<br>*CCAGGAGCGCACCATCTTCTTCAAGGACGACGGCAACT*<br>*ACAAGACCCGCGCCGAGGTGAAGTTCGAGGGCGACACC* | **MNTQILVFALVAV**<br>**IPTNADKICL**PRA<br>RDPPVAT*MVSKGE*<br>*ELFTGVVPILVEL*<br>*DGDVNGHKFSVSG*<br>*EGEGDATYGKLTL*<br>*KFICTTGKLPVPW*<br>*PTLVTTFGYGLQC*<br>*FARYPDHMKQHDF*<br>*FKSAMPEGYVQER*<br>*TIFFKDDGNYKTR*<br>*AEVKFEGDTLVNR* |

| | | |
|---|---|---|
| | *CTGGTGAACCGCATCGAGCTGAAGGGCATCGACTTCAA*<br>*GGAGGACGGCAACATCCTGGGGCACAAGCTGGAGTACA*<br>*ACTACAACAGCCACAACGTCTATATCATGGCCGACAAG*<br>*CAGAAGAACGGCATCAAGGTGAACTTCAAGATCCGCCA*<br>*CAACATCGAGGACGGCAGCGTGCAGCTCGCCGACCACT*<br>*ACCAGCAGAACACCCCCATCGGCGACGGCCCCGTGCTG*<br>*CTGCCCGACAACCACTACCTGAGCTACCAGTCCGCCCT*<br>*GAGCAAAGACCCCAACGAGAAGCGCGATCACATGGTCC*<br>*TGCTGGAGTTCGTGACCGCCGCCGGGATCACTCTCGGC*<br>*ATGGACGAGCTGTACAAG*GCTAGTATAAGGAACAATAC<br>TTATGATCACAGCAAATACAGAGAAGAAGCGATGCAAA<br>ATAGAATACAAATTGACCCAGTCAAATTGAGTAGTGGC<br>TACAAAGATGTGATACTTTGGTTTAGCTTCGGGGCATC<br>ATGCTTTTTGCTTCTTGCCATTGCAATGGGCCTTGTTT<br>TCATATGTGTGAAGAACGGAAACATGCGGTGCACTATT<br>TGTATATAA<br><br>**Bold**: HA signal sequence, underlined: linker, in *italics*: YFP, in normal typeface: HA-TMD/CT. | *IELKGIDFKEDGN*<br>*ILGHKLEYNYNSH*<br>*NVYIMADKQKNGI*<br>*KVNFKIRHNIEDG*<br>*SVQLADHYQQNTP*<br>*IGDGPVLLPDNHY*<br>*LSYQSALSKDPNE*<br>*KRDHMVLLEFVTA*<br>*AGITLGMDELYK*A<br>SIRNNTYDHSKYR<br>EEAMQNRIQIDPV<br>KLSSGYKDVILWF<br>SFGASCFLLLAIA<br>MGLVFICVKNGNM<br>RCTICI |
| 4 (Artificial-HA-CFP): | **ATGAACACTCAAATCCTGGTTTTCGCCCTTGTGGCAGT**<br>**CATTCCCACAAATGCAGACAAAATTTGTCTT**CCGCGGG<br>CCCGGGATCCACCGGTCGCCACC*ATGGTGAGCAAGGGC*<br>*GAGGAGCTGTTCACCGGGGTGGTGCCCATCCTGGTCGA*<br>*GCTGGACGGCGACGTAAACGGCCACAAGTTCAGCGTGT*<br>*CCGGCGAGGGCGAGGGCGATGCCACCTACGGCAAGCTG*<br>*ACCCTGAAGTTCATCTGCACCACCGGCAAGCTGCCCGT*<br>*GCCCTGGCCCACCCTCGTGACCACCCTGACCTGGGGCG*<br>*TGCAGTGCTTCAGCCGCTACCCCGACCACATGAAGCAG*<br>*CACGACTTCTTCAAGTCCGCCATGCCCGAAGGCTACGT*<br>*CCAGGAGCGCACCATCTTCTTCAAGGACGACGGCAACT*<br>*ACAAGACCCGCGCCGAGGTGAAGTTCGAGGGCGACACC*<br>*CTGGTGAACCGCATCGAGCTGAAGGGCATCGACTTCAA*<br>*GGAGGACGGCAACATCCTGGGGCACAAGCTGGAGTACA*<br>*ACTACATCAGCCACAACGTCTATATCACCGCCGACAAG*<br>*CAGAAGAACGGCATCAAGGCCAACTTCAAGATCCGCCA*<br>*CAACATCGAGGACGGCAGCGTGCAGCTCGCCGACCACT*<br>*ACCAGCAGAACACCCCCATCGGCGACGGCCCCGTGCTG*<br>*CTGCCCGACAACCACTACCTGAGCACCCAGTCCGCCCT*<br>*GAGCAAAGACCCCAACGAGAAGCGCGATCACATGGTCC*<br>*TGCTGGAGTTCGTGACCGCCGCCGGGATCACTCTCGGC*<br>*ATGGACGAGCTGTACAAG*GCTAGTATAAGGAACAATAC<br>TTATGATCACAGCAAATACAGAGAAGAAGCGATGCAAA<br>ATAGAATACAAATTGACCCAGTCAAATTGAGTAGTGGC<br>TACAAAGATGTGATACTTTGGTTTAGCTTCGGGGCATC<br>ATGCTTTTTGCTTCTTGCCATTGCAATGGGCCTTGTTT<br>TCATATGTGTGAAGAACGGAAACATGCGGTGCACTATT<br>TGTATATAA<br><br>**Bold**: HA signal sequence, underlined: linker, in *italics*: CFP, in normal ty- | **MNTQILVFALVAV**<br>**IPTNADKICL**PRA<br>RDPPVAT*MVSKGE*<br>*ELFTGVVPILVEL*<br>*DGDVNGHKFSVSG*<br>*EGEGDATYGKLTL*<br>*KFICTTGKLPVPW*<br>*PTLVTTLTWGVQC*<br>*FSRYPDHMKQHDF*<br>*FKSAMPEGYVQER*<br>*TIFFKDDGNYKTR*<br>*AEVKFEGDTLVNR*<br>*IELKGIDFKEDGN*<br>*ILGHKLEYNYISH*<br>*NVYITADKQKNGI*<br>*KANFKIRHNIEDG*<br>*SVQLADHYQQNTP*<br>*IGDGPVLLPDNHY*<br>*LSTQSALSKDPNE*<br>*KRDHMVLLEFVTA*<br>*AGITLGMDELYK*A<br>SIRNNTYDHSKYR<br>EEAMQNRIQIDPV<br>KLSSGYKDVILWF<br>SFGASCFLLLAIA<br>MGLVFICVKNGNM<br>RCTICI |

| | | |
|---|---|---|
| | peface: HA-TMD/CT. | |
| 5 (Artifi-cal-HA-YFPM3): | **ATGAACACTCAAATCCTGGTTTTCGCCCTTGTGGCAGT**<br>**CATTCCCACAAATGCAGACAAAATTTGTCTT**CCGCGGG<br>CCCGGGATCCACCGGTCGCCACC*ATGGTGAGCAAGGGC*<br>*GAGGAGCTGTTCACCGGGGTGGTGCCCATCCTGGTCGA*<br>*GCTGGACGGCGACGTAAACGGCCACAAGTTCAGCGTGT*<br>*CCGGCGAGGGCGAGGGCGATGCCACCTACGGCAAGCTG*<br>*ACCCTGAAGTTCATCTGCACCACCGGCAAGCTGCCCGT*<br>*GCCCTGGCCCACCCTCGTGACCACCTTCGGCTACGGCC*<br>*TGCAGTGCTTCGCCCGCTACCCCGACCACATGAAGCAG*<br>*CACGACTTCTTCAAGTCCGCCATGCCCGAAGGCTACGT*<br>*CCAGGAGCGCACCATCTTCTTCAAGGACGACGGCAACT*<br>*ACAAGACCCGCGCCGAGGTGAAGTTCGAGGGCGACACC*<br>*CTGGTGAACCGCATCGAGCTGAAGGGCATCGACTTCAA*<br>*GGAGGACGGCAACATCCTGGGGCACAAGCTGGAGTACA*<br>*ACTACAACAGCCACAACGTCTATATCATGGCCGACAAG*<br>*CAGAAGAACGGCATCAAGGTGAACTTCAAGATCCGCCA*<br>*CAACATCGAGGACGGCAGCGTGCAGCTCGCCGACCACT*<br>*ACCAGCAGAACACCCCCATCGGCGACGGCCCCGTGCTG*<br>*CTGCCCGACAACCACTACCTGAGCTACCAGTCCGCCCT*<br>*GAGCAAAGACCCCAACGAGAAGCGCGATCACATGGTCC*<br>*TGCTGGAGTTCGTGACCGCCGCCGGGATCACTCTCGGC*<br>*ATGGACGAGCTGTACAAG*GCTAGTATAAGGAACAATAC<br>TTATGATCACAGCAAATACAGAGAAGAAGCGATGCAAA<br>ATAGAATACAAATTGACCCAGTCAAATTGAGTAGTGGC<br>TACAAAGATGTGATACTTTGGTTTAGCTTCGGGGCATC<br>ATGCTTTTTGCTTCTTGCCATTGCAATGGGCCTTGTTT<br>TCATATCTGTGAAGAACGGAAACATGCGGTCCACTATT<br>TCTATATAA<br><br>**Bold**: HA signal peptide, underlined: linker, in *italics*: YFP, underlined: residues changed for Cys → Ser mutation. | MNTQILVFALVAV<br>IPTNADKICLPRA<br>RDPPVAT*MVSKGE*<br>*ELFTGVVPILVEL*<br>*DGDVNGHKFSVSG*<br>*EGEGDATYGKLTL*<br>*KFICTTGKLPVPW*<br>*PTLVTTFGYGLQC*<br>*FARYPDHMKQHDF*<br>*FKSAMPEGYVQER*<br>*TIFFKDDGNYKTR*<br>*AEVKFEGDTLVNR*<br>*IELKGIDFKEDGN*<br>*ILGHKLEYNYNSH*<br>*NVYIMADKQKNGI*<br>*KVNFKIRHNIEDG*<br>*SVQLADHYQQNTP*<br>*IGDGPVLLPDNHY*<br>*LSYQSALSKDPNE*<br>*KRDHMVLLEFVTA*<br>*AGITLGMDELYK*A<br>SIRNNTYDHSKYR<br>EEAMQNRIQIDPV<br>KLSSGYKDVILWF<br>SFGASCFLLLAIA<br>MGLVFISVKNGNM<br>RSTISI |
| 6 (Artifi-cal-HA-CFPM3): | **ATGAACACTCAAATCCTGGTTTTCGCCCTTGTGGCAGT**<br>**CATTCCCACAAATGCAGACAAAATTTGTCTT**CCGCGGG<br>CCCGGGATCCACCGGTCGCCACC*ATGGTGAGCAAGGGC*<br>*GAGGAGCTGTTCACCGGGGTGGTGCCCATCCTGGTCGA*<br>*GCTGGACGGCGACGTAAACGGCCACAAGTTCAGCGTGT*<br>*CCGGCGAGGGCGAGGGCGATGCCACCTACGGCAAGCTG*<br>*ACCCTGAAGTTCATCTGCACCACCGGCAAGCTGCCCGT*<br>*GCCCTGGCCCACCCTCGTGACCACCCTGACCTGGGGCG*<br>*TGCAGTGCTTCAGCCGCTACCCCGACCACATGAAGCAG*<br>*CACGACTTCTTCAAGTCCGCCATGCCCGAAGGCTACGT*<br>*CCAGGAGCGCACCATCTTCTTCAAGGACGACGGCAACT*<br>*ACAAGACCCGCGCCGAGGTGAAGTTCGAGGGCGACACC*<br>*CTGGTGAACCGCATCGAGCTGAAGGGCATCGACTTCAA*<br>*GGAGGACGGCAACATCCTGGGGCACAAGCTGGAGTACA*<br>*ACTACATCAGCCACAACGTCTATATCACCGCCGACAAG*<br>*CAGAAGAACGGCATCAAGGCCAACTTCAAGATCCGCCA*<br>*CAACATCGAGGACGGCAGCGTGCAGCTCGCCGACCACT*<br>*ACCAGCAGAACACCCCCATCGGCGACGGCCCCGTGCTG* | **MNTQILVFALVAV**<br>**IPTNADKICL**PRA<br>RDPPVAT*MVSKGE*<br>*ELFTGVVPILVEL*<br>*DGDVNGHKFSVSG*<br>*EGEGDATYGKLTL*<br>*KFICTTGKLPVPW*<br>*PTLVTTLTWGVQC*<br>*FSRYPDHMKQHDF*<br>*FKSAMPEGYVQER*<br>*TIFFKDDGNYKTR*<br>*AEVKFEGDTLVNR*<br>*IELKGIDFKEDGN*<br>*ILGHKLEYNYISH*<br>*NVYITADKQKNGI*<br>*KANFKIRHNIEDG*<br>*SVQLADHYQQNTP*<br>*IGDGPVLLPDNHY* |

| | | |
|---|---|---|
| | *CTGCCCGACAACCACTACCTGAGCACCCAGTCCGCCCT*<br>*GAGCAAAGACCCCAACGAGAAGCGCGATCACATGGTCC*<br>*TGCTGGAGTTCGTGACCGCCGCCGGGATCACTCTCGGC*<br>*ATGGACGAGCTGTACAAG*GCTAGTATAAGGAACAATAC<br>TTATGATCACAGCAAATACAGAGAAGAAGCGATGCAAA<br>ATAGAATACAAATTGACCCAGTCAAATTGAGTAGTGGC<br>TACAAAGATGTGATACTTTGGTTTAGCTTCGGGGCATC<br>ATGCTTTTTGCTTCTTGCCATTGCAATGGGCCTTGTTT<br>TCATATCTGTGAAGAACGGAAACATGCGGTCCACTATT<br>TCTATATAA<br>**Bold**: HA signal peptide, underlined: linker, in *italics*: CFP, underlined: residues changed for Cys → Ser mutation. | *LSTQSALSKDPNE*<br>*KRDHMVLLEFVTA*<br>*AGITLGMDELYK*A<br>SIRNNTYDHSKYR<br>EEAMQNRIQIDPV<br>KLSSGYKDVILWF<br>SFGASCFLLLAIA<br>MGLVFISVKNGNM<br>RSTISI |
| 7 (GPI(DAF) -CFP): | **ATGAACACTCAAATCCTGGTTTTCGCCCTTGTGGCAGT**<br>**CATTCCCACAAATGCAGACAAAATTTGTCTT**CCGCGGG<br>CCCGGGATCCACCGGTCGCCACC*ATGGTGAGCAAGGGC*<br>*GAGGAGCTGTTCACCGGGGTGGTGCCCATCCTGGTCGA*<br>*GCTGGACGGCGACGTAAACGGCCACAAGTTCAGCGTGT*<br>*CCGGCGAGGGCGAGGGCGATGCCACCTACGGCAAGCTG*<br>*ACCCTGAAGTTCATCTGCACCACCGGCAAGCTGCCCGT*<br>*GCCCTGGCCCACCCTCGTGACCACCCTGACCTGGGGCG*<br>*TGCAGTGCTTCAGCCGCTACCCCGACCACATGAAGCAG*<br>*CACGACTTCTTCAAGTCCGCCATGCCCGAAGGCTACGT*<br>*CCAGGAGCGCACCATCTTCTTCAAGGACGACGGCAACT*<br>*ACAAGACCCGCGCCGAGGTGAAGTTCGAGGGCGACACC*<br>*CTGGTGAACCGCATCGAGCTGAAGGGCATCGACTTCAA*<br>*GGAGGACGGCAACATCCTGGGGCACAAGCTGGAGTACA*<br>*ACTACATCAGCCACAACGTCTATATCACCGCCGACAAG*<br>*CAGAAGAACGGCATCAAGGCCAACTTCAAGATCCGCCA*<br>*CAACATCGAGGACGGCAGCGTGCAGCTCGCCGACCACT*<br>*ACCAGCAGAACACCCCCATCGGCGACGGCCCCGTGCTG*<br>*CTGCCCGACAACCACTACCTGAGCACCCAGTCCGCCCT*<br>*GAGCAAAGACCCCAACGAGAAGCGCGATCACATGGTCC*<br>*TGCTGGAGTTCGTGACCGCCGCCGGGATCACTCTCGGC*<br>*ATGGACGAGCTGTACAAG*CCAAATAAAGGAAGTGGAAC<br>CACTTCAGGTACTACCCGTCTTCTATCTGGGCACACGT<br>GTTTCACGTTGACAGGTTTGCTTGGGACGCTAGTAACC<br>ATGGGCTTGCTGACTTA<br>**Bold**: HA signal peptide, underlined: linker, *italics*: CFP, normal typeface: GPI anchor sequence derived from DAF. | **MNTQILVFALVAV**<br>**IPTNADKICL**PRA<br>RDPPVAT*MVSKGE*<br>*ELFTGVVPILVEL*<br>*DGDVNGHKFSVSG*<br>*EGEGDATYGKLTL*<br>*KFICTTGKLPVPW*<br>*PTLVTTLTWGVQC*<br>*FSRYPDHMKQHDF*<br>*FKSAMPEGYVQER*<br>*TIFFKDDGNYKTR*<br>*AEVKFEGDTLVNR*<br>*IELKGIDFKEDGN*<br>*ILGHKLEYNYISH*<br>*NVYITADKQKNGI*<br>*KANFKIRHNIEDG*<br>*SVQLADHYQQNTP*<br>*IGDGPVLLPDNHY*<br>*LSTQSALSKDPNE*<br>*KRDHMVLLEFVTA*<br>*AGITLGMDELYK*P<br>NKGSGTTSGTTRL<br>LSGHTCFTLTGLL<br>GTLVTMGLLT |
| 8 (GPI(DAF) -YFP): | **ATGAACACTCAAATCCTGGTTTTCGCCCTTGTGGCAGT**<br>**CATTCCCACAAATGCAGACAAAATTTGTCTT**CCGCGGG<br>CCCGGGATCCACCGGTCGCCACC*ATGGTGAGCAAGGGC*<br>*GAGGAGCTGTTCACCGGGGTGGTGCCCATCCTGGTCGA*<br>*GCTGGACGGCGACGTAAACGGCCACAAGTTCAGCGTGT*<br>*CCGGCGAGGGCGAGGGCGATGCCACCTACGGCAAGCTG*<br>*ACCCTGAAGTTCATCTGCACCACCGGCAAGCTGCCCGT*<br>*GCCCTGGCCCACCCTCGTGACCACCTTCGGCTACGGCC*<br>*TGCAGTGCTTCGCCCGCTACCCCGACCACATGAAGCAG*<br>*CACGACTTCTTCAAGTCCGCCATGCCCGAAGGCTACGT* | **MNTQILVFALVAV**<br>**IPTNADKICL**PRA<br>RDPPVAT*MVSKGE*<br>*ELFTGVVPILVEL*<br>*DGDVNGHKFSVSG*<br>*EGEGDATYGKLTL*<br>*KFICTTGKLPVPW*<br>*PTLVTTFGYGLQC*<br>*FARYPDHMKQHDF*<br>*FKSAMPEGYVQER* |

| | | |
|---|---|---|
| | *CCAGGAGCGCACCATCTTCTTCAAGGACGACGGCAACT ACAAGACCCGCGCCGAGGTGAAGTTCGAGGGCGACACC CTGGTGAACCGCATCGAGCTGAAGGGCATCGACTTCAA GGAGGACGGCAACATCCTGGGGCACAAGCTGGAGTACA ACTACAACAGCCACAACGTCTATATCATGGCCGACAAG CAGAAGAACGGCATCAAGGTGAACTTCAAGATCCGCCA CAACATCGAGGACGGCAGCGTGCAGCTCGCCGACCACT ACCAGCAGAACACCCCCATCGGCGACGGCCCCGTGCTG CTGCCCGACAACCACTACCTGAGCTACCAGTCCGCCCT GAGCAAAGACCCCAACGAGAAGCGCGATCACATGGTCC TGCTGGAGTTCGTGACCGCCGCCGGGATCACTCTCGGC ATGGACGAGCTGTACAAG*CCAAATAAAGGAAGTGGAAC CACTTCAGGTACTACCCGTCTTCTATCTGGGCACACGT GTTTCACGTTGACAGGTTTGCTTGGGACGCTAGTAACC ATGGGCTTGCTGACTTA<br>**Bold**: HA signal peptide, underlined: linker, *italics*: YFP, normal typeface: GPI anchor sequence derived from DAF. | *TIFFKDDGNYKTR AEVKFEGDTLVNR IELKGIDFKEDGN ILGHKLEYNYNSH NVYIMADKQKNGI KVNFKIRHNIEDG SVQLADHYQQNTP IGDGPVLLPDNHY LSYQSALSKDPNE KRDHMVLLEFVTA AGITLGMDELYK*P NKGSGTTSGTTRL LSGHTCFTLTGLL GTLVTMGLLT |
| 9 (HA-mCer): | *ATGAACACTCAAATCCTGGTTTTCGCCCTTGTGGCAGT CATTCCCACAAATGCAGACAAAATTTGTCTTGGACATC ATGCTGTATCAAATGGCACCAAAGTAAACACACTCACT GAGAGAGGAGTAGAAGTTGTCAATGCAACGGAAACAGT GGAGCGGACAAACATCCCCAAAATTTGCTCAAAAGGGA AAAGAACCACTGATCTTGGCCAATGCGGACTGTTAGGG ACCATTACCGGACCACCTCAATGCGACCAATTTCTAGA ATTTTCAGCTGATCTAATAATCGAGAGACGAGAAGGAA ATGATGTTTGTTACCCGGGGAAGTTTGTTAATGAAGAG GCATTGCGACAAATCCTCAGAGGATCAGGTGGGATTGA CAAAGAAACAATGGGATTCACATATAGTGGAATAAGGA CCAACGGAACAACTAGTGCATGTAGAAGATCAGGGTCT TCATTCTATGCAGAAATGGAGTGGCTCCTGTCAAATAC AGACAATGCTTCTTTCCCACAAATGACAAAATCATACA AAAACACAAGGAGAGAATCAGCTCTGATAGTATGGGGA ATCCACCATTCAGGATCAACCACCGAACAGACCAAACT ATATGGGAGTGGAAATAAACTGATAACAGTCGGGAGTT CCAAATATCATCAATCTTTTGTGCCGAGTCCAGGAACA CGACCGCAGATAAATGGCCAGTCCGGACGGATTGATTT TCATTGGTTGATCTTGGATCCCAATGATACAGTTACTT TTAGTTTCAATGGGGCTTTCATAGCTCCAAATCGTGCC AGCTTCTTGAGGGGAAAGTCCATGGGGATCCAGAGCGA TGTGCAGGTTGATGCCAATTGCGAAGGGGAATGCTACC ACAGTGGAGGGACTATAACAAGCAGATTGCCTTTTCAA AACATCAATAGCAGAGCAGTTGGCAAATGCCCAAGATA TGTAAAACAGGAAAGTTTATTATTGGCAACTGGGATGA AGAACGTTCCCGAACCTTCCAAAAAAAGGAAAAAAAGA GGCCTGTTTGGCGCTATAGCAGGGTTTATTGAAAATGG TTGGGAAGGTCTGGTCGACGGGTGGTACGGTTTCAGGC ATCAGAATGCACAAGGAGAAGGAACTGCAGCAGACTAC AAAAGCACCCAATCGGCAATTGATCAGATAACCGGAAA GTTAAATAGACTCATTGAGAAAACCAACCAGCAATTTG AGCTAATAGATAATGAATTCACTGAGGTGGAAAAGCAG ATTGGCAATTTAATTAACTGGACCAAAGACTCCATCAC* | *MNTQILVFALVAV IPTNADKICLGHH AVSNGTKVNTLTE RGVEVVNATETVE RTNIPKICSKGKR TTDLGQCGLLGTI TGPPQCDQFLEFS ADLIIERREGNDV CYPGKFVNEEALR QILRGSGGIDKET MGFTYSGIRTNGT TSACRRSGSSFYA EMEWLLSNTDNAS FPQMTKSYKNTRR ESALIVWGIHHSG STTEQTKLYGSGN KLITVGSSKYHQS FVPSPGTRPQING QSGRIDFHWLILD PNDTVTFSFNGAF IAPNRASFLRGKS MGIQSDVQVDANC EGECYHSGGTITS RLPFQNINSRAVG KCPRYVKQESLLL ATGMKNVPEPSKK RKKRGLFGAIAGF IENGWEGLVDGWY GFRHQNAQGEGTA ADYKSTQSAIDQI TGKLNRLIEKTNQ QFELIDNEFTEVE KQIGNLINWTKDS ITEVWSYNAELIV* |

| | | |
|---|---|---|
| | *AGAAGTATGGTCTTACAATGCTGAACTTATTGTGGCAA*<br>*TGGAAAACCAGCACACTATTGATTTGGCTGATTCAGAG*<br>*ATGAACAGGCTGTATGAGCGAGTGAGGAAACAATTAAG*<br>*GGAAAATGCTGAAGAGGATGGTACTGGTTGCTTTGAAA*<br>*TTTTTCATAAATGTGACGATGATTGTATGGCTAGTATA*<br>*AGGAACAATACTTATGATCACAGCAAATACAGAGAAGA*<br>*AGCGATGCAAAATAGAATACAAATTGACCCAGTCAAAT*<br>*TGAGTAGTGGCTACAAAGATGTGATACTTTGGTTTAGC*<br>*TTCGGGGCATCATGCTTTTTGCTTCTTGCCATTGCAAT*<br>*GGGCCTTGTTTTCATATGTGTGAAGAACGGAAACATGC*<br>*GGTGCACTATTTGTATA***CTCCGGCCTGAAGCTCCGCGG**<br>**GCCCGGGATCCACCGGTCGCCACC**ATGGTGAGCAAGGG<br>CGAGGAGCTGTTCACCGGGGTGGTGCCCATCCTGGTCG<br>AGCTGGACGGCGACGTAAACGGCCACAAGTTCAGCGTG<br>TCCGGCGAGGGCGAGGGCGATGCCACCTACGGCAAGCT<br>GACCCTGAAGTTCATCTGCACCACCGGCAAGCTGCCCG<br>TGCCCTGGCCCACCCTCGTGACCACCCTGACCTGGGGC<br>GTGCAGTGCTTCGCCCGCTACCCCGACCACATGAAGCA<br>GCACGACTTCTTCAAGTCCGCCATGCCCGAAGGCTACG<br>TCCAGGAGCGCACCATCTTCTTCAAGGACGACGGCAAC<br>TACAAGACCCGCGCCGAGGTGAAGTTCGAGGGCGACAC<br>CCTGGTGAACCGCATCGAGCTGAAGGGCATCGACTTCA<br>AGGAGGACGGCAACATCCTGGGGCACAAGCTGGAGTAC<br>AACGCCATCAGCGACAACGTCTATATCACCGCCGACAA<br>GCAGAAGAACGGCATCAAGGCCAACTTCAAGATCCGCC<br>ACAACATCGAGGACGGCAGCGTGCAGCTCGCCGACCAC<br>TACCAGCAGAACACCCCCATCGGCGACGGCCCCGTGCT<br>GCTGCCCGACAACCACTACCTGAGCACCCAGTCCAAAC<br>TGAGCAAAGACCCCAACGAGAAGCGCGATCACATGGTC<br>CTGCTGGAGTTCGTGACCGCCGCCGGGATCACTCTCGG<br>CATGGACGAGCTGTACAAGTAA<br>*Italics*: HA, **bold:** linker, normal typeface: mCer. | *AMENQHTIDLADS*<br>*EMNRLYERVRKQL*<br>*RENAEEDGTGCFE*<br>*IFHKCDDDCMASI*<br>*RNNTYDHSKYREE*<br>*AMQNRIQIDPVKL*<br>*SSGYKDVILWFSF*<br>*GASCFLLLAIAMG*<br>*LVFICVKNGNMRC*<br>*TICI***LRPEAPRAR**<br>**DPPVAT**MVSKGEE<br>LFTGVVPILVELD<br>GDVNGHKFSVSGE<br>GEGDATYGKLTLK<br>FICTTGKLPVPWP<br>TLVTTLTWGVQCF<br>ARYPDHMKQHDFF<br>KSAMPEGYVQERT<br>IFFKDDGNYKTRA<br>EVKFEGDTLVNRI<br>ELKGIDFKEDGNI<br>LGHKLEYNAISDN<br>VYITADKQKNGIK<br>ANFKIRHNIEDGS<br>VQLADHYQQNTPI<br>GDGPVLLPDNHYL<br>STQSALSKDPNEK<br>RDHMVLLEFVTAA<br>GITLGMDELYK |
| 10 (HA-mYFP): | *ATGAACACTCAAATCCTGGTTTTCGCCCTTGTGGCAGT*<br>*CATTCCCACAAATGCAGACAAAATTTGTCTTGGACATC*<br>*ATGCTGTATCAAATGGCACCAAAGTAAACACACTCACT*<br>*GAGAGAGGAGTAGAAGTTGTCAATGCAACGGAAACAGT*<br>*GGAGCGGACAAACATCCCCAAAATTTGCTCAAAAGGGA*<br>*AAAGAACCACTGATCTTGGCCAATGCGGACTGTTAGGG*<br>*ACCATTACCGGACCACCTCAATGCGACCAATTTCTAGA*<br>*ATTTTCAGCTGATCTAATAATCGAGAGACGAGAAGGAA*<br>*ATGATGTTTGTTACCCGGGGAAGTTTGTTAATGAAGAG*<br>*GCATTGCGACAAATCCTCAGAGGATCAGGTGGGATTGA*<br>*CAAAGAAACAATGGGATTCACATATAGTGGAATAAGGA*<br>*CCAACGGAACAACTAGTGCATGTAGAAGATCAGGGTCT*<br>*TCATTCTATGCAGAAATGGAGTGGCTCCTGTCAAATAC*<br>*AGACAATGCTTCTTTCCCACAAATGACAAAATCATACA*<br>*AAAACACAAGGAGAGAATCAGCTCTGATAGTATGGGGA*<br>*ATCCACCATTCAGGATCAACCACCGAACAGACCAAACT*<br>*ATATGGGAGTGGAAATAAACTGATAACAGTCGGGAGTT*<br>*CCAAATATCATCAATCTTTTGTGCCGAGTCCAGGAACA*<br>*CGACCGCAGATAAATGGCCAGTCCGGACGGATTGATTT* | *MNTQILVFALVAV*<br>*IPTNADKICLGHH*<br>*AVSNGTKVNTLTE*<br>*RGVEVVNATETVE*<br>*RTNIPKICSKGKR*<br>*TTDLGQCGLLGTI*<br>*TGPPQCDQFLEFS*<br>*ADLIIERREGNDV*<br>*CYPGKFVNEEALR*<br>*QILRGSGGIDKET*<br>*MGFTYSGIRTNGT*<br>*TSACRRSGSSFYA*<br>*EMEWLLSNTDNAS*<br>*FPQMTKSYKNTRR*<br>*ESALIVWGIHHSG*<br>*STTEQTKLYGSGN*<br>*KLITVGSSKYHQS*<br>*FVPSPGTRPQING*<br>*QSGRIDFHWLILD* |

| | | |
|---|---|---|
| | *TCATTGGTTGATCTTGGATCCCAATGATACAGTTACTT* | *PNDTVTFSFNGAF* |
| | *TTAGTTTCAATGGGGCTTTCATAGCTCCAAATCGTGCC* | *IAPNRASFLRGKS* |
| | *AGCTTCTTGAGGGGAAAGTCCATGGGGATCCAGAGCGA* | *MGIQSDVQVDANC* |
| | *TGTGCAGGTTGATGCCAATTGCGAAGGGGAATGCTACC* | *EGECYHSGGTITS* |
| | *ACAGTGGAGGGACTATAACAAGCAGATTGCCTTTTCAA* | *RLPFQNINSRAVG* |
| | *AACATCAATAGCAGAGCAGTTGGCAAATGCCCAAGATA* | *KCPRYVKQESLLL* |
| | *TGTAAAACAGGAAAGTTTATTATTGGCAACTGGGATGA* | *ATGMKNVPEPSKK* |
| | *AGAACGTTCCCGAACCTTCCAAAAAAAGGAAAAAAAGA* | *RKKRGLFGAIAGF* |
| | *GGCCTGTTTGGCGCTATAGCAGGGTTTATTGAAAATGG* | *IENGWEGLVDGWY* |
| | *TTGGGAAGGTCTGGTCGACGGGTGGTACGGTTTCAGGC* | *GFRHQNAQGEGTA* |
| | *ATCAGAATGCACAAGGAGAAGGAACTGCAGCAGACTAC* | *ADYKSTQSAIDQI* |
| | *AAAAGCACCCAATCGGCAATTGATCAGATAACCGGAAA* | *TGKLNRLIEKTNQ* |
| | *GTTAAATAGACTCATTGAGAAAACCAACCAGCAATTTG* | *QFELIDNEFTEVE* |
| | *AGCTAATAGATAATGAATTCACTGAGGTGGAAAAGCAG* | *KQIGNLINWTKDS* |
| | *ATTGGCAATTTAATTAACTGGACCAAAGACTCCATCAC* | *ITEVWSYNAELIV* |
| | *AGAAGTATGGTCTTACAATGCTGAACTTATTGTGGCAA* | *AMENQHTIDLADS* |
| | *TGGAAAACCAGCACACTATTGATTTGGCTGATTCAGAG* | *EMNRLYERVRKQL* |
| | *ATGAACAGGCTGTATGAGCGAGTGAGGAAACAATTAAG* | *RENAEEDGTGCFE* |
| | *GGAAAATGCTGAAGAGGATGGTACTGGTTGCTTTGAAA* | *IFHKCDDDCMASI* |
| | *TTTTTCATAAATGTGACGATGATTGTATGGCTAGTATA* | *RNNTYDHSKYREE* |
| | *AGGAACAATACTTATGATCACAGCAAATACAGAGAAGA* | *AMQNRIQIDPVKL* |
| | *AGCGATGCAAAATAGAATACAAATTGACCCAGTCAAAT* | *SSGYKDVILWFSF* |
| | *TGAGTAGTGGCTACAAAGATGTGATACTTTGGTTTAGC* | *GASCFLLLAIAMG* |
| | *TTCGGGGCATCATGCTTTTTGCTTCTTGCCATTGCAAT* | *LVFICVKNGNMRC* |
| | *GGGCCTTGTTTTCATATGTGTGAAGAACGGAAACATGC* | *TICI***LRPEAPRAR** |
| | *GGTGCACTATTTGTATA***CTCCGGCCTGAAGCTCCGCGG** | **DPPVAT**MVSKGEE |
| | **GCCCGGGATCCACCGGTCGCCACC**ATGGTGAGCAAGGG | LFTGVVPILVELD |
| | CGAGGAGCTGTTCACCGGGGTGGTGCCCATCCTGGTCG | GDVNGHKFSVSGE |
| | AGCTGGACGGCGACGTAAACGGCCACAAGTTCAGCGTG | GEGDATYGKLTLK |
| | TCCGGCGAGGGCGAGGGCGATGCCACCTACGGCAAGCT | FICTTGKLPVPWP |
| | GACCCTGAAGTTCATCTGCACCACCGGCAAGCTGCCCG | TLVTTFGYGLQCF |
| | TGCCCTGGCCCACCCTCGTGACCACCTTCGGCTACGGC | ARYPDHMKQHDFF |
| | CTGCAGTGCTTCGCCCGCTACCCCGACCACATGAAGCA | KSAMPEGYVQERT |
| | GCACGACTTCTTCAAGTCCGCCATGCCCGAAGGCTACG | IFFKDDGNYKTRA |
| | TCCAGGAGCGCACCATCTTCTTCAAGGACGACGGCAAC | EVKFEGDTLVNRI |
| | TACAAGACCCGCGCCGAGGTGAAGTTCGAGGGCGACAC | ELKGIDFKEDGNI |
| | CCTGGTGAACCGCATCGAGCTGAAGGGCATCGACTTCA | LGHKLEYNYNSHN |
| | AGGAGGACGGCAACATCCTGGGGCACAAGCTGGAGTAC | VYIMADKQKNGIK |
| | AACTACAACAGCCACAACGTCTATATCATGGCCGACAA | VNFKIRHNIEDGS |
| | GCAGAAGAACGGCATCAAGGTGAACTTCAAGATCCGCC | VQLADHYQQNTPI |
| | ACAACATCGAGGACGGCAGCGTGCAGCTCGCCGACCAC | GDGPVLLPDNHYL |
| | TACCAGCAGAACACCCCCATCGGCGACGGCCCCGTGCT | SYQSALSKDPNEK |
| | GCTGCCCGACAACCACTACCTGAGCTACCAGTCCAAAC | RDHMVLLEFVTAA |
| | TGAGCAAAGACCCCAACGAGAAGCGCGATCACATGGTC | GITLGMDELYK |
| | CTGCTGGAGTTCGTGACCGCCGCCGGGATCACTCTCGG | |
| | CATGGACGAGCTGTACAAGTAA | |
| | *Italics*: HA, **bold**: linker, normal typeface: mYFP. | |
| 11 (M3-HA-mCer): | *ATGAACACTCAAATCCTGGTTTTCGCCCTTGTGGCAGT* | *MNTQILVFALVAV* |
| | *CATTCCCACAAATGCAGACAAAATTTGTCTTGGACATC* | *IPTNADKICLGHH* |
| | *ATGCTGTATCAAATGGCACCAAAGTAAACACACTCACT* | *AVSNGTKVNTLTE* |
| | *GAGAGAGGAGTAGAAGTTGTCAATGCAACGGAAACAGT* | *RGVEVVNATETVE* |

| | | |
|---|---|---|
| | *GGAGCGGACAAACATCCCCAAAATTTGCTCAAAAGGGA* | *RTNIPKICSKGKR* |
| | *AAAGAACCACTGATCTTGGCCAATGCGGACTGTTAGGG* | *TTDLGQCGLLGTI* |
| | *ACCATTACCGGACCACCTCAATGCGACCAATTTCTAGA* | *TGPPQCDQFLEFS* |
| | *ATTTTCAGCTGATCTAATAATCGAGAGACGAGAAGGAA* | *ADLIIERREGNDV* |
| | *ATGATGTTTGTTACCCGGGGAAGTTTGTTAATGAAGAG* | *CYPGKFVNEEALR* |
| | *GCATTGCGACAAATCCTCAGAGGATCAGGTGGGATTGA* | *QILRGSGGIDKET* |
| | *CAAAGAAACAATGGGATTCACATATAGTGGAATAAGGA* | *MGFTYSGIRTNGT* |
| | *CCAACGGAACAACTAGTGCATGTAGAAGATCAGGGTCT* | *TSACRRSGSSFYA* |
| | *TCATTCTATGCAGAAATGGAGTGGCTCCTGTCAAATAC* | *EMEWLLSNTDNAS* |
| | *AGACAATGCTTCTTTCCCACAAATGACAAAATCATACA* | *FPQMTKSYKNTRR* |
| | *AAAACACAAGGAGAGAATCAGCTCTGATAGTATGGGGA* | *ESALIVWGIHHSG* |
| | *ATCCACCATTCAGGATCAACCACCGAACAGACCAAACT* | *STTEQTKLYGSGN* |
| | *ATATGGGAGTGGAAATAAACTGATAACAGTCGGGAGTT* | *KLITVGSSKYHQS* |
| | *CCAAATATCATCAATCTTTTGTGCCGAGTCCAGGAACA* | *FVPSPGTRPQING* |
| | *CGACCGCAGATAAATGGCCAGTCCGGACGGATTGATTT* | *QSGRIDFHWLILD* |
| | *TCATTGGTTGATCTTGGATCCCAATGATACAGTTACTT* | *PNDTVTFSFNGAF* |
| | *TTAGTTTCAATGGGGCTTTCATAGCTCCAAATCGTGCC* | *IAPNRASFLRGKS* |
| | *AGCTTCTTGAGGGGAAAGTCCATGGGGATCCAGAGCGA* | *MGIQSDVQVDANC* |
| | *TGTGCAGGTTGATGCCAATTGCGAAGGGGAATGCTACC* | *EGECYHSGGTITS* |
| | *ACAGTGGAGGGACTATAACAAGCAGATTGCCTTTTCAA* | *RLPFQNINSRAVG* |
| | *AACATCAATAGCAGAGCAGTTGGCAAATGCCCAAGATA* | *KCPRYVKQESLLL* |
| | *TGTAAAACAGGAAAGTTTATTATTGGCAACTGGGATGA* | *ATGMKNVPEPSKK* |
| | *AGAACGTTCCCGAACCTTCCAAAAAAAGGAAAAAAAGA* | *RKKRGLFGAIAGF* |
| | *GGCCTGTTTGGCGCTATAGCAGGGTTTATTGAAAATGG* | *IENGWEGLVDGWY* |
| | *TTGGGAAGGTCTGGTCGACGGGTGGTACGGTTTCAGGC* | *GFRHQNAQGEGTA* |
| | *ATCAGAATGCACAAGGAGAAGGAACTGCAGCAGACTAC* | *ADYKSTQSAIDQI* |
| | *AAAAGCACCCAATCGGCAATTGATCAGATAACCGGAAA* | *TGKLNRLIEKTNQ* |
| | *GTTAAATAGACTCATTGAGAAAACCAACCAGCAATTTG* | *QFELIDNEFTEVE* |
| | *AGCTAATAGATAATGAATTCACTGAGGTGGAAAAGCAG* | *KQIGNLINWTKDS* |
| | *ATTGGCAATTTAATTAACTGGACCAAAGACTCCATCAC* | *ITEVWSYNAELIV* |
| | *AGAAGTATGGTCTTACAATGCTGAACTTATTGTGGCAA* | *AMENQHTIDLADS* |
| | *TGGAAAACCAGCACACTATTGATTTGGCTGATTCAGAG* | *EMNRLYERVRKQL* |
| | *ATGAACAGGCTGTATGAGCGAGTGAGGAAACAATTAAG* | *RENAEEDGTGCFE* |
| | *GGAAAATGCTGAAGAGGATGGTACTGGTTGCTTTGAAA* | *IFHKCDDDCMASI* |
| | *TTTTTCATAAATGTGACGATGATTGTATGGCTAGTATA* | *RNNTYDHSKYREE* |
| | *AGGAACAATACTTATGATCACAGCAAATACAGAGAAGA* | *AMQNRIQIDPVKL* |
| | *AGCGATGCAAAATAGAATACAAATTGACCCAGTCAAAT* | *SSGYKDVILWFSF* |
| | *TGAGTAGTGGCTACAAAGATGTGATACTTTGGTTTAGC* | *GASCFLLLAIAMG* |
| | *TTCGGGGCATCATGCTTTTTGCTTCTTGCCATTGCAAT* | *LVFISVKNGNMRS* |
| | *GGGCCTTGTTTTCATATCTGTGAAGAACGGAAACATGC* | *TISI***LRPEAPRAR** |
| | *GGTCCACTATTTCTATA***CTCCGGCCTGAAGCTCCGCGG** | **DPPVAT**MVSKGEE |
| | **GCCCGGGATCCACCGGTCGCCACC**ATGGTGAGCAAGGG | LFTGVVPILVELD |
| | CGAGGAGCTGTTCACCGGGGTGGTGCCCATCCTGGTCG | GDVNGHKFSVSGE |
| | AGCTGGACGGCGACGTAAACGGCCACAAGTTCAGCGTG | GEGDATYGKLTLK |
| | TCCGGCGAGGGCGAGGGCGATGCCACCTACGGCAAGCT | FICTTGKLPVPWP |
| | GACCCTGAAGTTCATCTGCACCACCGGCAAGCTGCCCG | TLVTTLTWGVQCF |
| | TGCCCTGGCCCACCCTCGTGACCACCCTGACCTGGGGC | ARYPDHMKQHDFF |
| | GTGCAGTGCTTCGCCCGCTACCCCGACCACATGAAGCA | KSAMPEGYVQERT |
| | GCACGACTTCTTCAAGTCCGCCATGCCCGAAGGCTACG | IFFKDDGNYKTRA |
| | TCCAGGAGCGCACCATCTTCTTCAAGGACGACGGCAAC | EVKFEGDTLVNRI |
| | TACAAGACCCGCGCCGAGGTGAAGTTCGAGGGCGACAC | ELKGIDFKEDGNI |
| | CCTGGTGAACCGCATCGAGCTGAAGGGCATCGACTTCA | LGHKLEYNAISDN |

| | | |
|---|---|---|
| | AGGAGGACGGCAACATCCTGGGGCACAAGCTGGAGTAC<br>AACGCCATCAGCGACAACGTCTATATCACCGCCGACAA<br>GCAGAAGAACGGCATCAAGGCCAACTTCAAGATCCGCC<br>ACAACATCGAGGACGGCAGCGTGCAGCTCGCCGACCAC<br>TACCAGCAGAACACCCCCATCGGCGACGGCCCCGTGCT<br>GCTGCCCGACAACCACTACCTGAGCACCCAGTCCAAAC<br>TGAGCAAAGACCCCAACGAGAAGCGCGATCACATGGTC<br>CTGCTGGAGTTCGTGACCGCCGCCGGGATCACTCTCGG<br>CATGGACGAGCTGTACAAGTAA<br>*Italics*: HA, underlined: mutations to obtain Cys → Ser exchange (no palmitoylation), **bold**: linker, normal typeface: mCer. | VYITADKQKNGIK<br>ANFKIRHNIEDGS<br>VQLADHYQQNTPI<br>GDGPVLLPDNHYL<br>STQSALSKDPNEK<br>RDHMVLLEFVTAA<br>GITLGMDELYK |
| 12 (M3-HA-mYFP): | *ATGAACACTCAAATCCTGGTTTTCGCCCTTGTGGCAGT*<br>*CATTCCCACAAATGCAGACAAAATTTGTCTTGGACATC*<br>*ATGCTGTATCAAATGGCACCAAAGTAAACACACTCACT*<br>*GAGAGAGGAGTAGAAGTTGTCAATGCAACGGAAACAGT*<br>*GGAGCGGACAAACATCCCCAAAATTTGCTCAAAAGGGA*<br>*AAAGAACCACTGATCTTGGCCAATGCGGACTGTTAGGG*<br>*ACCATTACCGGACCACCTCAATGCGACCAATTTCTAGA*<br>*ATTTTCAGCTGATCTAATAATCGAGAGACGAGAAGGAA*<br>*ATGATGTTTGTTACCCGGGGAAGTTTGTTAATGAAGAG*<br>*GCATTGCGACAAATCCTCAGAGGATCAGGTGGGATTGA*<br>*CAAAGAAACAATGGGATTCACATATAGTGGAATAAGGA*<br>*CCAACGGAACAACTAGTGCATGTAGAAGATCAGGGTCT*<br>*TCATTCTATGCAGAAATGGAGTGGCTCCTGTCAAATAC*<br>*AGACAATGCTTCTTTCCCACAAATGACAAAATCATACA*<br>*AAAACACAAGGAGAGAATCAGCTCTGATAGTATGGGGA*<br>*ATCCACCATTCAGGATCAACCACCGAACAGACCAAACT*<br>*ATATGGGAGTGGAAATAAACTGATAACAGTCGGGAGTT*<br>*CCAAATATCATCAATCTTTTGTGCCGAGTCCAGGAACA*<br>*CGACCGCAGATAAATGGCCAGTCCGGACGGATTGATTT*<br>*TCATTGGTTGATCTTGGATCCCAATGATACAGTTACTT*<br>*TTAGTTTCAATGGGGCTTTCATAGCTCCAAATCGTGCC*<br>*AGCTTCTTGAGGGGAAAGTCCATGGGGATCCAGAGCGA*<br>*TGTGCAGGTTGATGCCAATTGCGAAGGGGAATGCTACC*<br>*ACAGTGGAGGGACTATAACAAGCAGATTGCCTTTTCAA*<br>*AACATCAATAGCAGAGCAGTTGGCAAATGCCCAAGATA*<br>*TGTAAAACAGGAAAGTTTATTATTGGCAACTGGGATGA*<br>*AGAACGTTCCCGAACCTTCCAAAAAAAGGAAAAAAAGA*<br>*GGCCTGTTTGGCGCTATAGCAGGGTTTATTGAAAATGG*<br>*TTGGGAAGGTCTGGTCGACGGGTGGTACGGTTTCAGGC*<br>*ATCAGAATGCACAAGGAGAAGGAACTGCAGCAGACTAC*<br>*AAAAGCACCCAATCGGCAATTGATCAGATAACCGGAAA*<br>*GTTAAATAGACTCATTGAGAAAACCAACCAGCAATTTG*<br>*AGCTAATAGATAATGAATTCACTGAGGTGGAAAAGCAG*<br>*ATTGGCAATTTAATTAACTGGACCAAAGACTCCATCAC*<br>*AGAAGTATGGTCTTACAATGCTGAACTTATTGTGGCAA*<br>*TGGAAAACCAGCACACTATTGATTTGGCTGATTCAGAG*<br>*ATGAACAGGCTGTATGAGCGAGTGAGGAAACAATTAAG*<br>*GGAAAATGCTGAAGAGGATGGTACTGGTTGCTTTGAAA*<br>*TTTTTCATAAATGTGACGATGATTGTATGGCTAGTATA* | *MNTQILVFALVAV*<br>*IPTNADKICLGHH*<br>*AVSNGTKVNTLTE*<br>*RGVEVVNATETVE*<br>*RTNIPKICSKGKR*<br>*TTDLGQCGLLGTI*<br>*TGPPQCDQFLEFS*<br>*ADLIIERREGNDV*<br>*CYPGKFVNEEALR*<br>*QILRGSGGIDKET*<br>*MGFTYSGIRTNGT*<br>*TSACRRSGSSFYA*<br>*EMEWLLSNTDNAS*<br>*FPQMTKSYKNTRR*<br>*ESALIVWGIHHSG*<br>*STTEQTKLYGSGN*<br>*KLITVGSSKYHQS*<br>*FVPSPGTRPQING*<br>*QSGRIDFHWLILD*<br>*PNDTVTFSFNGAF*<br>*IAPNRASFLRGKS*<br>*MGIQSDVQVDANC*<br>*EGECYHSGGTITS*<br>*RLPFQNINSRAVG*<br>*KCPRYVKQESLLL*<br>*ATGMKNVPEPSKK*<br>*RKKRGLFGAIAGF*<br>*IENGWEGLVDGWY*<br>*GFRHQNAQGEGTA*<br>*ADYKSTQSAIDQI*<br>*TGKLNRLIEKTNQ*<br>*QFELIDNEFTEVE*<br>*KQIGNLINWTKDS*<br>*ITEVWSYNAELIV*<br>*AMENQHTIDLADS*<br>*EMNRLYERVRKQL*<br>*RENAEEDGTGCFE*<br>*IFHKCDDDCMASI*<br>*RNNTYDHSKYREE* |

| | | |
|---|---|---|
| | *AGGAACAATACTTATGATCACAGCAAATACAGAGAAGA*<br>*AGCGATGCAAAATAGAATACAAATTGACCCAGTCAAAT*<br>*TGAGTAGTGGCTACAAAGATGTGATACTTTGGTTTAGC*<br>*TTCGGGGCATCATGCTTTTTGCTTCTTGCCATTGCAAT*<br>*GGGCCTTGTTTTCATATCTGTGAAGAACGGAAACATGC*<br>*GGTCCACTATTTCTATA***CTCCGGCCTGAAGCTCCGCGG**<br>**GCCCGGGATCCACCGGTCGCCACC**ATGGTGAGCAAGGG<br>CGAGGAGCTGTTCACCGGGGTGGTGCCCATCCTGGTCG<br>AGCTGGACGGCGACGTAAACGGCCACAAGTTCAGCGTG<br>TCCGGCGAGGGCGAGGGCGATGCCACCTACGGCAAGCT<br>GACCCTGAAGTTCATCTGCACCACCGGCAAGCTGCCCG<br>TGCCCTGGCCCACCCTCGTGACCACCTTCGGCTACGGC<br>CTGCAGTGCTTCGCCCGCTACCCCGACCACATGAAGCA<br>GCACGACTTCTTCAAGTCCGCCATGCCCGAAGGCTACG<br>TCCAGGAGCGCACCATCTTCTTCAAGGACGACGGCAAC<br>TACAAGACCCGCGCCGAGGTGAAGTTCGAGGGCGACAC<br>CCTGGTGAACCGCATCGAGCTGAAGGGCATCGACTTCA<br>AGGAGGACGGCAACATCCTGGGGCACAAGCTGGAGTAC<br>AACTACAACAGCCACAACGTCTATATCATGGCCGACAA<br>GCAGAAGAACGGCATCAAGGTGAACTTCAAGATCCGCC<br>ACAACATCGAGGACGGCAGCGTGCAGCTCGCCGACCAC<br>TACCAGCAGAACACCCCCATCGGCGACGGCCCCGTGCT<br>GCTGCCCGACAACCACTACCTGAGCTACCAGTCCAAAC<br>TGAGCAAAGACCCCAACGAGAAGCGCGATCACATGGTC<br>CTGCTGGAGTTCGTGACCGCCGCCGGGATCACTCTCGG<br>CATGGACGAGCTGTACAAGTAA<br>*Italics*: HA, underlined: mutations to obtain Cys → Ser exchange (no palmitoylation), **bold**: linker, normal typeface: mYFP. | *AMQNRIQIDPVKL*<br>*SSGYKDVILWFSF*<br>*GASCFLLLAIAMG*<br>*LVFISVKNGNMRS*<br>*TISI***LRPEAPRAR**<br>**DPPVAT**MVSKGEE<br>LFTGVVPILVELD<br>GDVNGHKFSVSGE<br>GEGDATYGKLTLK<br>FICTTGKLPVPWP<br>TLVTTFGYGLQCF<br>ARYPDHMKQHDFF<br>KSAMPEGYVQERT<br>IFFKDDGNYKTRA<br>EVKFEGDTLVNRI<br>ELKGIDFKEDGNI<br>LGHKLEYNYNSHN<br>VYIMADKQKNGIK<br>VNFKIRHNIEDGS<br>VQLADHYQQNTPI<br>GDGPVLLPDNHYL<br>SYQSALSKDPNEK<br>RDHMVLLEFVTAA<br>GITLGMDELYK |
| 13 (Lyn-mCer): | **ATGGGATGTATTAAATCAAAAAGGAAAGACAATCTCAA**<br>**TGACGATGAACCGGTCGCCACC**ATGGTGAGCAAGGGCG<br>AGGAGCTGTTCACCGGGGTGGTGCCCATCCTGGTCGAG<br>CTGGACGGCGACGTAAACGGCCACAAGTTCAGCGTGTC<br>CGGCGAGGGCGAGGGCGATGCCACCTACGGCAAGCTGA<br>CCCTGAAGTTCATCTGCACCACCGGCAAGCTGCCCGTG<br>CCCTGGCCCACCCTCGTGACCACCCTGACCTGGGGCGT<br>GCAGTGCTTCGCCCGCTACCCCGACCACATGAAGCAGC<br>ACGACTTCTTCAAGTCCGCCATGCCCGAAGGCTACGTC<br>CAGGAGCGCACCATCTTCTTCAAGGACGACGGCAACTA<br>CAAGACCCGCGCCGAGGTGAAGTTCGAGGGCGACACCC<br>TGGTGAACCGCATCGAGCTGAAGGGCATCGACTTCAAG<br>GAGGACGGCAACATCCTGGGGCACAAGCTGGAGTACAA<br>CGCCATCAGCGACAACGTCTATATCACCGCCGACAAGC<br>AGAAGAACGGCATCAAGGCCAACTTCAAGATCCGCCAC<br>AACATCGAGGACGGCAGCGTGCAGCTCGCCGACCACTA<br>CCAGCAGAACACCCCCATCGGCGACGGCCCCGTGCTGC<br>TGCCCGACAACCACTACCTGAGCACCCAGTCCAAACTG<br>AGCAAAGACCCCAACGAGAAGCGCGATCACATGGTCCT<br>GCTGGAGTTCGTGACCGCCGCCGGGATCACTCTCGGCA<br>TGGACGAGCTGTACAAGTAA | **MGCIKSKRKDNLN**<br>**DDEPVAT**MVSKGE<br>ELFTGVVPILVEL<br>DGDVNGHKFSVSG<br>EGEGDATYGKLTL<br>KFICTTGKLPVPW<br>PTLVTTLTWGVQC<br>FARYPDHMKQHDF<br>FKSAMPEGYVQER<br>TIFFKDDGNYKTR<br>AEVKFEGDTLVNR<br>IELKGIDFKEDGN<br>ILGHKLEYNAISD<br>NVYITADKQKNGI<br>KANFKIRHNIEDG<br>SVQLADHYQQNTP<br>IGDGPVLLPDNHY<br>LSTQSALSKDPNE<br>KRDHMVLLEFVTA<br>AGITLGMDELYK |

| | | |
|---|---|---|
| | **Bold**: Lyn kinase sequence, **bold** and underlined: linker, normal typeface: mCer. | |
| 14 (Lyn-mYFP): | **ATGGGATGTATTAAATCAAAAAGGAAAGACAATCTCAA**<br>**TGACGATGAACCGGTCGCCACC**ATGGTGAGCAAGGGCG<br>AGGAGCTGTTCACCGGGGTGGTGCCCATCCTGGTCGAG<br>CTGGACGGCGACGTAAACGGCCACAAGTTCAGCGTGTC<br>CGGCGAGGGCGAGGGCGATGCCACCTACGGCAAGCTGA<br>CCCTGAAGTTCATCTGCACCACCGGCAAGCTGCCCGTG<br>CCCTGGCCCACCCTCGTGACCACCTTCGGCTACGGCCT<br>GCAGTGCTTCGCCCGCTACCCCGACCACATGAAGCAGC<br>ACGACTTCTTCAAGTCCGCCATGCCCGAAGGCTACGTC<br>CAGGAGCGCACCATCTTCTTCAAGGACGACGGCAACTA<br>CAAGACCCGCGCCGAGGTGAAGTTCGAGGGCGACACCC<br>TGGTGAACCGCATCGAGCTGAAGGGCATCGACTTCAAG<br>GAGGACGGCAACATCCTGGGGCACAAGCTGGAGTACAA<br>CTACAACAGCCACAACGTCTATATCATGGCCGACAAGC<br>AGAAGAACGGCATCAAGGTGAACTTCAAGATCCGCCAC<br>AACATCGAGGACGGCAGCGTGCAGCTCGCCGACCACTA<br>CCAGCAGAACACCCCCATCGGCGACGGCCCCGTGCTGC<br>TGCCCGACAACCACTACCTGAGCTACCAGTCCAAACTG<br>AGCAAAGACCCCAACGAGAAGCGCGATCACATGGTCCT<br>GCTGGAGTTCGTGACCGCCGCCGGGATCACTCTCGGCA<br>TGGACGAGCTGTACAAGTAA<br>**Bold**: Lyn kinase sequence, **bold** and underlined: linker, normal typeface: mYFP. | **MGCIKSKRKDNLN**<br>**DDEPVAT**MVSKGE<br>ELFTGVVPILVEL<br>DGDVNGHKFSVSG<br>EGEGDATYGKLTL<br>KFICTTGKLPVPW<br>PTLVTTFGYGLQC<br>FARYPDHMKQHDF<br>FKSAMPEGYVQER<br>TIFFKDDGNYKTR<br>AEVKFEGDTLVNR<br>IELKGIDFKEDGN<br>ILGHKLEYNYNSH<br>NVYIMADKQKNGI<br>KVNFKIRHNIEDG<br>SVQLADHYQQNTP<br>IGDGPVLLPDNHY<br>LSYQSALSKDPNE<br>KRDHMVLLEFVTA<br>AGITLGMDELYK |
| 15 (Mem-mCer): | **ATGCTGTGCTGTATGAGAAGAACCAAACAGGTTGAAAA**<br>**GAATGATGAGGACCAACCGGTCGCCACC**ATGGTGAGCA<br>AGGGCGAGGAGCTGTTCACCGGGGTGGTGCCCATCCTG<br>GTCGAGCTGGACGGCGACGTAAACGGCCACAAGTTCAG<br>CGTGTCCGGCGAGGGCGAGGGCGATGCCACCTACGGCA<br>AGCTGACCCTGAAGTTCATCTGCACCACCGGCAAGCTG<br>CCCGTGCCCTGGCCCACCCTCGTGACCACCCTGACCTG<br>GGGCGTGCAGTGCTTCGCCCGCTACCCCGACCACATGA<br>AGCAGCACGACTTCTTCAAGTCCGCCATGCCCGAAGGC<br>TACGTCCAGGAGCGCACCATCTTCTTCAAGGACGACGG<br>CAACTACAAGACCCGCGCCGAGGTGAAGTTCGAGGGCG<br>ACACCCTGGTGAACCGCATCGAGCTGAAGGGCATCGAC<br>TTCAAGGAGGACGGCAACATCCTGGGGCACAAGCTGGA<br>GTACAACGCCATCAGCGACAACGTCTATATCACCGCCG<br>ACAAGCAGAAGAACGGCATCAAGGCCAACTTCAAGATC<br>CGCCACAACATCGAGGACGGCAGCGTGCAGCTCGCCGA<br>CCACTACCAGCAGAACACCCCCATCGGCGACGGCCCCG<br>TGCTGCTGCCCGACAACCACTACCTGAGCACCCAGTCC<br>AAACTGAGCAAAGACCCCAACGAGAAGCGCGATCACAT<br>GGTCCTGCTGGAGTTCGTGACCGCCGCCGGGATCACTC<br>TCGGCATGGACGAGCTGTACAAGTAA<br><br>**Bold**: GAP43 sequence, **bold** and underlined: linker, normal typeface: mCer. | **MLCCMRRTKQVEK**<br>**NDEDQPVAT**MVSK<br>GEELFTGVVPILV<br>ELDGDVNGHKFSV<br>SGEGEGDATYGKL<br>TLKFICTTGKLPV<br>PWPTLVTTLTWGV<br>QCFARYPDHMKQH<br>DFFKSAMPEGYVQ<br>ERTIFFKDDGNYK<br>TRAEVKFEGDTLV<br>NRIELKGIDFKED<br>GNILGHKLEYNAI<br>SDNVYITADKQKN<br>GIKANFKIRHNIE<br>DGSVQLADHYQQN<br>TPIGDGPVLLPDN<br>HYLSTQSALSKDP<br>NEKRDHMVLLEFV<br>TAAGITLGMDELY<br>K |
| 16 (Mem- | **ATGCTGTGCTGTATGAGAAGAACCAAACAGGTTGAAAA** | **MLCCMRRTKQVEK** |

| | | |
|---|---|---|
| mYFP): | **GAATGATGAGGACCAA<u>CCGGTCGCCACC</u>**ATGGTGAGCAAGGGCGAGGAGCTGTTCACCGGGGTGGTGCCCATCCTGGTCGAGCTGGACGGCGACGTAAACGGCCACAAGTTCAGCGTGTCCGGCGAGGGCGAGGGCGATGCCACCTACGGCAAGCTGACCCTGAAGTTCATCTGCACCACCGGCAAGCTGCCCGTGCCCTGGCCCACCCTCGTGACCACCTTCGGCTACGGCCTGCAGTGCTTCGCCCGCTACCCCGACCACATGAAGCAGCACGACTTCTTCAAGTCCGCCATGCCCGAAGGCTACGTCCAGGAGCGCACCATCTTCTTCAAGGACGACGGCAACTACAAGACCCGCGCCGAGGTGAAGTTCGAGGGCGACACCCTGGTGAACCGCATCGAGCTGAAGGGCATCGACTTCAAGGAGGACGGCAACATCCTGGGGCACAAGCTGGAGTACAACTACAACAGCCACAACGTCTATATCATGGCCGACAAGCAGAAGAACGGCATCAAGGTGAACTTCAAGATCCGCCACAACATCGAGGACGGCAGCGTGCAGCTCGCCGACCACTACCAGCAGAACACCCCCATCGGCGACGGCCCCGTGCTGCTGCCCGACAACCACTACCTGAGCTACCAGTCCAAACTGAGCAAAGACCCCAACGAGAAGCGCGATCACATGGTCCTGCTGGAGTTCGTGACCGCCGCCGGGATCACTCTCGGCATGGACGAGCTGTACAAGTAA<br>**Bold**: GAP43 sequence, **bold** and <u>underlined</u>: linker, normal typeface: mYFP. | **NDEDQ<u>PVAT</u>**MVSKGEELFTGVVPILVELDGDVNGHKFSVSGEGEGDATYGKLTLKFICTTGKLPVPWPTLVTTFGYGLQCFARYPDHMKQHDFFKSAMPEGYVQERTIFFKDDGNYKTRAEVKFEGDTLVNRIELKGIDFKEDGNILGHKLEYNYNSHNVYIMADKQKNGIKVNFKIRHNIEDGSVQLADHYQQNTPIGDGPVLLPDNHYLSYQSALSKDPNEKRDHMVLLEFVTAAGITLGMDELYK |
| 17 (linker signal peptide–fluorophore for constructs 3 and 4) | **ATGAACACTCAAATCCTGGTTTTCGCCCTTGTGGCAGTCATTCCCACAAATGCAGACAAAATTTGTCTT**<u>CCGCGGGCCCGGGATCCACCGGTCGCCACC</u>*ATGGTG*<br>**Bold**: HA signal peptide, underlined: linker region, *italics*: start of CFP/YFP. | **MNTQILVFALVAVIPTNADKICL**<u>PRARDPPVAT</u>*MV* |
| 18 (linker fluorophore–HA TMD/CT for constructs 3, 4) | *GACGAGCTGTACAAG*GCTAGTATAAGGAACAATACTTATGATCACAGCAAATACAGAGAAGAAGCGATGCAAAATAGAATACAAATTGACCCAGTCAAATTGAGTAGTGGCTACAAAGATGTGATACTTTGGTTTAGCTTCGGGGCATCATGCTTTTTGCTTCTTGCCATTGCAATGGGCCTTGTTTTCATATGTGTGAAGAACGGAAACATGCGGTGCACTATTTGTATATAA<br><br>in *italics*: end of CFP/YFP, in normal typeface: sequence for TMD and CT of HA. In **bold**: glycosylation sites, underlined: transmembrane region, underlined and **bold**: C-terminal domain, C*: palmitoylated cysteine residues. | *DELYK*ASIR**NNT**YDHSKYREEAMQNRIQIDPVKLSSGYKD<u>VILWFSF-GASCFLLLAIAMGLVFIC*</u>**<u>VKNGNMRC*TIC*I</u>** |
| 19 (oligonucleotide for the generation of 1–6) | GATCTCATGAACACTCAAATCCTGGTTTTCGCCCTTGTGGCAGTCATTCCCACAAATGCAGACAAAATTTGTCTTCCGC | |
| 20 (antisense of 19) | GGAAGACAAATTTTGTCTGCATTTGTGGGAATGACTGCCACAAGGGCGAAAACCAGGATTTGAGTGTTCATGA | |
| 21 (forward | GGCTGTACAAGGCTAGTATAAGGAACAATACT | |

| | | |
|---|---|---|
| primer for the generation of 3–6) | | |
| 22 (reverse primer for 3, 4) | GCGCGGCCGCTTATATACAAATAGTGCACCGC | |
| 23 (reverse primer for 5, 6) | GCGCGGCCGCTTATATAGAAATAGTGGACCGC | |
| 24 (oligonucleotide GPI[DAF], for constructs 7, 8) | GTACAAGCCAAATAAAGGAAGTGGAACCACTTCAGGTACTACCCGTCTTCTATCTGGGCACACGTGTTTCACGTTGACAGGTTTGCTTGGGACGCTAGTAACCATGGGCTTGCTGACTTAGC | |
| 25 (antisense of 24) | GGCCGCTAAGTCAGCAAGCCCATGGTTACTAGCGTCCCAAGCAAACCTGTCAACGTGAAACACGTGTGCCCAGATAGAAGACGGGTAGTACCTGAAGTGGTTCCACTTCCTTTATTTGGCTT | |
| 26 (linker for constructs 9, 10) | *TGCACTATTTGTATA***CTCCGGCCTGAAGCTCCGCGGGCCCGGGATCCACCGGTCGCCACC**ATGGTGAGCAAG in *italics*: end of HA sequence, palmitoylated cysteines underlined, in **bold**: linker, in normal typeface: start of mCerulean. | *CTICI***LRPEAPRARDPPVAT**MVSK |
| 27 (linker for Lyn-mYFP/-mCer, constructs 13, 14) | ccgcggaccATGGGATGTATTAAATCAAAAAGGAAAGACAATCTCAATGACGATGAACCGGTCGCCACCATGGTG small letters: Kozak sequence, ATG: start codon of Lyn kinase, in **bold**: myristoylation and palmitoylation signal from Lyn kinase, **bold** and underlined: linker, normal typeface: start of mYFP/mCer. Myristoylated glycine and palmitoylated cysteine residue marked with asterisk. | MG*C*IKSKRKDNLNDDEPVATMV |
| 28 (linker for Mem-mYFP/-mCer, 15, 16) | accATGCTGTGCTGTATGAGAAGAACCAAACAGGTTGAAAAGAATGATGAGGACCAACCGGTCGCCACCATGGTG small letters: Kozak sequence, ATG: start codon of Lyn kinase, in **bold**: palmitoylation signal from GAP43, **bold** and underlined: linker, normal typeface: start of mYFP/mCer. Palmitoylated cysteine residues marked with asterisk. | MLC*C*MRRTKQVEKNDEDQPVATMV |
| 29 (primer for A206K mutation in YFP to generate mYFP, 10, 12, 14, 16) | GGGGTCTTTGCTCAGTTTGGACTGGTAGCTCAG | |

| | | |
|---|---|---|
| 30 (primer for A206K mutation in YFP to generate mYFP, 10, 12, 14, 16) | CTGAGCTACCAGTCCAAACTGAGCAAAGACCCC | |
| 31 (primer for generation of HA, 9–12) | CTCAGATCTCATGAACACTCAAATCCTGGTTTTC | |
| 32 (reverse primer for HA, 9, 10) | GACCCGCGGAGCTTCAGGCCGGAGTATACAAATAGTGC ACCG | |
| 33 (reverse primer for M3-HA, 11, 12) | GACCCGCGGAGCTTCAGGCCGGAGTATAGAAATAGTGG ACCG | |
| 34 (Mem oligonucleotide, 15, 16) | GGGCCGCGGATGCTGTGCTGTATGAGAAGAACCAAACA GGTTGAAAAGAATGATGAGGACCAACCGGTCAAA | |
| 35 (antisense of 34) | TTTGACCGGTTGGTCCTCATCATTCTTTTCAACCTGTT TGGTTCTTCTCATACAGCACAGCATCCGCGGCCC | |
| 36 (Lyn oligonucleotide, 13, 14) | GGGCCGCGGACCATGGGATGTATTAAATCAAAAAGGAA AGACAATCTCAATGACGATGAACCGGTCCCC | |
| 37 (antisense of 36) | GGGGACCGGTTCATCGTCATTGAGATTGTCTTTCCTTT TTGATTTAATACATCCCATGGTCCGCGGCCC | |
| 38 (linker CFP–M1 in construct 1) | *TACAAG*TCCGGACTCAGATCTCGAGCT**ATGAGT** | *YK*SGLRSRA**MS** |
| 39 (nuclear export signal derived from Rev) | | LQLPPLERLTL |
| 40 (NES in construct 1) | *TTCAAG***CTTCAGCTACCACCGCTTGAGAGACTTACTCT TTGA** | *FK***LQLPPLERLTL** |
| 41 (primer for NES construction) | GCTTACCTCGAGCTATGAGTCTTCTAACCGAGG | |
| 42 (reverse primer for NES construction) | GTCTCAGGTACCTCA*AAGAGTAAGTCTCTCAAGCGGTG GTAGCTGAAG*CTTGAATCGTTGC | |
| 43 (Myris- | **ATGGGATCAATCAAATCAAAG**ATGAGTCTT | **MGSIKSK**MSL |

| | | |
|---|---|---|
| toylation signal in construct 2) | | |
| 44 (linker M1–CFP in construct 2) | TTCAAG**CCGCGGGCCCGGGATCCACCGGTCGCCACC***ATGGTGAGC* | FK**PRARDPPVAT***MVS* |
| 45 (primer for Myr-M1-CFP) | GCTTACCTCGAG*ATGGGATCAATCAAATCAAAG*ATGAGTCTTCTAACC | |
| 46 (reverse primer for Myr-M1-CFP) | GTCTTACCGCGGCTTGAATCGTTGCATCTGCAC | |

## Claims

1. A matrix protein M1 mutant comprising a nuclear export signal of Rev protein and a fluorescent protein or comprising a myristoylation signal of Lyn kinase and a fluorescent protein.

2. The mutant according to claim 1, wherein the fluorescent protein is cyan-fluorescent protein CFP.

3. The mutant according to claim 1 or 2, comprising the nuclear export signal of Rev protein at the C-terminus and the fluorescent protein at the N-terminus.

4. The mutant according to any of the preceding claims, comprising a linker according to SEQ ID NO: 60 between the nuclear export signal of Rev protein and the fluorescent protein.

5. The mutant according to claim 1 or 2, comprising the myristoylation signal of Lyn kinase at the N-terminus and the fluorescent protein at the C-terminus.

6. A nucleic acid encoding the matrix protein M1 mutant according to any of claims 1 to 4, preferably a nucleic acid comprising a nucleic acid sequence according to SEQ ID NO: 1.

7. A nucleic acid encoding the matrix protein M1 mutant according to any of claims 1, 2 and 5, preferably a nucleic acid comprising a nucleic acid sequence according to SEQ ID NO: 2.

8. A nucleic acid comprising a nucleic acid sequence according to SEQ ID NO: 59.

9. An amino acid sequence comprising an amino acid sequence according to SEQ ID NO: 2 or SEQ ID NO: 4.

10. A matrix protein M1 mutant comprising an amino acid sequence according to SEQ ID NO: 2 or SEQ ID NO: 4.

11. A vector comprising a nucleic acid according to any of claims 6 to 8.

12. A cell comprising a mutant according to any of claims 1 to 5 or 10 and/or a nucleic acid according to any of claims 6 to 8 and/or an amino acid sequence according to claim 9 and/or a vector according to claim 11.

13. A use of a mutant according to any of claims 1 to 5 or 10 and/or of a nucleic acid according to any of claims 6 to 8 and/or of an amino acid sequence according to claim 9 and/or of a vector according to claim 11 and/or of a cell according to claim 12 for an evaluation of an association of the matrix protein M1 mutant with a cellular membrane.

14. A method for an evaluation of an association of a matrix protein M1 mutant according to any of claims 1 to 5 or 10 with a cellular membrane, preferably using fluorescence microscopy on an intact cell.

15. A method for an evaluation of an interference of a candidate compound with an association of a matrix protein M1 mutant according to any of claims 1 to 5 or 10 with a cellular membrane, comprising the steps of:

(a) incubating a cell according to claim 12 with the candidate compound;
(b) detecting the association of the matrixprotein M1 mutant with the cellular membrane in the cell.

16. The method according to claim 15, further comprising the steps of:

(a') incubating a control cell according to claim 12 without the candidate compound;
(b') detecting the association of the matrix protein M1 mutant with the cellular membrane in the control cell;
(c) comparing the results obtained in steps (b) and (b').

17. The method according to claim 15 or 16, wherein steps (b) and (b') involve fluorescence microscopy on the intact cell.

18. The method according to any of claims 15 to 17, wherein interference is an inhibition, and the candidate compound is a putative inhibitor of an association of the matrix protein M1 with the cellular membrane.

**Fig. 1**

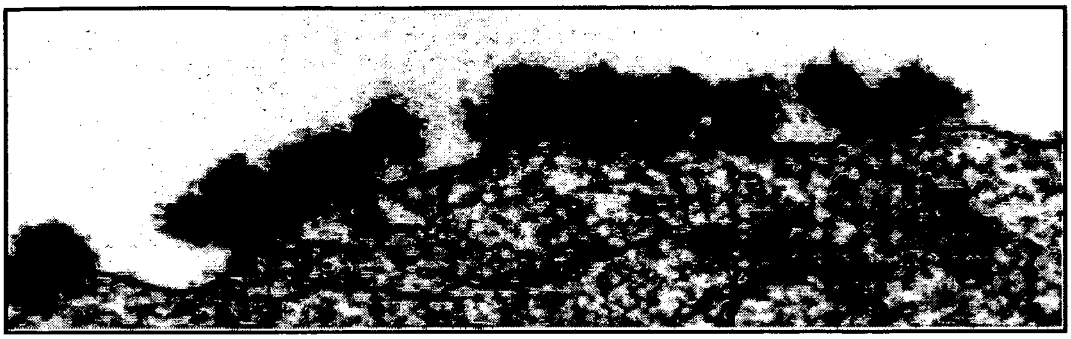

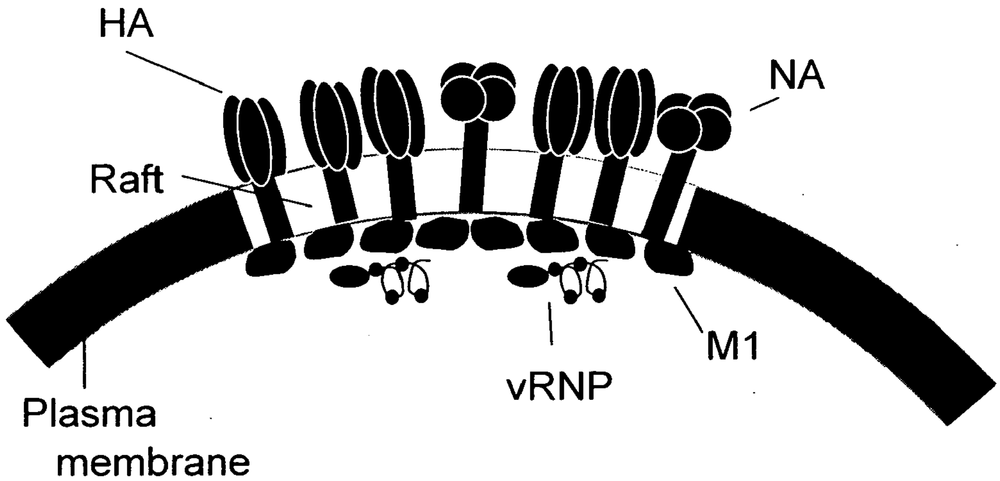

**Fig. 2**

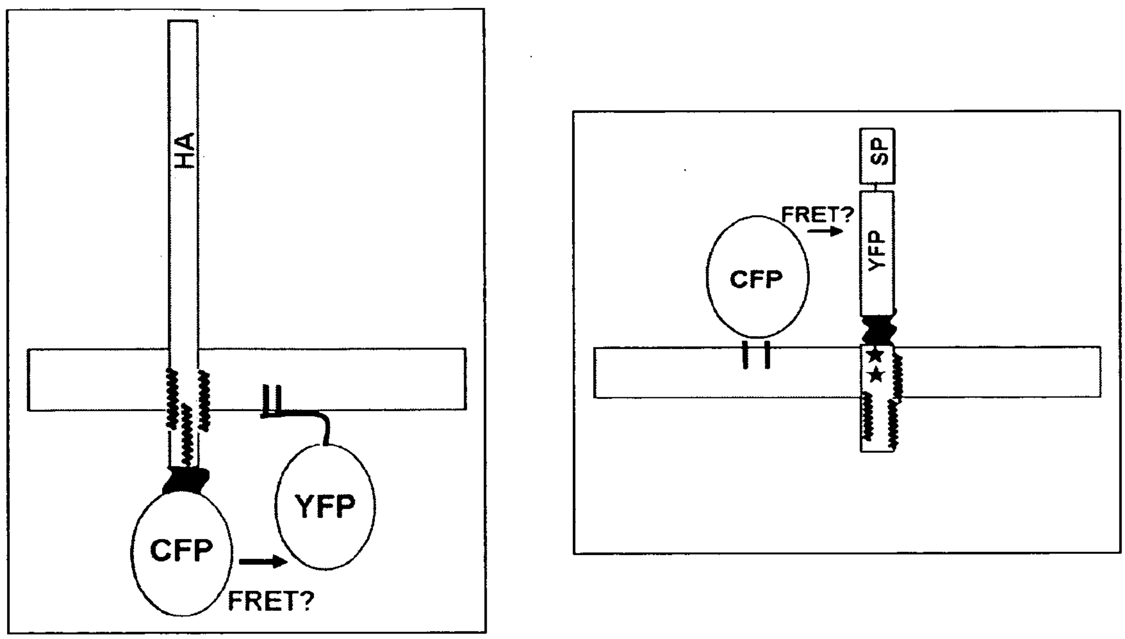

**Fig. 3a**

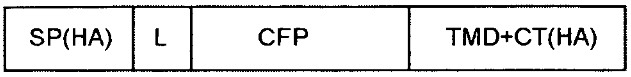


**Fig. 3b**

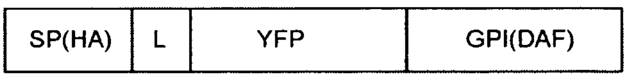

Fig. 4a

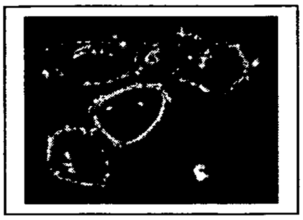

Fig. 4b

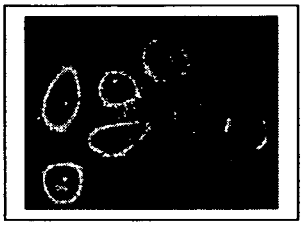

Fig. 4c

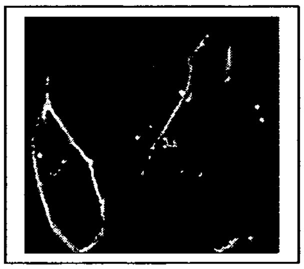

**Fig. 5**

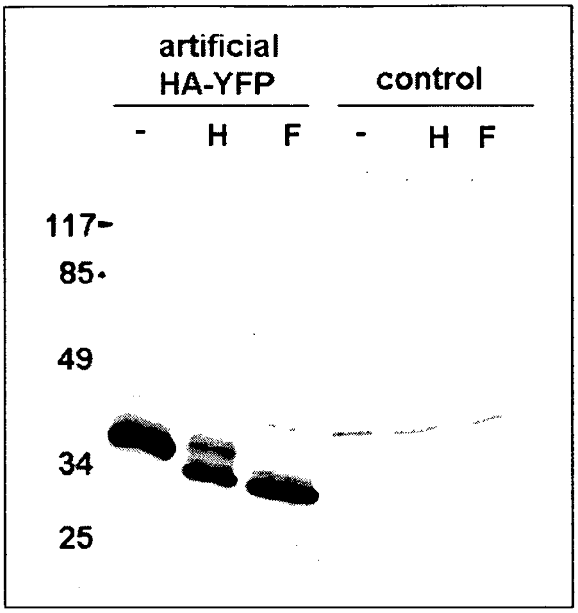

**Fig. 6a**

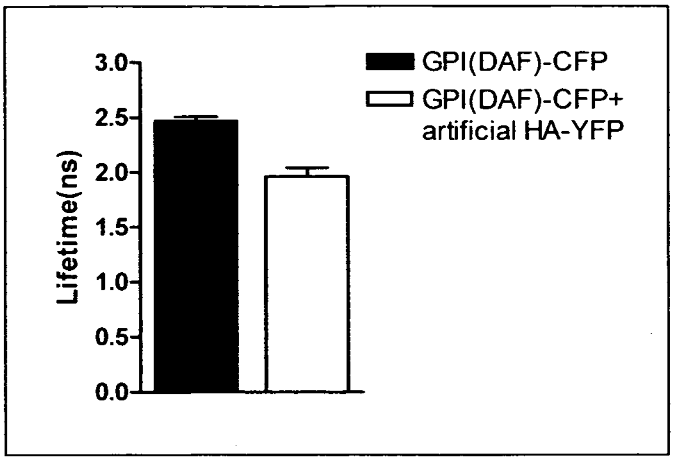
Lifetime(ns)
3.0
2.5
2.0
1.5
1.0
0.5
0.0
GPI(DAF)-CFP
GPI(DAF)-CFP+ artificial HA-YFP

**Fig. 6b**

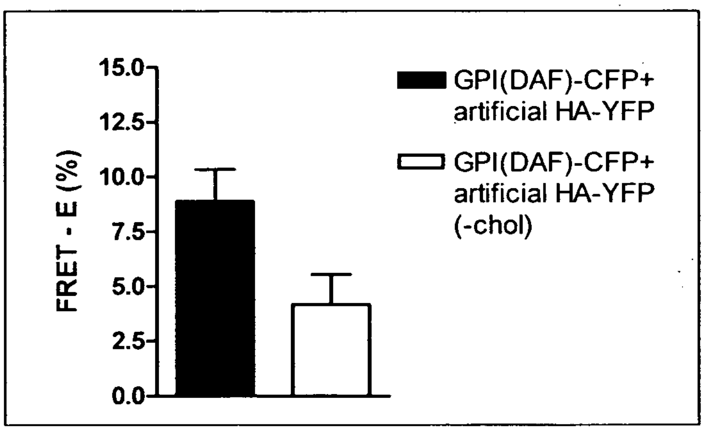
FRET - E (%)
15.0
12.5
10.0
7.5
5.0
2.5
0.0
GPI(DAF)-CFP+ artificial HA-YFP
GPI(DAF)-CFP+ artificial HA-YFP (-chol)

Fig. 7a

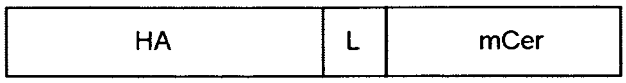


Fig. 7b

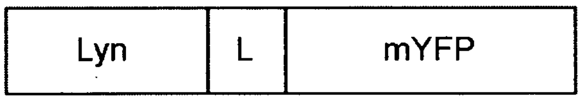


Fig. 7c

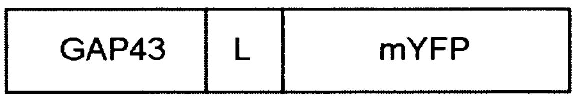

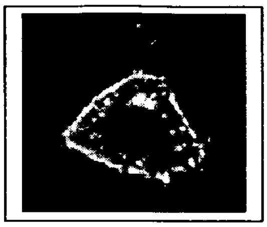

Fig. 8a

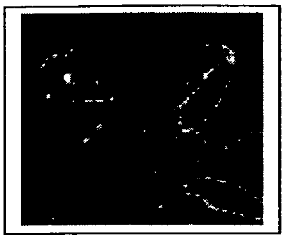

Fig. 8b

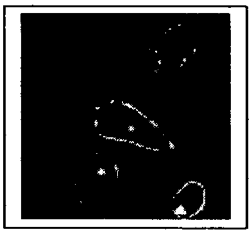

Fig. 8c

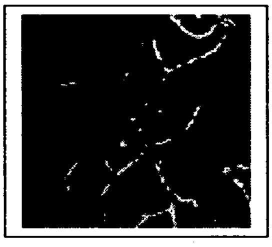

Fig. 8d

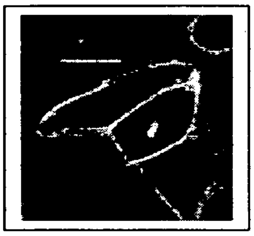

Fig. 8e

**Fig. 9a**

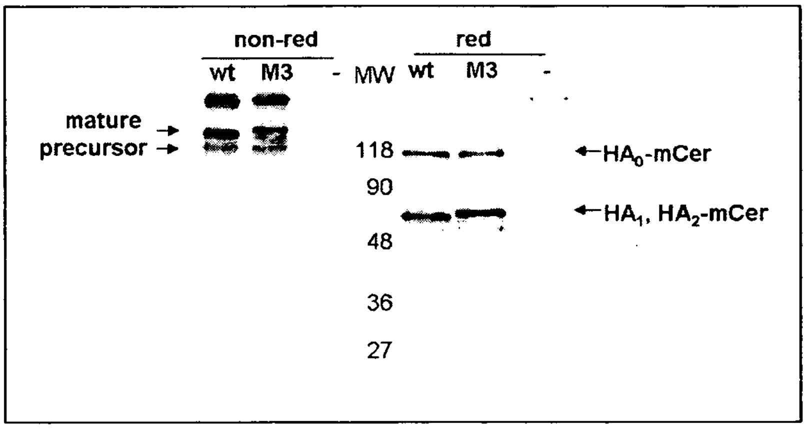


**Fig. 9b**

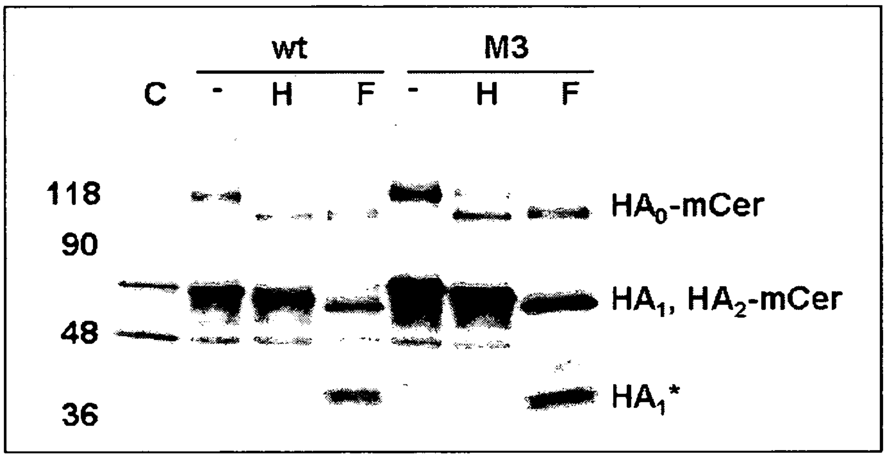


**Fig. 9c**

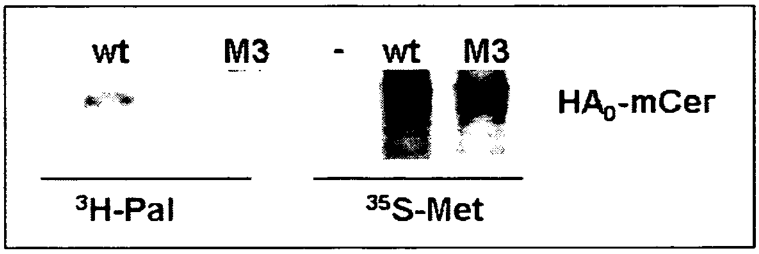

Fig. 10a

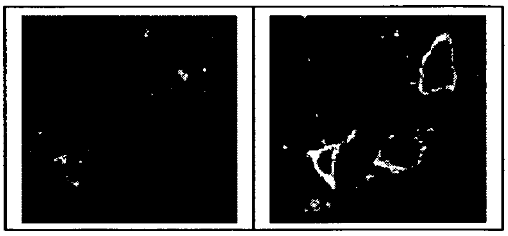

Fig. 10b

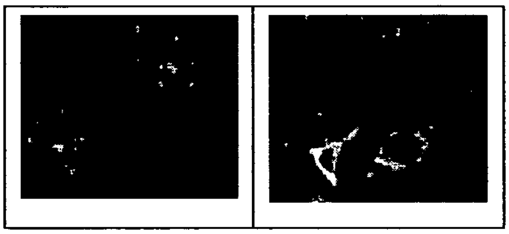

**Fig. 11**

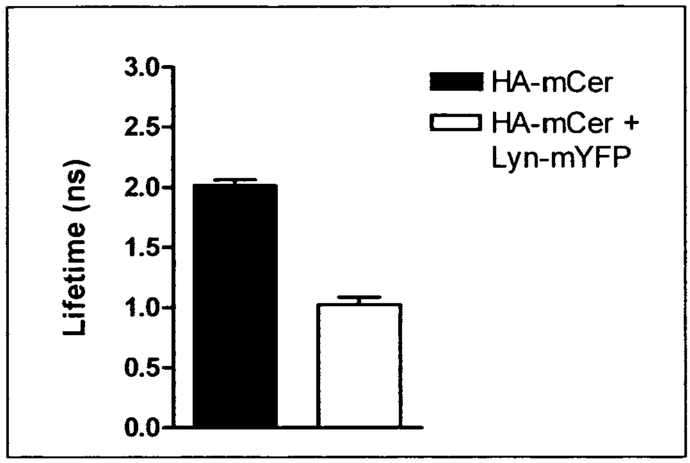

**Fig. 12a**

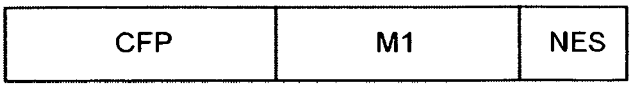


**Fig. 12b**

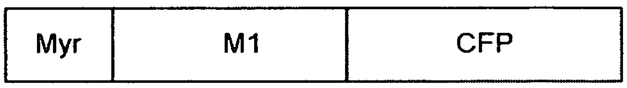

**Fig. 13**

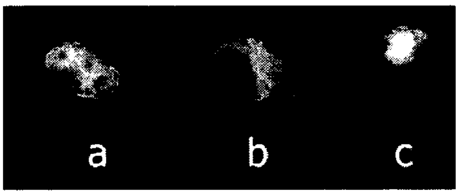


**Fig. 14**

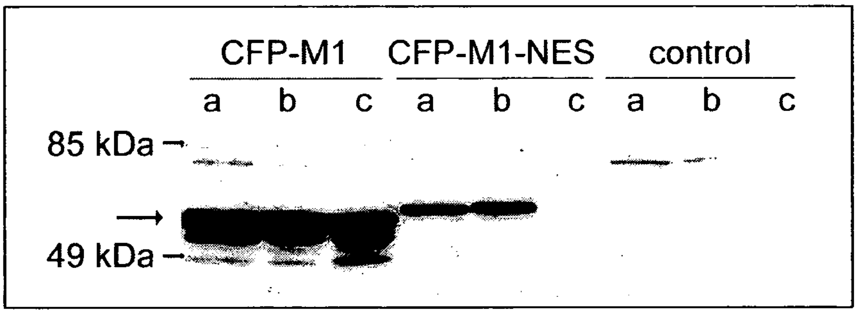

European Patent Office

# EUROPEAN SEARCH REPORT

Application Number
EP 08 00 1073

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2004/009028 A (MYRIAD GENETICS INC [US]; MORHAM SCOTT [US]; ZAVITZ KENTON [US]; HOBDE) 29 January 2004 (2004-01-29)<br>* example 4 *<br>----- | 1-18 | INV.<br>C07K14/11<br>C12N15/62<br>G01N33/542<br>G01N33/92 |
| A | ALI A ET AL: "Influenza virus assembly: Effect of influenza virus glycoproteins on the membrane association of M1 protein"<br>JOURNAL OF VIROLOGY, THE AMERICAN SOCIETY FOR MICROBIOLOGY, US,<br>vol. 74, no. 18,<br>1 September 2000 (2000-09-01), pages 8709-8719, XP002982630<br>ISSN: 0022-538X<br>* abstract *<br>----- | 1-18 | |
| A | NOTON SARAH L ET AL: "Identification of the domains of the influenza A virus M1 matrix protein required for NP binding, oligomerization and incorporation into virions"<br>JOURNAL OF GENERAL VIROLOGY,<br>vol. 88, no. Part 8,<br>August 2007 (2007-08), pages 2280-2290, XP002480369<br>ISSN: 0022-1317<br>* figure 8a *<br>----- | 1-18 | TECHNICAL FIELDS SEARCHED (IPC)<br>G01N<br>C07K<br>C12N |
| X | DATABASE EM-PAT<br>EBI Hinxton U.K.;<br>31 March 2005 (2005-03-31),<br>KABAT D ET AL: "Sequence 3 from Patent WO2005024422."<br>XP002480370<br>Database accession no. CS055383<br>nt.712-742 show 100% identiy in 31nt overlap with SeqIdNo.59<br>* abstract *<br>-----<br>-/-- | 8 | |

The present search report has been drawn up for all claims

1

| Place of search | Date of completion of the search | Examiner |
|---|---|---|
| The Hague | 16 May 2008 | Lonnoy, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 08 00 1073

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE GENESEQ<br>EBI Hinxton U.K.;<br>24 August 2006 (2006-08-24),<br>SUGAYA K ET AL: "pEGFP-C1 vector DNA fragment"<br>XP002480371<br>Database accession no. AEI27107<br>nt.1-27 show 100% identity in 27nt overlap with SeqIDNo.59<br>* abstract *<br>----- | 8 | |
| | | | TECHNICAL FIELDS SEARCHED (IPC) |

1

The present search report has been drawn up for all claims

| Place of search | Date of completion of the search | Examiner |
|---|---|---|
| The Hague | 16 May 2008 | Lonnoy, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 08 00 1073

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2008

| Patent document cited in search report | Publication date | Patent family member(s) | Publication date |
|---|---|---|---|
| WO 2004009028 A | 29-01-2004 | AU 2003259198 A1 | 09-02-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Ali A ; Avalos RT ; Ponimaskin E ; Nayak DP.** Influenza virus assembly: effect of influenza virus glycoproteins on the membrane association of M1 protein. *J Virol.,* 2000, vol. 74, 8709-8719
- **Anderson, RG ; Jacobson, K.** A role for lipid shells in targeting proteins to caveolae, rafts and other lipid domains. *Science,* 2002, vol. 296, 1821-1825
- **Bacia K ; Schuette CG ; Kahya N ; Jahn R ; Schwille P.** SNAREs prefer liquid-disordered over "raft" (liquid-ordered) domains when reconstituted into giant unilamellar vesicles. *J Biol Chem.,* 2004, vol. 279, 37951-5
- **Barmann, S ; Ali, A ; Hui, EKW ; Adhikary, L ; Nayak, DP.** Transport of viral proteins to the apical membranes and interaction of matrix protein with glycoproteins in the assembly of influenza viruses. *Virus Res.,* 2001, vol. 77, 61-69
- **Baumgart, T ; Hess, ST ; Webb, WW.** Imaging coexisting fluid domains in biomembrane models coupling curvature and line tension. *Nature,* 2003, vol. 425, 821-825
- **Briggs, JAG ; Wilk, T ; Fuller, SD.** Do lipid rafts mediate virus assembly and pseudotyping?. *J. gen. Virol.,* 2003, vol. 84, 757-768
- **Carrasco, M ; Amorim, MJ ; Digard, P.** Lipid-raft dependent targeting of the influenza A virus nucleoprotein to the apical plasma membrane. *Traffic,* 2004, vol. 5, 979-992
- **Dietrich, C ; Bagatolli, LA ; Volovyk, ZN ; Thompson, NL ; Levi, M. ; Jacobson, K ; Gratton, E.** Lipid rafts reconstituted in model membranes. *Biophys. J.,* 2001, vol. 80, 1417-1428
- **Edidin, M.** The state of lipid rafts: from model membranes to cells. *Annu Rev Biophys Biomol Struct.,* 2003, vol. 32, 257-83
- **Epand, RM.** Do proteins facilitate formation of cholesterol-rich domains?. *Biochim. Biophys. Acta,* 2004, vol. 1666, 227-238
- **Fiedler, K ; Kobayashi, T ; Kurzchalia, TV ; Simons K.** Glycosphingolipid-enriched, detergent-insoluble complexes in protein sorting in epithelial cells. *Biochemistry,* 1993, vol. 32, 6365-6373
- **Gomez-Puertas P ; Albo C ; Perez-Pastrana E ; Vivo A ; Portela A.** Influenza virus matrix protein is the major driving force in virus budding. *J Virol.,* 2000, vol. 74, 11538-11547
- **Harder T ; Scheiffele P ; Verkade P ; Simons K.** Lipid domain structure of the plasma membrane revealed by patching of membrane components. *J Cell Biol.,* 1998, vol. 141, 929-942
- **Heerklotz, H.** Triton promotes domain formation in lipid raft mixtures. *Biophys J.,* 2002, vol. 83, 2693-2701
- **Jin, H ; Leser, GP ; Zhang, J ; Lamb, RA.** Influenza virus hemagglutinin and neuraminidase cytoplasmic tails control particle shape. *EMBO. J,* 1997, vol. 16, 1236-1247
- **Keller, P ; Simons, K.** Cholesterol is required for surface transport of influenza virus hemagglutinin. *J. Cell Biol.,* 1998, vol. 140, 1357-1367
- **LaCour T ; Kiemer L ; Mølgaard A ; Gupta R ; Skriver K ; Brunak S.** Analysis and prediction of leucine-rich nuclear export signals. *Protein Engineering, Design & Selection,* 2004, vol. 17, 527-536
- **Latham T ; Galarza JM.** Formation of wild-type and chimeric influenza virus-like particles following simultaneous expression of only four structural proteins. *J Virol.,* 2001, vol. 75, 6154-6165
- **Legler, D. F. ; Doucey, M. et al.** Differential insertion of GPI-anchored GFPs into lipid rafts of live cells. *The FASEB Journal,* 2005, vol. 19, 73-75
- **Mayor, S ; Rao, M.** Rafts: Scale dependent, active lipid organization at the cell surface. *Traffic,* 2004, vol. 5, 231-240
- **Melkonian KA ; Ostermeyer AG ; Chen JZ ; Roth MG ; Brown DA.** Role of lipid modifications in targeting proteins to detergent-resistant membrane rafts. Many raft proteins are acylated, while few are prenylated. *J Biol Chem.,* 1999, vol. 274, 3910-3917
- **Munro, S.** Lipid rafts: elusive or illusive?. *Cell,* 2003, vol. 115, 377-388
- **Rizzo, M. A. ; G. H. Springer et al.** An improved cyan fluorescent protein variant useful for FRET. *Nat Biotechnol,* 2004, vol. 22 (4), 445-9
- **Scheiffele, P ; Roth, MG ; Simons, K.** Interaction of influenza virus hemagglutinin with sphingolipid-cholesterol membrane domains via its transmembrane domain. *EMBO-J.,* 1997, vol. 16, 5501-5508
- **Scheiffele, P ; Rietveld, A ; Wilk, T ; Simons, K.** Influenza virus select ordered lipid domains during budding from the plasma membrane. *J. Biol. Chem.,* 1999, vol. 274, 2038-2044

- **Schmitt, AP ; Lamb, RA.** Escaping from the cell: assembly and budding of negative-strand RNA viruses. *Curr Top Microbiol Immunol.,* 2004, vol. 283, 145-196
- **Schroeder C ; Heider H ; Moncke-Buchner E ; Lin TI.** The influenza virus ion channel and maturation cofactor M2 is a cholesterol-binding protein. *Eur Biophys J,* 25 June 2004
- **Sekar, RB ; Periasamy, A.** Fluorescence resonance energy transfer (FRET) microscopy imaging of live cell protein localizations. *J. Cell Biol.,* 2003, vol. 160, 629-633
- **Sha, B. ; Luo, M.** Structure of a bifunctional membrane-RNA binding protein, influenza virus matrix protein M1. *Nat. Struct. Biol.,* 1997, vol. 4, 239-244
- **Sharma, P. ; Varma, R. ; Sarasij, RC, Ira ; Gousset, K. ; Krishnamoorthy, G. ; Rao, M. ; Mayor, S.** Nanoscale organization of multiple GPI-anchored proteins in living cell membranes. *Cell,* 2004, vol. 116, 577-589
- **Simons, K ; van Meer, G.** Lipid sorting in epithelial cells. *Biochemistry,* 1988, vol. 27, 6197-6202
- **Simons K ; Ikonen E.** Functional rafts in cell membranes. *Nature,* 1997, vol. 387, 569-72
- **Simons, K. ; Vaz, WLC.** Model systems, lipid rafts and cell membranes. *Annu Rev Biophys Biomol Struct,* 2004, vol. 33, 269-295
- **Skibens, JE ; Roth, MG ; Matlin, KS.** Differential extractability of influenza virus hemagglutinin during intracellular transport in polarized epithelial cells and nonpolar fibroblasts. *J. Cell. Biol.,* 1989, vol. 108, 821-832
- **Tall, RD ; Alonso, MA ; Roth, MG.** Features of influenza HA required for apical sorting differ from those required for assocation with DRMs or MAL. *Traffic,* 2003, vol. 4, 838-849
- **Takeda M ; Leser GP ; Russell CJ ; Lamb RA.** Influenza virus hemagglutinin concentrates in lipid raft microdomains for efficient viral fusion. *Proc Natl Acad Sci U S A.,* 2003, vol. 100, 14610-7
- **Varma., R ; Mayor, S.** GPI-anchored proteins are organized in submicron domains at the cell surface. *Nature,* 1998, vol. 394, 798-801
- **Wagner R ; Herwig A ; Azzouz N ; Klenk HD.** Acylation-Mediated Membrane Anchoring of Avian Influenza Virus Hemagglutinin Is Essential for Fusion Pore Formation and Virus Infectivity. *J Virol,* 2005, vol. 79, 6449-6458
- **Zacharias, DA ; Violin, JD ; Newton, AC ; Tsien, RY.** Partitioning of lipid-modified monomeric GFPs into membrane microdomains of live cells. *Science,* 2002, vol. 296, 913-916
- **Zhang, J ; Pekosz, A ; Lamb, RA.** Influenza virus assembly and lipid raft microdomains: a role for the cytoplsmic tails of the spike glycoproteins. *J. Virol,* 2000, vol. 74, 4634-4644
- **Zhao H ; Ekstrom M ; Garoff H.** The M1 and NP proteins of influenza A virus form homo- but not heterooligomeric complexes when coexpressed in BHK-21 cells. *J Gen Virol.,* 1998, vol. 79, 2435-46

SEQUENCE LISTING

<110> Freie Universität Berlin und Humboldt Universität

<120> Method for Studying Virus Particle Release

<130> FB19496

<160> 71

<170> PatentIn version 3.3

<210> 1
<211> 1530
<212> DNA
<213> Artificial

<220>
<223> CFP-M1-NES

<400> 1
atggtgagca agggcgagga gctgttcacc ggggtggtgc ccatcctggt cgagctggac 60

ggcgacgtaa acggccacaa gttcagcgtg tccggcgagg gcgagggcga tgccacctac 120

ggcaagctga ccctgaagtt catctgcacc accggcaagc tgcccgtgcc ctggcccacc 180

ctcgtgacca ccctgacctg gggcgtgcag tgcttcagcc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacat cagccacaac gtctatatca ccgccgacaa gcagaagaac 480

ggcatcaagg ccaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actctcggca tggacgagct gtacaagtcc 720

ggactcagat ctcgagctat gagtcttcta accgaggttg aaacgtacgt tctctctatc 780

atcccatcag gcccccctcaa agccgagatc gcgcagagac ttgaagatgt ctttgcaggg 840

aaaaacacag accttgaggt tctcatggaa tggctaaaga caagaccaat cctgtcacct 900

ctgactaaag ggattttggg gtttgtgttt acgctcaccg tgcccagtga gcaaggactg 960

cagcgtagac gctttgtcca aaatgcccta aatgggaatg gggatccaaa taacatggat 1020

aaagccgtca aactatacag gaagttgaaa agggagataa cattctatgg agctaaggaa 1080

gtggcactca gttactctac tggagcactt gccagttgta tgggcctcat atacaacaga 1140

atgggaactg tgaccacaga ggtggcattt ggcctagtgt gtgccacttg tgagcagatt 1200

gctgattcac agcatcggtc tcacagacag atggtggcta ccaccaatcc actaatcagg 1260

catgagaaca gaatggtaat ggccagcact acagctaagg ctatggagca aatggctggg 1320

tcaattgaac aggcagcgga ggccatggag gttgctagcc aggctaggca gatggtgcag 1380

gcaatgagga caattgggac tcatcctagc tccagtgctg gtctgaaaga tgatcttctt 1440

gaaaatttgc aggcctacca gaaacggatg ggagtgcaga tgcaacgatt caagcttcag 1500

ctaccaccgc ttgagagact tactctttga 1530

<210> 2
<211> 509
<212> PRT
<213> Artificial

<220>
<223> CFP-M1-NES

<400> 2

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
180 185 190

Pro Val Leu Leu Pro Asp Asn His Tyr Leu Ser Thr Gln Ser Ala Leu
195 200 205

Ser Lys Asp Pro Asn Glu Lys Arg Asp His Met Val Leu Leu Glu Phe
210 215 220

Val Thr Ala Ala Gly Ile Thr Leu Gly Met Asp Glu Leu Tyr Lys Ser
225 230 235 240

Gly Leu Arg Ser Arg Ala Met Ser Leu Leu Thr Glu Val Glu Thr Tyr
245 250 255

Val Leu Ser Ile Ile Pro Ser Gly Pro Leu Lys Ala Glu Ile Ala Gln
260 265 270

Arg Leu Glu Asp Val Phe Ala Gly Lys Asn Thr Asp Leu Glu Val Leu
275 280 285

Met Glu Trp Leu Lys Thr Arg Pro Ile Leu Ser Pro Leu Thr Lys Gly
290 295 300

Ile Leu Gly Phe Val Phe Thr Leu Thr Val Pro Ser Glu Gln Gly Leu
305 310 315 320

Gln Arg Arg Arg Phe Val Gln Asn Ala Leu Asn Gly Asn Gly Asp Pro
325 330 335

Asn Asn Met Asp Lys Ala Val Lys Leu Tyr Arg Lys Leu Lys Arg Glu
340 345 350

Ile Thr Phe Tyr Gly Ala Lys Glu Val Ala Leu Ser Tyr Ser Thr Gly
355 360 365

Ala Leu Ala Ser Cys Met Gly Leu Ile Tyr Asn Arg Met Gly Thr Val
370 375 380

Thr Thr Glu Val Ala Phe Gly Leu Val Cys Ala Thr Cys Glu Gln Ile
385 390 395 400

Ala Asp Ser Gln His Arg Ser His Arg Gln Met Val Ala Thr Thr Asn
405 410 415

Pro Leu Ile Arg His Glu Asn Arg Met Val Met Ala Ser Thr Thr Ala

420 425 430

Lys Ala Met Glu Gln Met Ala Gly Ser Ile Glu Gln Ala Ala Glu Ala
435 440 445

Met Glu Val Ala Ser Gln Ala Arg Gln Met Val Gln Ala Met Arg Thr
450 455 460

Ile Gly Thr His Pro Ser Ser Ser Ala Gly Leu Lys Asp Asp Leu Leu
465 470 475 480

Glu Asn Leu Gln Ala Tyr Gln Lys Arg Met Gly Val Gln Met Gln Arg
485 490 495

Phe Lys Leu Gln Leu Pro Pro Leu Glu Arg Leu Thr Leu
500 505

<210> 3
<211> 1527
<212> DNA
<213> Artificial

<220>
<223> Myr-M1-CFP

<400> 3
atgggatcaa tcaaatcaaa gatgagtctt ctaaccgagg ttgaaacgta cgttctctct 60

atcatcccat caggcccccct caaagccgag atcgcgcaga gacttgaaga tgtctttgca 120

gggaaaaaca cagaccttga ggttctcatg gaatggctaa agacaagacc aatcctgtca 180

cctctgacta aagggatttt ggggtttgtg tttacgctca ccgtgcccag tgagcaagga 240

ctgcagcgta gacgctttgt ccaaaatgcc ctaaatggga atggggatcc aaataacatg 300

gataaagccg tcaaactata caggaagttg aaaagggaga taacattcta tggagctaag 360

gaagtggcac tcagttactc tactggagca cttgccagtt gtatgggcct catatacaac 420

agaatgggaa ctgtgaccac agaggtggca tttggcctag tgtgtgccac ttgtgagcag 480

attgctgatt cacagcatcg gtctcacaga cagatggtgg ctaccaccaa tccactaatc 540

aggcatgaga acagaatggt aatggccagc actacagcta aggctatgga gcaaatggct 600

gggtcaattg aacaggcagc ggaggccatg gaggttgcta gccaggctag gcagatggtg 660

caggcaatga ggacaattgg gactcatcct agctccagtg ctggtctgaa agatgatctt 720

cttgaaaatt tgcaggccta ccagaaacgg atgggagtgc agatgcaacg attcaagccg 780

cgggcccggg atccaccggt cgccaccatg gtgagcaagg gcgaggagct gttcaccggg 840

gtggtgccca tcctggtcga gctggacggc gacgtaaacg gccacaagtt cagcgtgtcc 900

ggcgagggcg agggcgatgc cacctacggc aagctgaccc tgaagttcat ctgcaccacc 960

ggcaagctgc ccgtgccctg gcccaccctc gtgaccaccc tgacctgggg cgtgcagtgc 1020

ttcagccgct accccgacca catgaagcag cacgacttct tcaagtccgc catgcccgaa 1080

ggctacgtcc aggagcgcac catcttcttc aaggacgacg gcaactacaa gacccgcgcc 1140

gaggtgaagt tcgagggcga caccctggtg aaccgcatcg agctgaaggg catcgacttc 1200

aaggaggacg gcaacatcct ggggcacaag ctggagtaca actacatcag ccacaacgtc 1260

tatatcaccg ccgacaagca gaagaacggc atcaaggcca acttcaagat ccgccacaac 1320

atcgaggacg gcagcgtgca gctcgccgac cactaccagc agaacacccc catcggcgac 1380

ggccccgtgc tgctgcccga caaccactac ctgagcaccc agtccgccct gagcaaagac 1440

cccaacgaga agcgcgatca catggtcctg ctggagttcg tgaccgccgc cgggatcact 1500

ctcggcatgg acgagctgta caagtaa 1527

<210> 4
<211> 508
<212> PRT
<213> Artificial

<220>
<223> Myr-M1-CFP

<400> 4

Met Gly Ser Ile Lys Ser Lys Met Ser Leu Leu Thr Glu Val Glu Thr
1 5 10 15

Tyr Val Leu Ser Ile Ile Pro Ser Gly Pro Leu Lys Ala Glu Ile Ala
20 25 30

Gln Arg Leu Glu Asp Val Phe Ala Gly Lys Asn Thr Asp Leu Glu Val
35 40 45

Leu Met Glu Trp Leu Lys Thr Arg Pro Ile Leu Ser Pro Leu Thr Lys
50 55 60

Gly Ile Leu Gly Phe Val Phe Thr Leu Thr Val Pro Ser Glu Gln Gly
65 70 75 80

Leu Gln Arg Arg Arg Phe Val Gln Asn Ala Leu Asn Gly Asn Gly Asp
85 90 95

Pro Asn Asn Met Asp Lys Ala Val Lys Leu Tyr Arg Lys Leu Lys Arg
100 105 110

Glu Ile Thr Phe Tyr Gly Ala Lys Glu Val Ala Leu Ser Tyr Ser Thr
115 120 125

```
Gly Ala Leu Ala Ser Cys Met Gly Leu Ile Tyr Asn Arg Met Gly Thr
    130                 135                 140

Val Thr Thr Glu Val Ala Phe Gly Leu Val Cys Ala Thr Cys Glu Gln
145                 150                 155                 160

Ile Ala Asp Ser Gln His Arg Ser His Arg Gln Met Val Ala Thr Thr
                165                 170                 175

Asn Pro Leu Ile Arg His Glu Asn Arg Met Val Met Ala Ser Thr Thr
            180                 185                 190

Ala Lys Ala Met Glu Gln Met Ala Gly Ser Ile Glu Gln Ala Ala Glu
        195                 200                 205

Ala Met Glu Val Ala Ser Gln Ala Arg Gln Met Val Gln Ala Met Arg
    210                 215                 220

Thr Ile Gly Thr His Pro Ser Ser Ser Ala Gly Leu Lys Asp Asp Leu
225                 230                 235                 240

Leu Glu Asn Leu Gln Ala Tyr Gln Lys Arg Met Gly Val Gln Met Gln
                245                 250                 255

Arg Phe Lys Pro Arg Ala Arg Asp Pro Pro Val Ala Thr Met Val Ser
            260                 265                 270

Lys Gly Glu Glu Leu Phe Thr Gly Val Val Pro Ile Leu Val Glu Leu
        275                 280                 285

Asp Gly Asp Val Asn Gly His Lys Phe Ser Val Ser Gly Glu Gly Glu
    290                 295                 300

Gly Asp Ala Thr Tyr Gly Lys Leu Thr Leu Lys Phe Ile Cys Thr Thr
305                 310                 315                 320

Gly Lys Leu Pro Val Pro Trp Pro Thr Leu Val Thr Thr Leu Thr Trp
                325                 330                 335

Gly Val Gln Cys Phe Ser Arg Tyr Pro Asp His Met Lys Gln His Asp
            340                 345                 350

Phe Phe Lys Ser Ala Met Pro Glu Gly Tyr Val Gln Glu Arg Thr Ile
        355                 360                 365

Phe Phe Lys Asp Asp Gly Asn Tyr Lys Thr Arg Ala Glu Val Lys Phe
    370                 375                 380
```

Glu Gly Asp Thr Leu Val Asn Arg Ile Glu Leu Lys Gly Ile Asp Phe
385 390 395 400

Lys Glu Asp Gly Asn Ile Leu Gly His Lys Leu Glu Tyr Asn Tyr Ile
405 410 415

Ser His Asn Val Tyr Ile Thr Ala Asp Lys Gln Lys Asn Gly Ile Lys
420 425 430

Ala Asn Phe Lys Ile Arg His Asn Ile Glu Asp Gly Ser Val Gln Leu
435 440 445

Ala Asp His Tyr Gln Gln Asn Thr Pro Ile Gly Asp Gly Pro Val Leu
450 455 460

Leu Pro Asp Asn His Tyr Leu Ser Thr Gln Ser Ala Leu Ser Lys Asp
465 470 475 480

Pro Asn Glu Lys Arg Asp His Met Val Leu Leu Glu Phe Val Thr Ala
485 490 495

Ala Gly Ile Thr Leu Gly Met Asp Glu Leu Tyr Lys
500 505

<210> 5
<211> 1035
<212> DNA
<213> Artificial

<220>
<223> Artifical-HA-YFP

<400> 5
atgaacactc aaatcctggt tttcgccctt gtggcagtca ttcccacaaa tgcagacaaa 60

atttgtcttc cgcgggcccg ggatccaccg gtcgccacca tggtgagcaa gggcgaggag 120

ctgttcaccg gggtggtgcc catcctggtc gagctggacg gcgacgtaaa cggccacaag 180

ttcagcgtgt ccggcgaggg cgagggcgat gccacctacg gcaagctgac cctgaagttc 240

atctgcacca ccggcaagct gcccgtgccc tggcccaccc tcgtgaccac cttcggctac 300

ggcctgcagt gcttcgcccg ctaccccgac cacatgaagc agcacgactt cttcaagtcc 360

gccatgcccg aaggctacgt ccaggagcgc accatcttct tcaaggacga cggcaactac 420

aagacccgcg ccgaggtgaa gttcgagggc gacaccctgg tgaaccgcat cgagctgaag 480

ggcatcgact tcaaggagga cggcaacatc ctggggcaca agctggagta caactacaac 540

agccacaacg tctatatcat ggccgacaag cagaagaacg gcatcaaggt gaacttcaag 600

atccgccaca acatcgagga cggcagcgtg cagctcgccg accactacca gcagaacacc 660

cccatcggcg acggccccgt gctgctgccc gacaaccact acctgagcta ccagtccgcc 720

ctgagcaaag accccaacga gaagcgcgat cacatggtcc tgctggagtt cgtgaccgcc 780

gccgggatca ctctcggcat ggacgagctg tacaaggcta gtataaggaa caatacttat 840

gatcacagca aatacagaga agaagcgatg caaaatagaa tacaaattga cccagtcaaa 900

ttgagtagtg gctacaaaga tgtgatactt tggtttagct tcggggcatc atgctttttg 960

cttcttgcca ttgcaatggg ccttgttttc atatgtgtga agaacggaaa catgcggtgc 1020

actatttgta tataa 1035

<210> 6
<211> 344
<212> PRT
<213> Artificial

<220>
<223> Artifical-HA-YFP

<400> 6

Met Asn Thr Gln Ile Leu Val Phe Ala Leu Val Ala Val Ile Pro Thr
1 5 10 15

Asn Ala Asp Lys Ile Cys Leu Pro Arg Ala Arg Asp Pro Pro Val Ala
20 25 30

Thr Met Val Ser Lys Gly Glu Glu Leu Phe Thr Gly Val Val Pro Ile
35 40 45

Leu Val Glu Leu Asp Gly Asp Val Asn Gly His Lys Phe Ser Val Ser
50 55 60

Gly Glu Gly Glu Gly Asp Ala Thr Tyr Gly Lys Leu Thr Leu Lys Phe
65 70 75 80

Ile Cys Thr Thr Gly Lys Leu Pro Val Pro Trp Pro Thr Leu Val Thr
85 90 95

Thr Phe Gly Tyr Gly Leu Gln Cys Phe Ala Arg Tyr Pro Asp His Met
100 105 110

Lys Gln His Asp Phe Phe Lys Ser Ala Met Pro Glu Gly Tyr Val Gln
115 120 125

Glu Arg Thr Ile Phe Phe Lys Asp Asp Gly Asn Tyr Lys Thr Arg Ala
130 135 140

```
Glu Val Lys Phe Glu Gly Asp Thr Leu Val Asn Arg Ile Glu Leu Lys
145                 150                 155                 160

Gly Ile Asp Phe Lys Glu Asp Gly Asn Ile Leu Gly His Lys Leu Glu
                165                 170                 175

Tyr Asn Tyr Asn Ser His Asn Val Tyr Ile Met Ala Asp Lys Gln Lys
            180                 185                 190

Asn Gly Ile Lys Val Asn Phe Lys Ile Arg His Asn Ile Glu Asp Gly
        195                 200                 205

Ser Val Gln Leu Ala Asp His Tyr Gln Gln Asn Thr Pro Ile Gly Asp
    210                 215                 220

Gly Pro Val Leu Leu Pro Asp Asn His Tyr Leu Ser Tyr Gln Ser Ala
225                 230                 235                 240

Leu Ser Lys Asp Pro Asn Glu Lys Arg Asp His Met Val Leu Leu Glu
                245                 250                 255

Phe Val Thr Ala Ala Gly Ile Thr Leu Gly Met Asp Glu Leu Tyr Lys
            260                 265                 270

Ala Ser Ile Arg Asn Asn Thr Tyr Asp His Ser Lys Tyr Arg Glu Glu
        275                 280                 285

Ala Met Gln Asn Arg Ile Gln Ile Asp Pro Val Lys Leu Ser Ser Gly
    290                 295                 300

Tyr Lys Asp Val Ile Leu Trp Phe Ser Phe Gly Ala Ser Cys Phe Leu
305                 310                 315                 320

Leu Leu Ala Ile Ala Met Gly Leu Val Phe Ile Cys Val Lys Asn Gly
                325                 330                 335

Asn Met Arg Cys Thr Ile Cys Ile
            340
```

<210> 7
<211> 1035
<212> DNA
<213> Artificial

<220>
<223> Artifical-HA-CFP

<400> 7
atgaacactc aaatcctggt tttcgccctt gtggcagtca ttcccacaaa tgcagacaaa 60

```
atttgtcttc cgcgggcccg ggatccaccg gtcgccacca tggtgagcaa gggcgaggag    120

ctgttcaccg gggtggtgcc catcctggtc gagctggacg gcgacgtaaa cggccacaag    180

ttcagcgtgt ccggcgaggg cgagggcgat gccacctacg gcaagctgac cctgaagttc    240

atctgcacca ccggcaagct gcccgtgccc tggcccaccc tcgtgaccac cctgacctgg    300

ggcgtgcagt gcttcagccg ctaccccgac cacatgaagc agcacgactt cttcaagtcc    360

gccatgcccg aaggctacgt ccaggagcgc accatcttct tcaaggacga cggcaactac    420

aagacccgcg ccgaggtgaa gttcgagggc gacaccctgg tgaaccgcat cgagctgaag    480

ggcatcgact tcaaggagga cggcaacatc ctggggcaca agctggagta caactacatc    540

agccacaacg tctatatcac cgccgacaag cagaagaacg gcatcaaggc caacttcaag    600

atccgccaca acatcgagga cggcagcgtg cagctcgccg accactacca gcagaacacc    660

cccatcggcg acggccccgt gctgctgccc gacaaccact acctgagcac ccagtccgcc    720

ctgagcaaag accccaacga gaagcgcgat cacatggtcc tgctggagtt cgtgaccgcc    780

gccgggatca ctctcggcat ggacgagctg tacaaggcta gtataaggaa caatacttat    840

gatcacagca aatacagaga agaagcgatg caaaatagaa tacaaattga cccagtcaaa    900

ttgagtagtg gctacaaaga tgtgatactt tggtttagct tcggggcatc atgctttttg    960

cttcttgcca ttgcaatggg ccttgttttc atatgtgtga agaacggaaa catgcggtgc   1020

actatttgta tataa                                                    1035
```

<210> 8
<211> 344
<212> PRT
<213> Artificial

<220>
<223> Artifical-HA-CFP

<400> 8

```
Met Asn Thr Gln Ile Leu Val Phe Ala Leu Val Ala Val Ile Pro Thr
1               5                   10                  15

Asn Ala Asp Lys Ile Cys Leu Pro Arg Ala Arg Asp Pro Pro Val Ala
            20                  25                  30

Thr Met Val Ser Lys Gly Glu Glu Leu Phe Thr Gly Val Val Pro Ile
        35                  40                  45

Leu Val Glu Leu Asp Gly Asp Val Asn Gly His Lys Phe Ser Val Ser
    50                  55                  60

Gly Glu Gly Glu Gly Asp Ala Thr Tyr Gly Lys Leu Thr Leu Lys Phe
65                  70                  75                  80
```

```
Ile Cys Thr Thr Gly Lys Leu Pro Val Pro Trp Pro Thr Leu Val Thr
                85                  90                  95

Thr Leu Thr Trp Gly Val Gln Cys Phe Ser Arg Tyr Pro Asp His Met
            100                 105                 110

Lys Gln His Asp Phe Phe Lys Ser Ala Met Pro Glu Gly Tyr Val Gln
        115                 120                 125

Glu Arg Thr Ile Phe Phe Lys Asp Asp Gly Asn Tyr Lys Thr Arg Ala
    130                 135                 140

Glu Val Lys Phe Glu Gly Asp Thr Leu Val Asn Arg Ile Glu Leu Lys
145                 150                 155                 160

Gly Ile Asp Phe Lys Glu Asp Gly Asn Ile Leu Gly His Lys Leu Glu
                165                 170                 175

Tyr Asn Tyr Ile Ser His Asn Val Tyr Ile Thr Ala Asp Lys Gln Lys
            180                 185                 190

Asn Gly Ile Lys Ala Asn Phe Lys Ile Arg His Asn Ile Glu Asp Gly
        195                 200                 205

Ser Val Gln Leu Ala Asp His Tyr Gln Gln Asn Thr Pro Ile Gly Asp
    210                 215                 220

Gly Pro Val Leu Leu Pro Asp Asn His Tyr Leu Ser Thr Gln Ser Ala
225                 230                 235                 240

Leu Ser Lys Asp Pro Asn Glu Lys Arg Asp His Met Val Leu Leu Glu
                245                 250                 255

Phe Val Thr Ala Ala Gly Ile Thr Leu Gly Met Asp Glu Leu Tyr Lys
            260                 265                 270

Ala Ser Ile Arg Asn Asn Thr Tyr Asp His Ser Lys Tyr Arg Glu Glu
        275                 280                 285

Ala Met Gln Asn Arg Ile Gln Ile Asp Pro Val Lys Leu Ser Ser Gly
    290                 295                 300

Tyr Lys Asp Val Ile Leu Trp Phe Ser Phe Gly Ala Ser Cys Phe Leu
305                 310                 315                 320

Leu Leu Ala Ile Ala Met Gly Leu Val Phe Ile Cys Val Lys Asn Gly
```

325 330 335

Asn Met Arg Cys Thr Ile Cys Ile
340

<210> 9
<211> 1035
<212> DNA
<213> Artificial

<220>
<223> Artifical-HA-YFPM3

<400> 9
atgaacactc aaatcctggt tttcgccctt gtggcagtca ttcccacaaa tgcagacaaa 60

atttgtcttc cgcgggcccg ggatccaccg gtcgccacca tggtgagcaa gggcgaggag 120

ctgttcaccg gggtggtgcc catcctggtc gagctggacg gcgacgtaaa cggccacaag 180

ttcagcgtgt ccggcgaggg cgagggcgat gccacctacg gcaagctgac cctgaagttc 240

atctgcacca ccggcaagct gcccgtgccc tggcccaccc tcgtgaccac cttcggctac 300

ggcctgcagt gcttcgcccg ctaccccgac cacatgaagc agcacgactt cttcaagtcc 360

gccatgcccg aaggctacgt ccaggagcgc accatcttct tcaaggacga cggcaactac 420

aagacccgcg ccgaggtgaa gttcgagggc gacaccctgg tgaaccgcat cgagctgaag 480

ggcatcgact tcaaggagga cggcaacatc ctggggcaca agctggagta caactacaac 540

agccacaacg tctatatcat ggccgacaag cagaagaacg gcatcaaggt gaacttcaag 600

atccgccaca acatcgagga cggcagcgtg cagctcgccg accactacca gcagaacacc 660

cccatcggcg acggccccgt gctgctgccc gacaaccact acctgagcta ccagtccgcc 720

ctgagcaaag accccaacga gaagcgcgat cacatggtcc tgctggagtt cgtgaccgcc 780

gccgggatca ctctcggcat ggacgagctg tacaaggcta gtataaggaa caatacttat 840

gatcacagca aatacagaga agaagcgatg caaaatagaa tacaaattga cccagtcaaa 900

ttgagtagtg gctacaaaga tgtgatactt tggtttagct tcggggcatc atgctttttg 960

cttcttgcca ttgcaatggg ccttgttttc atatctgtga agaacggaaa catgcggtcc 1020

actatttcta tataa 1035

<210> 10
<211> 344
<212> PRT
<213> Artificial

<220>
<223> Artifical-HA-YFPM3

<400> 10

```
Met Asn Thr Gln Ile Leu Val Phe Ala Leu Val Ala Val Ile Pro Thr
1               5                   10                  15

Asn Ala Asp Lys Ile Cys Leu Pro Arg Ala Arg Asp Pro Pro Val Ala
            20                  25                  30

Thr Met Val Ser Lys Gly Glu Glu Leu Phe Thr Gly Val Val Pro Ile
        35                  40                  45

Leu Val Glu Leu Asp Gly Asp Val Asn Gly His Lys Phe Ser Val Ser
    50                  55                  60

Gly Glu Gly Glu Gly Asp Ala Thr Tyr Gly Lys Leu Thr Leu Lys Phe
65                  70                  75                  80

Ile Cys Thr Thr Gly Lys Leu Pro Val Pro Trp Pro Thr Leu Val Thr
                85                  90                  95

Thr Phe Gly Tyr Gly Leu Gln Cys Phe Ala Arg Tyr Pro Asp His Met
            100                 105                 110

Lys Gln His Asp Phe Phe Lys Ser Ala Met Pro Glu Gly Tyr Val Gln
        115                 120                 125

Glu Arg Thr Ile Phe Phe Lys Asp Asp Gly Asn Tyr Lys Thr Arg Ala
    130                 135                 140

Glu Val Lys Phe Glu Gly Asp Thr Leu Val Asn Arg Ile Glu Leu Lys
145                 150                 155                 160

Gly Ile Asp Phe Lys Glu Asp Gly Asn Ile Leu Gly His Lys Leu Glu
                165                 170                 175

Tyr Asn Tyr Asn Ser His Asn Val Tyr Ile Met Ala Asp Lys Gln Lys
            180                 185                 190

Asn Gly Ile Lys Val Asn Phe Lys Ile Arg His Asn Ile Glu Asp Gly
        195                 200                 205

Ser Val Gln Leu Ala Asp His Tyr Gln Gln Asn Thr Pro Ile Gly Asp
    210                 215                 220

Gly Pro Val Leu Leu Pro Asp Asn His Tyr Leu Ser Tyr Gln Ser Ala
225                 230                 235                 240

Leu Ser Lys Asp Pro Asn Glu Lys Arg Asp His Met Val Leu Leu Glu
                245                 250                 255
```

Phe Val Thr Ala Ala Gly Ile Thr Leu Gly Met Asp Glu Leu Tyr Lys
260 265 270

Ala Ser Ile Arg Asn Asn Thr Tyr Asp His Ser Lys Tyr Arg Glu Glu
275 280 285

Ala Met Gln Asn Arg Ile Gln Ile Asp Pro Val Lys Leu Ser Ser Gly
290 295 300

Tyr Lys Asp Val Ile Leu Trp Phe Ser Phe Gly Ala Ser Cys Phe Leu
305 310 315 320

Leu Leu Ala Ile Ala Met Gly Leu Val Phe Ile Ser Val Lys Asn Gly
325 330 335

Asn Met Arg Ser Thr Ile Ser Ile
340

<210> 11
<211> 1035
<212> DNA
<213> Artificial

<220>
<223> Artifical-HA-CFPM3

<400> 11
atgaacactc aaatcctggt tttcgccctt gtggcagtca ttcccacaaa tgcagacaaa 60

atttgtcttc cgcgggcccg ggatccaccg gtcgccacca tggtgagcaa gggcgaggag 120

ctgttcaccg gggtggtgcc catcctggtc gagctggacg gcgacgtaaa cggccacaag 180

ttcagcgtgt ccggcgaggg cgagggcgat gccacctacg gcaagctgac cctgaagttc 240

atctgcacca ccggcaagct gcccgtgccc tggcccaccc tcgtgaccac cctgacctgg 300

ggcgtgcagt gcttcagccg ctaccccgac cacatgaagc agcacgactt cttcaagtcc 360

gccatgcccg aaggctacgt ccaggagcgc accatcttct tcaaggacga cggcaactac 420

aagacccgcg ccgaggtgaa gttcgagggc gacaccctgg tgaaccgcat cgagctgaag 480

ggcatcgact tcaaggagga cggcaacatc ctggggcaca agctggagta caactacatc 540

agccacaacg tctatatcac cgccgacaag cagaagaacg gcatcaaggc caacttcaag 600

atccgccaca acatcgagga cggcagcgtg cagctcgccg accactacca gcagaacacc 660

cccatcggcg acggccccgt gctgctgccc gacaaccact acctgagcac ccagtccgcc 720

ctgagcaaag accccaacga gaagcgcgat cacatggtcc tgctggagtt cgtgaccgcc 780

gccgggatca ctctcggcat ggacgagctg tacaaggcta gtataaggaa caatacttat 840

gatcacagca aatacagaga agaagcgatg caaaatagaa tacaaattga cccagtcaaa 900

```
ttgagtagtg gctacaaaga tgtgatactt tggtttagct tcggggcatc atgcttttg    960

cttcttgcca ttgcaatggg ccttgttttc atatctgtga agaacggaaa catgcggtcc   1020

actatttcta tataa                                                    1035


<210>  12
<211>  344
<212>  PRT
<213>  Artificial

<220>
<223>  Artifical-HA-CFPM3

<400>  12

Met Asn Thr Gln Ile Leu Val Phe Ala Leu Val Ala Val Ile Pro Thr
1               5                   10                  15

Asn Ala Asp Lys Ile Cys Leu Pro Arg Ala Arg Asp Pro Pro Val Ala
            20                  25                  30

Thr Met Val Ser Lys Gly Glu Glu Leu Phe Thr Gly Val Val Pro Ile
        35                  40                  45

Leu Val Glu Leu Asp Gly Asp Val Asn Gly His Lys Phe Ser Val Ser
    50                  55                  60

Gly Glu Gly Glu Gly Asp Ala Thr Tyr Gly Lys Leu Thr Leu Lys Phe
65                  70                  75                  80

Ile Cys Thr Thr Gly Lys Leu Pro Val Pro Trp Pro Thr Leu Val Thr
                85                  90                  95

Thr Leu Thr Trp Gly Val Gln Cys Phe Ser Arg Tyr Pro Asp His Met
            100                 105                 110

Lys Gln His Asp Phe Phe Lys Ser Ala Met Pro Glu Gly Tyr Val Gln
        115                 120                 125

Glu Arg Thr Ile Phe Phe Lys Asp Asp Gly Asn Tyr Lys Thr Arg Ala
    130                 135                 140

Glu Val Lys Phe Glu Gly Asp Thr Leu Val Asn Arg Ile Glu Leu Lys
145                 150                 155                 160

Gly Ile Asp Phe Lys Glu Asp Gly Asn Ile Leu Gly His Lys Leu Glu
                165                 170                 175

Tyr Asn Tyr Ile Ser His Asn Val Tyr Ile Thr Ala Asp Lys Gln Lys
```

180 185 190

Asn Gly Ile Lys Ala Asn Phe Lys Ile Arg His Asn Ile Glu Asp Gly
195 200 205

Ser Val Gln Leu Ala Asp His Tyr Gln Gln Asn Thr Pro Ile Gly Asp
210 215 220

Gly Pro Val Leu Leu Pro Asp Asn His Tyr Leu Ser Thr Gln Ser Ala
225 230 235 240

Leu Ser Lys Asp Pro Asn Glu Lys Arg Asp His Met Val Leu Leu Glu
245 250 255

Phe Val Thr Ala Ala Gly Ile Thr Leu Gly Met Asp Glu Leu Tyr Lys
260 265 270

Ala Ser Ile Arg Asn Asn Thr Tyr Asp His Ser Lys Tyr Arg Glu Glu
275 280 285

Ala Met Gln Asn Arg Ile Gln Ile Asp Pro Val Lys Leu Ser Ser Gly
290 295 300

Tyr Lys Asp Val Ile Leu Trp Phe Ser Phe Gly Ala Ser Cys Phe Leu
305 310 315 320

Leu Leu Ala Ile Ala Met Gly Leu Val Phe Ile Ser Val Lys Asn Gly
325 330 335

Asn Met Arg Ser Thr Ile Ser Ile
340

<210> 13
<211> 929
<212> DNA
<213> Artificial

<220>
<223> GPI(DAF)-CFP

<400> 13
atgaacactc aaatcctggt tttcgccctt gtggcagtca ttcccacaaa tgcagacaaa 60

atttgtcttc cgcgggcccg ggatccaccg gtcgccacca tggtgagcaa gggcgaggag 120

ctgttcaccg gggtggtgcc catcctggtc gagctggacg gcgacgtaaa cggccacaag 180

ttcagcgtgt ccggcgaggg cgagggcgat gccacctacg gcaagctgac cctgaagttc 240

atctgcacca ccggcaagct gcccgtgccc tggcccaccc tcgtgaccac cctgacctgg 300

ggcgtgcagt gcttcagccg ctaccccgac cacatgaagc agcacgactt cttcaagtcc 360

```
gccatgcccg aaggctacgt ccaggagcgc accatcttct tcaaggacga cggcaactac    420
aagacccgcg ccgaggtgaa gttcgagggc gacaccctgg tgaaccgcat cgagctgaag    480
ggcatcgact tcaaggagga cggcaacatc ctggggcaca agctggagta caactacatc    540
agccacaacg tctatatcac cgccgacaag cagaagaacg gcatcaaggc caacttcaag    600
atccgccaca acatcgagga cggcagcgtg cagctcgccg accactacca gcagaacacc    660
cccatcggcg acggccccgt gctgctgccc gacaaccact acctgagcac ccagtccgcc    720
ctgagcaaag accccaacga gaagcgcgat cacatggtcc tgctggagtt cgtgaccgcc    780
gccgggatca ctctcggcat ggacgagctg tacaagccaa ataaaggaag tggaaccact    840
tcaggtacta cccgtcttct atctgggcac acgtgtttca cgttgacagg tttgcttggg    900
acgctagtaa ccatgggctt gctgactta                                      929
```

<210> 14
<211> 309
<212> PRT
<213> Artificial

<220>
<223> GPI(DAF)-CFP

<400> 14

```
Met Asn Thr Gln Ile Leu Val Phe Ala Leu Val Ala Val Ile Pro Thr
1               5                   10                  15

Asn Ala Asp Lys Ile Cys Leu Pro Arg Ala Arg Asp Pro Pro Val Ala
            20                  25                  30

Thr Met Val Ser Lys Gly Glu Glu Leu Phe Thr Gly Val Val Pro Ile
        35                  40                  45

Leu Val Glu Leu Asp Gly Asp Val Asn Gly His Lys Phe Ser Val Ser
    50                  55                  60

Gly Glu Gly Glu Gly Asp Ala Thr Tyr Gly Lys Leu Thr Leu Lys Phe
65                  70                  75                  80

Ile Cys Thr Thr Gly Lys Leu Pro Val Pro Trp Pro Thr Leu Val Thr
                85                  90                  95

Thr Leu Thr Trp Gly Val Gln Cys Phe Ser Arg Tyr Pro Asp His Met
            100                 105                 110

Lys Gln His Asp Phe Phe Lys Ser Ala Met Pro Glu Gly Tyr Val Gln
        115                 120                 125
```

Glu Arg Thr Ile Phe Phe Lys Asp Asp Gly Asn Tyr Lys Thr Arg Ala
    130                 135                 140

Glu Val Lys Phe Glu Gly Asp Thr Leu Val Asn Arg Ile Glu Leu Lys
145                 150                 155                 160

Gly Ile Asp Phe Lys Glu Asp Gly Asn Ile Leu Gly His Lys Leu Glu
                165                 170                 175

Tyr Asn Tyr Ile Ser His Asn Val Tyr Ile Thr Ala Asp Lys Gln Lys
            180                 185                 190

Asn Gly Ile Lys Ala Asn Phe Lys Ile Arg His Asn Ile Glu Asp Gly
        195                 200                 205

Ser Val Gln Leu Ala Asp His Tyr Gln Gln Asn Thr Pro Ile Gly Asp
    210                 215                 220

Gly Pro Val Leu Leu Pro Asp Asn His Tyr Leu Ser Thr Gln Ser Ala
225                 230                 235                 240

Leu Ser Lys Asp Pro Asn Glu Lys Arg Asp His Met Val Leu Leu Glu
                245                 250                 255

Phe Val Thr Ala Ala Gly Ile Thr Leu Gly Met Asp Glu Leu Tyr Lys
            260                 265                 270

Pro Asn Lys Gly Ser Gly Thr Thr Ser Gly Thr Thr Arg Leu Leu Ser
        275                 280                 285

Gly His Thr Cys Phe Thr Leu Thr Gly Leu Leu Gly Thr Leu Val Thr
    290                 295                 300

Met Gly Leu Leu Thr
305

<210> 15
<211> 929
<212> DNA
<213> Artificial

<220>
<223> GPI(DAF)-YFP

<400> 15
atgaacactc aaatcctggt tttcgccctt gtggcagtca ttcccacaaa tgcagacaaa 60

atttgtcttc cgcgggcccg ggatccaccg gtcgccacca tggtgagcaa gggcgaggag 120

ctgttcaccg gggtggtgcc catcctggtc gagctggacg gcgacgtaaa cggccacaag 180

```
ttcagcgtgt ccggcgaggg cgagggcgat gccacctacg gcaagctgac cctgaagttc    240
atctgcacca ccggcaagct gcccgtgccc tggcccaccc tcgtgaccac cttcggctac    300
ggcctgcagt gcttcgcccg ctaccccgac cacatgaagc agcacgactt cttcaagtcc    360
gccatgcccg aaggctacgt ccaggagcgc accatcttct tcaaggacga cggcaactac    420
aagacccgcg ccgaggtgaa gttcgagggc gacaccctgg tgaaccgcat cgagctgaag    480
ggcatcgact tcaaggagga cggcaacatc ctggggcaca agctggagta caactacaac    540
agccacaacg tctatatcat ggccgacaag cagaagaacg gcatcaaggt gaacttcaag    600
atccgccaca acatcgagga cggcagcgtg cagctcgccg accactacca gcagaacacc    660
cccatcggcg acggccccgt gctgctgccc gacaaccact acctgagcta ccagtccgcc    720
ctgagcaaag accccaacga gaagcgcgat cacatggtcc tgctggagtt cgtgaccgcc    780
gccgggatca ctctcggcat ggacgagctg tacaagccaa ataaaggaag tggaaccact    840
tcaggtacta cccgtcttct atctgggcac acgtgtttca cgttgacagg tttgcttggg    900
acgctagtaa ccatgggctt gctgactta                                       929
```

<210> 16
<211> 309
<212> PRT
<213> Artificial

<220>
<223> GPI(DAF)-YFP

<400> 16

```
Met Asn Thr Gln Ile Leu Val Phe Ala Leu Val Ala Val Ile Pro Thr
1               5                   10                  15

Asn Ala Asp Lys Ile Cys Leu Pro Arg Ala Arg Asp Pro Pro Val Ala
            20                  25                  30

Thr Met Val Ser Lys Gly Glu Glu Leu Phe Thr Gly Val Val Pro Ile
        35                  40                  45

Leu Val Glu Leu Asp Gly Asp Val Asn Gly His Lys Phe Ser Val Ser
    50                  55                  60

Gly Glu Gly Glu Gly Asp Ala Thr Tyr Gly Lys Leu Thr Leu Lys Phe
65                  70                  75                  80

Ile Cys Thr Thr Gly Lys Leu Pro Val Pro Trp Pro Thr Leu Val Thr
                85                  90                  95

Thr Phe Gly Tyr Gly Leu Gln Cys Phe Ala Arg Tyr Pro Asp His Met
```

```
            100                 105                 110

Lys Gln His Asp Phe Phe Lys Ser Ala Met Pro Glu Gly Tyr Val Gln
        115                 120                 125

Glu Arg Thr Ile Phe Phe Lys Asp Asp Gly Asn Tyr Lys Thr Arg Ala
    130                 135                 140

Glu Val Lys Phe Glu Gly Asp Thr Leu Val Asn Arg Ile Glu Leu Lys
145                 150                 155                 160

Gly Ile Asp Phe Lys Glu Asp Gly Asn Ile Leu Gly His Lys Leu Glu
                165                 170                 175

Tyr Asn Tyr Asn Ser His Asn Val Tyr Ile Met Ala Asp Lys Gln Lys
            180                 185                 190

Asn Gly Ile Lys Val Asn Phe Lys Ile Arg His Asn Ile Glu Asp Gly
        195                 200                 205

Ser Val Gln Leu Ala Asp His Tyr Gln Gln Asn Thr Pro Ile Gly Asp
    210                 215                 220

Gly Pro Val Leu Leu Pro Asp Asn His Tyr Leu Ser Tyr Gln Ser Ala
225                 230                 235                 240

Leu Ser Lys Asp Pro Asn Glu Lys Arg Asp His Met Val Leu Leu Glu
                245                 250                 255

Phe Val Thr Ala Ala Gly Ile Thr Leu Gly Met Asp Glu Leu Tyr Lys
            260                 265                 270

Pro Asn Lys Gly Ser Gly Thr Thr Ser Gly Thr Thr Arg Leu Leu Ser
        275                 280                 285

Gly His Thr Cys Phe Thr Leu Thr Gly Leu Leu Gly Thr Leu Val Thr
    290                 295                 300

Met Gly Leu Leu Thr
305

<210>  17
<211>  2454
<212>  DNA
<213>  Artificial

<220>
<223>  HA-mCer
```

<400> 17
atgaacactc aaatcctggt tttcgccctt gtggcagtca ttcccacaaa tgcagacaaa 60
atttgtcttg gacatcatgc tgtatcaaat ggcaccaaag taaacacact cactgagaga 120
ggagtagaag ttgtcaatgc aacggaaaca gtggagcgga caaacatccc caaaatttgc 180
tcaaaaggga aaagaaccac tgatcttggc caatgcggac tgttagggac cattaccgga 240
ccacctcaat gcgaccaatt tctagaattt tcagctgatc taataatcga gagacgagaa 300
ggaaatgatg tttgttaccc ggggaagttt gttaatgaag aggcattgcg acaaatcctc 360
agaggatcag gtgggattga caaagaaaca atgggattca catatagtgg aataaggacc 420
aacggaacaa ctagtgcatg tagaagatca gggtcttcat tctatgcaga aatggagtgg 480
ctcctgtcaa atacagacaa tgcttctttc ccacaaatga caaaatcata caaaaacaca 540
aggagagaat cagctctgat agtatgggga atccaccatt caggatcaac caccgaacag 600
accaaactat atgggagtgg aaataaactg ataacagtcg ggagttccaa atatcatcaa 660
tcttttgtgc cgagtccagg aacacgaccg cagataaatg gccagtccgg acggattgat 720
tttcattggt tgatcttgga tcccaatgat acagttactt ttagtttcaa tggggctttc 780
atagctccaa atcgtgccag cttcttgagg ggaaagtcca tggggatcca gagcgatgtg 840
caggttgatg ccaattgcga aggggaatgc taccacagtg gagggactat aacaagcaga 900
ttgccttttc aaaacatcaa tagcagagca gttggcaaat gcccaagata tgtaaaacag 960
gaaagtttat tattggcaac tgggatgaag aacgttcccg aaccttccaa aaaaaggaaa 1020
aaaagaggcc tgtttggcgc tatagcaggg tttattgaaa atggttggga aggtctggtc 1080
gacgggtggt acggtttcag gcatcagaat gcacaaggag aaggaactgc agcagactac 1140
aaaagcaccc aatcggcaat tgatcagata accggaaagt taaatagact cattgagaaa 1200
accaaccagc aatttgagct aatagataat gaattcactg aggtggaaaa gcagattggc 1260
aatttaatta actggaccaa agactccatc acagaagtat ggtcttacaa tgctgaactt 1320
attgtggcaa tggaaaacca gcacactatt gatttggctg attcagagat gaacaggctg 1380
tatgagcgag tgaggaaaca attaagggaa aatgctgaag aggatggtac tggttgcttt 1440
gaaatttttc ataaatgtga cgatgattgt atggctagta taaggaacaa tacttatgat 1500
cacagcaaat acagagaaga agcgatgcaa aatagaatac aaattgaccc agtcaaattg 1560
agtagtggct acaaagatgt gatactttgg tttagcttcg ggcatcatg ctttttgctt 1620
cttgccattg caatgggcct tgttttcata tgtgtgaaga acggaaacat gcggtgcact 1680
atttgtatac tccggcctga agctccgcgg gcccgggatc caccggtcgc caccatggtg 1740
agcaagggcg aggagctgtt caccggggtg gtgcccatcc tggtcgagct ggacggcgac 1800
gtaaacggcc acaagttcag cgtgtccggc gagggcgagg gcgatgccac ctacggcaag 1860

ctgaccctga agttcatctg caccaccggc aagctgcccg tgccctggcc caccctcgtg 1920

accaccctga cctggggcgt gcagtgcttc gcccgctacc ccgaccacat gaagcagcac 1980

gacttcttca agtccgccat gcccgaaggc tacgtccagg agcgcaccat cttcttcaag 2040

gacgacggca actacaagac ccgcgccgag gtgaagttcg agggcgacac cctggtgaac 2100

cgcatcgagc tgaagggcat cgacttcaag gaggacggca acatcctggg gcacaagctg 2160

gagtacaacg ccatcagcga caacgtctat atcaccgccg acaagcagaa gaacggcatc 2220

aaggccaact tcaagatccg ccacaacatc gaggacggca gcgtgcagct cgccgaccac 2280

taccagcaga acacccccat cggcgacggc cccgtgctgc tgcccgacaa ccactacctg 2340

agcacccagt ccaaactgag caaagacccc aacgagaagc gcgatcacat ggtcctgctg 2400

gagttcgtga ccgccgccgg gatcactctc ggcatggacg agctgtacaa gtaa 2454

<210> 18
<211> 817
<212> PRT
<213> Artificial

<220>
<223> HA-mCer

<400> 18

Met Asn Thr Gln Ile Leu Val Phe Ala Leu Val Ala Val Ile Pro Thr
1 5 10 15

Asn Ala Asp Lys Ile Cys Leu Gly His His Ala Val Ser Asn Gly Thr
20 25 30

Lys Val Asn Thr Leu Thr Glu Arg Gly Val Glu Val Val Asn Ala Thr
35 40 45

Glu Thr Val Glu Arg Thr Asn Ile Pro Lys Ile Cys Ser Lys Gly Lys
50 55 60

Arg Thr Thr Asp Leu Gly Gln Cys Gly Leu Leu Gly Thr Ile Thr Gly
65 70 75 80

Pro Pro Gln Cys Asp Gln Phe Leu Glu Phe Ser Ala Asp Leu Ile Ile
85 90 95

Glu Arg Arg Glu Gly Asn Asp Val Cys Tyr Pro Gly Lys Phe Val Asn
100 105 110

Glu Glu Ala Leu Arg Gln Ile Leu Arg Gly Ser Gly Gly Ile Asp Lys
115 120 125

```
Glu Thr Met Gly Phe Thr Tyr Ser Gly Ile Arg Thr Asn Gly Thr Thr
    130                 135                 140

Ser Ala Cys Arg Arg Ser Gly Ser Ser Phe Tyr Ala Glu Met Glu Trp
145                 150                 155                 160

Leu Leu Ser Asn Thr Asp Asn Ala Ser Phe Pro Gln Met Thr Lys Ser
                165                 170                 175

Tyr Lys Asn Thr Arg Arg Glu Ser Ala Leu Ile Val Trp Gly Ile His
            180                 185                 190

His Ser Gly Ser Thr Thr Glu Gln Thr Lys Leu Tyr Gly Ser Gly Asn
        195                 200                 205

Lys Leu Ile Thr Val Gly Ser Ser Lys Tyr His Gln Ser Phe Val Pro
    210                 215                 220

Ser Pro Gly Thr Arg Pro Gln Ile Asn Gly Gln Ser Gly Arg Ile Asp
225                 230                 235                 240

Phe His Trp Leu Ile Leu Asp Pro Asn Asp Thr Val Thr Phe Ser Phe
                245                 250                 255

Asn Gly Ala Phe Ile Ala Pro Asn Arg Ala Ser Phe Leu Arg Gly Lys
            260                 265                 270

Ser Met Gly Ile Gln Ser Asp Val Gln Val Asp Ala Asn Cys Glu Gly
        275                 280                 285

Glu Cys Tyr His Ser Gly Gly Thr Ile Thr Ser Arg Leu Pro Phe Gln
    290                 295                 300

Asn Ile Asn Ser Arg Ala Val Gly Lys Cys Pro Arg Tyr Val Lys Gln
305                 310                 315                 320

Glu Ser Leu Leu Leu Ala Thr Gly Met Lys Asn Val Pro Glu Pro Ser
                325                 330                 335

Lys Lys Arg Lys Lys Arg Gly Leu Phe Gly Ala Ile Ala Gly Phe Ile
            340                 345                 350

Glu Asn Gly Trp Glu Gly Leu Val Asp Gly Trp Tyr Gly Phe Arg His
        355                 360                 365

Gln Asn Ala Gln Gly Glu Gly Thr Ala Ala Asp Tyr Lys Ser Thr Gln
    370                 375                 380
```

```
Ser Ala Ile Asp Gln Ile Thr Gly Lys Leu Asn Arg Leu Ile Glu Lys
385                 390                 395                 400

Thr Asn Gln Gln Phe Glu Leu Ile Asp Asn Glu Phe Thr Glu Val Glu
                405                 410                 415

Lys Gln Ile Gly Asn Leu Ile Asn Trp Thr Lys Asp Ser Ile Thr Glu
            420                 425                 430

Val Trp Ser Tyr Asn Ala Glu Leu Ile Val Ala Met Glu Asn Gln His
        435                 440                 445

Thr Ile Asp Leu Ala Asp Ser Glu Met Asn Arg Leu Tyr Glu Arg Val
    450                 455                 460

Arg Lys Gln Leu Arg Glu Asn Ala Glu Glu Asp Gly Thr Gly Cys Phe
465                 470                 475                 480

Glu Ile Phe His Lys Cys Asp Asp Asp Cys Met Ala Ser Ile Arg Asn
                485                 490                 495

Asn Thr Tyr Asp His Ser Lys Tyr Arg Glu Glu Ala Met Gln Asn Arg
            500                 505                 510

Ile Gln Ile Asp Pro Val Lys Leu Ser Ser Gly Tyr Lys Asp Val Ile
        515                 520                 525

Leu Trp Phe Ser Phe Gly Ala Ser Cys Phe Leu Leu Leu Ala Ile Ala
    530                 535                 540

Met Gly Leu Val Phe Ile Cys Val Lys Asn Gly Asn Met Arg Cys Thr
545                 550                 555                 560

Ile Cys Ile Leu Arg Pro Glu Ala Pro Arg Ala Arg Asp Pro Pro Val
                565                 570                 575

Ala Thr Met Val Ser Lys Gly Glu Glu Leu Phe Thr Gly Val Val Pro
            580                 585                 590

Ile Leu Val Glu Leu Asp Gly Asp Val Asn Gly His Lys Phe Ser Val
        595                 600                 605

Ser Gly Glu Gly Glu Gly Asp Ala Thr Tyr Gly Lys Leu Thr Leu Lys
    610                 615                 620

Phe Ile Cys Thr Thr Gly Lys Leu Pro Val Pro Trp Pro Thr Leu Val
625                 630                 635                 640
```

```
Thr Thr Leu Thr Trp Gly Val Gln Cys Phe Ala Arg Tyr Pro Asp His
                645                 650                 655

Met Lys Gln His Asp Phe Phe Lys Ser Ala Met Pro Glu Gly Tyr Val
            660                 665                 670

Gln Glu Arg Thr Ile Phe Phe Lys Asp Asp Gly Asn Tyr Lys Thr Arg
        675                 680                 685

Ala Glu Val Lys Phe Glu Gly Asp Thr Leu Val Asn Arg Ile Glu Leu
    690                 695                 700

Lys Gly Ile Asp Phe Lys Glu Asp Gly Asn Ile Leu Gly His Lys Leu
705                 710                 715                 720

Glu Tyr Asn Ala Ile Ser Asp Asn Val Tyr Ile Thr Ala Asp Lys Gln
                725                 730                 735

Lys Asn Gly Ile Lys Ala Asn Phe Lys Ile Arg His Asn Ile Glu Asp
            740                 745                 750

Gly Ser Val Gln Leu Ala Asp His Tyr Gln Gln Asn Thr Pro Ile Gly
        755                 760                 765

Asp Gly Pro Val Leu Leu Pro Asp Asn His Tyr Leu Ser Thr Gln Ser
    770                 775                 780

Ala Leu Ser Lys Asp Pro Asn Glu Lys Arg Asp His Met Val Leu Leu
785                 790                 795                 800

Glu Phe Val Thr Ala Ala Gly Ile Thr Leu Gly Met Asp Glu Leu Tyr
                805                 810                 815

Lys
```

<210> 19
<211> 2454
<212> DNA
<213> Artificial

<220>
<223> HA-mYFP

<400> 19

```
atgaacactc aaatcctggt tttcgccctt gtggcagtca ttcccacaaa tgcagacaaa      60

atttgtcttg gacatcatgc tgtatcaaat ggcaccaaag taaacacact cactgagaga     120
```

ggagtagaag ttgtcaatgc aacggaaaca gtggagcgga caaacatccc caaaatttgc 180
tcaaaaggga aaagaaccac tgatcttggc caatgcggac tgttagggac cattaccgga 240
ccacctcaat gcgaccaatt tctagaattt tcagctgatc taataatcga gagacgagaa 300
ggaaatgatg tttgttaccc ggggaagttt gttaatgaag aggcattgcg acaaatcctc 360
agaggatcag gtgggattga caaagaaaca atgggattca catatagtgg aataaggacc 420
aacggaacaa ctagtgcatg tagaagatca gggtcttcat tctatgcaga aatggagtgg 480
ctcctgtcaa atacagacaa tgcttctttc ccacaaatga caaaatcata caaaaacaca 540
aggagagaat cagctctgat agtatgggga atccaccatt caggatcaac caccgaacag 600
accaaactat atgggagtgg aaataaactg ataacagtcg ggagttccaa atatcatcaa 660
tcttttgtgc cgagtccagg aacacgaccg cagataaatg gccagtccgg acggattgat 720
tttcattggt tgatcttgga tcccaatgat acagttactt ttagtttcaa tggggctttc 780
atagctccaa atcgtgccag cttcttgagg ggaaagtcca tggggatcca gagcgatgtg 840
caggttgatg ccaattgcga aggggaatgc taccacagtg gagggactat aacaagcaga 900
ttgccttttc aaaacatcaa tagcagagca gttggcaaat gcccaagata tgtaaaacag 960
gaaagtttat tattggcaac tgggatgaag aacgttcccg aaccttccaa aaaaaggaaa 1020
aaaagaggcc tgtttggcgc tatagcaggg tttattgaaa atggttggga aggtctggtc 1080
gacgggtggt acggtttcag gcatcagaat gcacaaggag aaggaactgc agcagactac 1140
aaaagcaccc aatcggcaat tgatcagata accggaaagt taaatagact cattgagaaa 1200
accaaccagc aatttgagct aatagataat gaattcactg aggtggaaaa gcagattggc 1260
aatttaatta actggaccaa agactccatc acagaagtat ggtcttacaa tgctgaactt 1320
attgtggcaa tggaaaacca gcacactatt gatttggctg attcagagat gaacaggctg 1380
tatgagcgag tgaggaaaca attaagggaa aatgctgaag aggatggtac tggttgcttt 1440
gaaatttttc ataaatgtga cgatgattgt atggctagta taaggaacaa tacttatgat 1500
cacagcaaat acagagaaga agcgatgcaa aatagaatac aaattgaccc agtcaaattg 1560
agtagtggct acaaagatgt gatactttgg tttagcttcg ggcatcatg ctttttgctt 1620
cttgccattg caatgggcct tgttttcata tgtgtgaaga acggaaacat gcggtgcact 1680
atttgtatac tccggcctga agctccgcgg gcccgggatc caccggtcgc caccatggtg 1740
agcaagggcg aggagctgtt caccggggtg gtgcccatcc tggtcgagct ggacggcgac 1800
gtaaacggcc acaagttcag cgtgtccggc gagggcgagg gcgatgccac ctacggcaag 1860
ctgaccctga agttcatctg caccaccggc aagctgcccg tgccctggcc caccctcgtg 1920
accaccttcg gctacggcct gcagtgcttc gcccgctacc ccgaccacat gaagcagcac 1980
gacttcttca agtccgccat gcccgaaggc tacgtccagg agcgcaccat cttcttcaag 2040

gacgacggca actacaagac ccgcgccgag gtgaagttcg agggcgacac cctggtgaac 2100

cgcatcgagc tgaagggcat cgacttcaag gaggacggca acatcctggg gcacaagctg 2160

gagtacaact acaacagcca caacgtctat atcatggccg acaagcagaa gaacggcatc 2220

aaggtgaact tcaagatccg ccacaacatc gaggacggca gcgtgcagct cgccgaccac 2280

taccagcaga acacccccat cggcgacggc cccgtgctgc tgcccgacaa ccactacctg 2340

agctaccagt ccaaactgag caaagacccc aacgagaagc gcgatcacat ggtcctgctg 2400

gagttcgtga ccgccgccgg gatcactctc ggcatggacg agctgtacaa gtaa 2454

<210> 20
<211> 817
<212> PRT
<213> Artificial

<220>
<223> HA-mYFP

<400> 20

Met Asn Thr Gln Ile Leu Val Phe Ala Leu Val Ala Val Ile Pro Thr
1 5 10 15

Asn Ala Asp Lys Ile Cys Leu Gly His His Ala Val Ser Asn Gly Thr
20 25 30

Lys Val Asn Thr Leu Thr Glu Arg Gly Val Glu Val Val Asn Ala Thr
35 40 45

Glu Thr Val Glu Arg Thr Asn Ile Pro Lys Ile Cys Ser Lys Gly Lys
50 55 60

Arg Thr Thr Asp Leu Gly Gln Cys Gly Leu Leu Gly Thr Ile Thr Gly
65 70 75 80

Pro Pro Gln Cys Asp Gln Phe Leu Glu Phe Ser Ala Asp Leu Ile Ile
85 90 95

Glu Arg Arg Glu Gly Asn Asp Val Cys Tyr Pro Gly Lys Phe Val Asn
100 105 110

Glu Glu Ala Leu Arg Gln Ile Leu Arg Gly Ser Gly Gly Ile Asp Lys
115 120 125

Glu Thr Met Gly Phe Thr Tyr Ser Gly Ile Arg Thr Asn Gly Thr Thr
130 135 140

Ser Ala Cys Arg Arg Ser Gly Ser Ser Phe Tyr Ala Glu Met Glu Trp

```
145                 150                 155                 160

Leu Leu Ser Asn Thr Asp Asn Ala Ser Phe Pro Gln Met Thr Lys Ser
                165                 170                 175

Tyr Lys Asn Thr Arg Arg Glu Ser Ala Leu Ile Val Trp Gly Ile His
            180                 185                 190

His Ser Gly Ser Thr Thr Glu Gln Thr Lys Leu Tyr Gly Ser Gly Asn
        195                 200                 205

Lys Leu Ile Thr Val Gly Ser Ser Lys Tyr His Gln Ser Phe Val Pro
    210                 215                 220

Ser Pro Gly Thr Arg Pro Gln Ile Asn Gly Gln Ser Gly Arg Ile Asp
225                 230                 235                 240

Phe His Trp Leu Ile Leu Asp Pro Asn Asp Thr Val Thr Phe Ser Phe
                245                 250                 255

Asn Gly Ala Phe Ile Ala Pro Asn Arg Ala Ser Phe Leu Arg Gly Lys
            260                 265                 270

Ser Met Gly Ile Gln Ser Asp Val Gln Val Asp Ala Asn Cys Glu Gly
        275                 280                 285

Glu Cys Tyr His Ser Gly Gly Thr Ile Thr Ser Arg Leu Pro Phe Gln
    290                 295                 300

Asn Ile Asn Ser Arg Ala Val Gly Lys Cys Pro Arg Tyr Val Lys Gln
305                 310                 315                 320

Glu Ser Leu Leu Leu Ala Thr Gly Met Lys Asn Val Pro Glu Pro Ser
                325                 330                 335

Lys Lys Arg Lys Lys Arg Gly Leu Phe Gly Ala Ile Ala Gly Phe Ile
            340                 345                 350

Glu Asn Gly Trp Glu Gly Leu Val Asp Gly Trp Tyr Gly Phe Arg His
        355                 360                 365

Gln Asn Ala Gln Gly Glu Gly Thr Ala Ala Asp Tyr Lys Ser Thr Gln
    370                 375                 380

Ser Ala Ile Asp Gln Ile Thr Gly Lys Leu Asn Arg Leu Ile Glu Lys
385                 390                 395                 400
```

```
Thr Asn Gln Gln Phe Glu Leu Ile Asp Asn Glu Phe Thr Glu Val Glu
                405                 410                 415

Lys Gln Ile Gly Asn Leu Ile Asn Trp Thr Lys Asp Ser Ile Thr Glu
            420                 425                 430

Val Trp Ser Tyr Asn Ala Glu Leu Ile Val Ala Met Glu Asn Gln His
        435                 440                 445

Thr Ile Asp Leu Ala Asp Ser Glu Met Asn Arg Leu Tyr Glu Arg Val
    450                 455                 460

Arg Lys Gln Leu Arg Glu Asn Ala Glu Glu Asp Gly Thr Gly Cys Phe
465                 470                 475                 480

Glu Ile Phe His Lys Cys Asp Asp Asp Cys Met Ala Ser Ile Arg Asn
                485                 490                 495

Asn Thr Tyr Asp His Ser Lys Tyr Arg Glu Glu Ala Met Gln Asn Arg
            500                 505                 510

Ile Gln Ile Asp Pro Val Lys Leu Ser Ser Gly Tyr Lys Asp Val Ile
        515                 520                 525

Leu Trp Phe Ser Phe Gly Ala Ser Cys Phe Leu Leu Leu Ala Ile Ala
    530                 535                 540

Met Gly Leu Val Phe Ile Cys Val Lys Asn Gly Asn Met Arg Cys Thr
545                 550                 555                 560

Ile Cys Ile Leu Arg Pro Glu Ala Pro Arg Ala Arg Asp Pro Pro Val
                565                 570                 575

Ala Thr Met Val Ser Lys Gly Glu Glu Leu Phe Thr Gly Val Val Pro
            580                 585                 590

Ile Leu Val Glu Leu Asp Gly Asp Val Asn Gly His Lys Phe Ser Val
        595                 600                 605

Ser Gly Glu Gly Glu Gly Asp Ala Thr Tyr Gly Lys Leu Thr Leu Lys
    610                 615                 620

Phe Ile Cys Thr Thr Gly Lys Leu Pro Val Pro Trp Pro Thr Leu Val
625                 630                 635                 640

Thr Thr Phe Gly Tyr Gly Leu Gln Cys Phe Ala Arg Tyr Pro Asp His
                645                 650                 655
```

Met Lys Gln His Asp Phe Phe Lys Ser Ala Met Pro Glu Gly Tyr Val
660 665 670

Gln Glu Arg Thr Ile Phe Phe Lys Asp Asp Gly Asn Tyr Lys Thr Arg
675 680 685

Ala Glu Val Lys Phe Glu Gly Asp Thr Leu Val Asn Arg Ile Glu Leu
690 695 700

Lys Gly Ile Asp Phe Lys Glu Asp Gly Asn Ile Leu Gly His Lys Leu
705 710 715 720

Glu Tyr Asn Tyr Asn Ser His Asn Val Tyr Ile Met Ala Asp Lys Gln
725 730 735

Lys Asn Gly Ile Lys Val Asn Phe Lys Ile Arg His Asn Ile Glu Asp
740 745 750

Gly Ser Val Gln Leu Ala Asp His Tyr Gln Gln Asn Thr Pro Ile Gly
755 760 765

Asp Gly Pro Val Leu Leu Pro Asp Asn His Tyr Leu Ser Tyr Gln Ser
770 775 780

Ala Leu Ser Lys Asp Pro Asn Glu Lys Arg Asp His Met Val Leu Leu
785 790 795 800

Glu Phe Val Thr Ala Ala Gly Ile Thr Leu Gly Met Asp Glu Leu Tyr
805 810 815

Lys

<210> 21
<211> 2454
<212> DNA
<213> Artificial

<220>
<223> M3-HA-mCer

<400> 21
atgaacactc aaatcctggt tttcgccctt gtggcagtca ttcccacaaa tgcagacaaa 60

atttgtcttg gacatcatgc tgtatcaaat ggcaccaaag taaacacact cactgagaga 120

ggagtagaag ttgtcaatgc aacggaaaca gtggagcgga caaacatccc caaaatttgc 180

tcaaaaggga aaagaaccac tgatcttggc caatgcggac tgttagggac cattaccgga 240

ccacctcaat gcgaccaatt tctagaattt tcagctgatc taataatcga gagacgagaa 300

```
ggaaatgatg tttgttaccc ggggaagttt gttaatgaag aggcattgcg acaaatcctc     360
agaggatcag gtgggattga caaagaaaca atgggattca catatagtgg aataaggacc     420
aacggaacaa ctagtgcatg tagaagatca gggtcttcat tctatgcaga aatggagtgg     480
ctcctgtcaa atacagacaa tgcttctttc ccacaaatga caaaatcata caaaaacaca     540
aggagagaat cagctctgat agtatgggga atccaccatt caggatcaac caccgaacag     600
accaaactat atgggagtgg aaataaactg ataacagtcg ggagttccaa atatcatcaa     660
tcttttgtgc cgagtccagg aacacgaccg cagataaatg gccagtccgg acggattgat     720
tttcattggt tgatcttgga tcccaatgat acagttactt ttagtttcaa tggggctttc     780
atagctccaa atcgtgccag cttcttgagg ggaaagtcca tggggatcca gagcgatgtg     840
caggttgatg ccaattgcga aggggaatgc taccacagtg gagggactat aacaagcaga     900
ttgccttttc aaaacatcaa tagcagagca gttggcaaat gcccaagata tgtaaaacag     960
gaaagtttat tattggcaac tgggatgaag aacgttcccg aaccttccaa aaaaaggaaa    1020
aaaagaggcc tgtttggcgc tatagcaggg tttattgaaa atggttggga aggtctggtc    1080
gacgggtggt acggtttcag gcatcagaat gcacaaggag aaggaactgc agcagactac    1140
aaaagcaccc aatcggcaat tgatcagata accggaaagt taaatagact cattgagaaa    1200
accaaccagc aatttgagct aatagataat gaattcactg aggtggaaaa gcagattggc    1260
aatttaatta actggaccaa agactccatc acagaagtat ggtcttacaa tgctgaactt    1320
attgtggcaa tggaaaacca gcacactatt gatttggctg attcagagat gaacaggctg    1380
tatgagcgag tgaggaaaca attaagggaa aatgctgaag aggatggtac tggttgcttt    1440
gaaatttttc ataaatgtga cgatgattgt atggctagta taaggaacaa tacttatgat    1500
cacagcaaat acagagaaga agcgatgcaa aatagaatac aaattgaccc agtcaaattg    1560
agtagtggct acaaagatgt gatactttgg tttagcttcg ggcatcatgc ctttttgctt    1620
cttgccattg caatgggcct tgttttcata tctgtgaaga acggaaacat gcggtccact    1680
atttctatac tccggcctga agctccgcgg gcccgggatc caccggtcgc caccatggtg    1740
agcaagggcg aggagctgtt caccggggtg gtgcccatcc tggtcgagct ggacggcgac    1800
gtaaacggcc acaagttcag cgtgtccggc gagggcgagg gcgatgccac ctacggcaag    1860
ctgaccctga agttcatctg caccaccggc aagctgcccg tgccctggcc caccctcgtg    1920
accaccctga cctggggcgt gcagtgcttc gcccgctacc ccgaccacat gaagcagcac    1980
gacttcttca agtccgccat gcccgaaggc tacgtccagg agcgcaccat cttcttcaag    2040
gacgacggca actacaagac ccgcgccgag gtgaagttcg agggcgacac cctggtgaac    2100
cgcatcgagc tgaagggcat cgacttcaag gaggacggca acatcctggg gcacaagctg    2160
```

gagtacaacg ccatcagcga caacgtctat atcaccgccg acaagcagaa gaacggcatc 2220

aaggccaact tcaagatccg ccacaacatc gaggacggca gcgtgcagct cgccgaccac 2280

taccagcaga acacccccat cggcgacggc cccgtgctgc tgcccgacaa ccactacctg 2340

agcacccagt ccaaactgag caaagacccc aacgagaagc gcgatcacat ggtcctgctg 2400

gagttcgtga ccgccgccgg gatcactctc ggcatggacg agctgtacaa gtaa 2454

<210> 22
<211> 817
<212> PRT
<213> Artificial

<220>
<223> M3-HA-mCer

<400> 22

Met Asn Thr Gln Ile Leu Val Phe Ala Leu Val Ala Val Ile Pro Thr
1 5 10 15

Asn Ala Asp Lys Ile Cys Leu Gly His His Ala Val Ser Asn Gly Thr
20 25 30

Lys Val Asn Thr Leu Thr Glu Arg Gly Val Glu Val Val Asn Ala Thr
35 40 45

Glu Thr Val Glu Arg Thr Asn Ile Pro Lys Ile Cys Ser Lys Gly Lys
50 55 60

Arg Thr Thr Asp Leu Gly Gln Cys Gly Leu Leu Gly Thr Ile Thr Gly
65 70 75 80

Pro Pro Gln Cys Asp Gln Phe Leu Glu Phe Ser Ala Asp Leu Ile Ile
85 90 95

Glu Arg Arg Glu Gly Asn Asp Val Cys Tyr Pro Gly Lys Phe Val Asn
100 105 110

Glu Glu Ala Leu Arg Gln Ile Leu Arg Gly Ser Gly Gly Ile Asp Lys
115 120 125

Glu Thr Met Gly Phe Thr Tyr Ser Gly Ile Arg Thr Asn Gly Thr Thr
130 135 140

Ser Ala Cys Arg Arg Ser Gly Ser Ser Phe Tyr Ala Glu Met Glu Trp
145 150 155 160

Leu Leu Ser Asn Thr Asp Asn Ala Ser Phe Pro Gln Met Thr Lys Ser
165 170 175

```
Tyr Lys Asn Thr Arg Arg Glu Ser Ala Leu Ile Val Trp Gly Ile His
            180                 185                 190

His Ser Gly Ser Thr Thr Glu Gln Thr Lys Leu Tyr Gly Ser Gly Asn
        195                 200                 205

Lys Leu Ile Thr Val Gly Ser Ser Lys Tyr His Gln Ser Phe Val Pro
    210                 215                 220

Ser Pro Gly Thr Arg Pro Gln Ile Asn Gly Gln Ser Gly Arg Ile Asp
225                 230                 235                 240

Phe His Trp Leu Ile Leu Asp Pro Asn Asp Thr Val Thr Phe Ser Phe
                245                 250                 255

Asn Gly Ala Phe Ile Ala Pro Asn Arg Ala Ser Phe Leu Arg Gly Lys
            260                 265                 270

Ser Met Gly Ile Gln Ser Asp Val Gln Val Asp Ala Asn Cys Glu Gly
        275                 280                 285

Glu Cys Tyr His Ser Gly Gly Thr Ile Thr Ser Arg Leu Pro Phe Gln
    290                 295                 300

Asn Ile Asn Ser Arg Ala Val Gly Lys Cys Pro Arg Tyr Val Lys Gln
305                 310                 315                 320

Glu Ser Leu Leu Leu Ala Thr Gly Met Lys Asn Val Pro Glu Pro Ser
                325                 330                 335

Lys Lys Arg Lys Lys Arg Gly Leu Phe Gly Ala Ile Ala Gly Phe Ile
            340                 345                 350

Glu Asn Gly Trp Glu Gly Leu Val Asp Gly Trp Tyr Gly Phe Arg His
        355                 360                 365

Gln Asn Ala Gln Gly Glu Gly Thr Ala Ala Asp Tyr Lys Ser Thr Gln
    370                 375                 380

Ser Ala Ile Asp Gln Ile Thr Gly Lys Leu Asn Arg Leu Ile Glu Lys
385                 390                 395                 400

Thr Asn Gln Gln Phe Glu Leu Ile Asp Asn Glu Phe Thr Glu Val Glu
                405                 410                 415

Lys Gln Ile Gly Asn Leu Ile Asn Trp Thr Lys Asp Ser Ile Thr Glu
```

420 425 430

Val Trp Ser Tyr Asn Ala Glu Leu Ile Val Ala Met Glu Asn Gln His
435 440 445

Thr Ile Asp Leu Ala Asp Ser Glu Met Asn Arg Leu Tyr Glu Arg Val
450 455 460

Arg Lys Gln Leu Arg Glu Asn Ala Glu Glu Asp Gly Thr Gly Cys Phe
465 470 475 480

Glu Ile Phe His Lys Cys Asp Asp Asp Cys Met Ala Ser Ile Arg Asn
485 490 495

Asn Thr Tyr Asp His Ser Lys Tyr Arg Glu Glu Ala Met Gln Asn Arg
500 505 510

Ile Gln Ile Asp Pro Val Lys Leu Ser Ser Gly Tyr Lys Asp Val Ile
515 520 525

Leu Trp Phe Ser Phe Gly Ala Ser Cys Phe Leu Leu Leu Ala Ile Ala
530 535 540

Met Gly Leu Val Phe Ile Ser Val Lys Asn Gly Asn Met Arg Ser Thr
545 550 555 560

Ile Ser Ile Leu Arg Pro Glu Ala Pro Arg Ala Arg Asp Pro Pro Val
565 570 575

Ala Thr Met Val Ser Lys Gly Glu Glu Leu Phe Thr Gly Val Val Pro
580 585 590

Ile Leu Val Glu Leu Asp Gly Asp Val Asn Gly His Lys Phe Ser Val
595 600 605

Ser Gly Glu Gly Glu Gly Asp Ala Thr Tyr Gly Lys Leu Thr Leu Lys
610 615 620

Phe Ile Cys Thr Thr Gly Lys Leu Pro Val Pro Trp Pro Thr Leu Val
625 630 635 640

Thr Thr Leu Thr Trp Gly Val Gln Cys Phe Ala Arg Tyr Pro Asp His
645 650 655

Met Lys Gln His Asp Phe Phe Lys Ser Ala Met Pro Glu Gly Tyr Val
660 665 670

```
Gln Glu Arg Thr Ile Phe Phe Lys Asp Asp Gly Asn Tyr Lys Thr Arg
      675                 680                 685

Ala Glu Val Lys Phe Glu Gly Asp Thr Leu Val Asn Arg Ile Glu Leu
    690                 695                 700

Lys Gly Ile Asp Phe Lys Glu Asp Gly Asn Ile Leu Gly His Lys Leu
705                 710                 715                 720

Glu Tyr Asn Ala Ile Ser Asp Asn Val Tyr Ile Thr Ala Asp Lys Gln
                725                 730                 735

Lys Asn Gly Ile Lys Ala Asn Phe Lys Ile Arg His Asn Ile Glu Asp
            740                 745                 750

Gly Ser Val Gln Leu Ala Asp His Tyr Gln Gln Asn Thr Pro Ile Gly
        755                 760                 765

Asp Gly Pro Val Leu Leu Pro Asp Asn His Tyr Leu Ser Thr Gln Ser
    770                 775                 780

Ala Leu Ser Lys Asp Pro Asn Glu Lys Arg Asp His Met Val Leu Leu
785                 790                 795                 800

Glu Phe Val Thr Ala Ala Gly Ile Thr Leu Gly Met Asp Glu Leu Tyr
                805                 810                 815

Lys


<210>  23
<211>  2454
<212>  DNA
<213>  Artificial

<220>
<223>  M3-HA-mYFP

<400>  23
atgaacactc aaatcctggt tttcgccctt gtggcagtca ttcccacaaa tgcagacaaa      60

atttgtcttg gacatcatgc tgtatcaaat ggcaccaaag taaacacact cactgagaga     120

ggagtagaag ttgtcaatgc aacggaaaca gtggagcgga caaacatccc caaaatttgc     180

tcaaaaggga aaagaaccac tgatcttggc caatgcggac tgttagggac cattaccgga     240

ccacctcaat gcgaccaatt tctagaattt tcagctgatc taataatcga gagacgagaa     300

ggaaatgatg tttgttaccc ggggaagttt gttaatgaag aggcattgcg acaaatcctc     360

agaggatcag gtgggattga caaagaaaca atgggattca catatagtgg aataaggacc     420
```

```
aacggaacaa ctagtgcatg tagaagatca gggtcttcat tctatgcaga aatggagtgg     480
ctcctgtcaa atacagacaa tgcttctttc ccacaaatga caaaatcata caaaaacaca     540
aggagagaat cagctctgat agtatgggga atccaccatt caggatcaac caccgaacag     600
accaaactat atgggagtgg aaataaactg ataacagtcg ggagttccaa atatcatcaa     660
tcttttgtgc cgagtccagg aacacgaccg cagataaatg gccagtccgg acggattgat     720
tttcattggt tgatcttgga tcccaatgat acagttactt ttagtttcaa tggggctttc     780
atagctccaa atcgtgccag cttcttgagg ggaaagtcca tggggatcca gagcgatgtg     840
caggttgatg ccaattgcga aggggaatgc taccacagtg gagggactat aacaagcaga     900
ttgccttttc aaaacatcaa tagcagagca gttggcaaat gcccaagata tgtaaaacag     960
gaaagtttat tattggcaac tgggatgaag aacgttcccg aaccttccaa aaaaaggaaa    1020
aaaagaggcc tgtttggcgc tatagcaggg tttattgaaa atggttggga aggtctggtc    1080
gacgggtggt acggtttcag gcatcagaat gcacaaggag aaggaactgc agcagactac    1140
aaaagcaccc aatcggcaat tgatcagata accggaaagt taaatagact cattgagaaa    1200
accaaccagc aatttgagct aatagataat gaattcactg aggtggaaaa gcagattggc    1260
aatttaatta actggaccaa agactccatc acagaagtat ggtcttacaa tgctgaactt    1320
attgtggcaa tggaaaacca gcacactatt gatttggctg attcagagat gaacaggctg    1380
tatgagcgag tgaggaaaca attaagggaa aatgctgaag aggatggtac tggttgcttt    1440
gaaatttttc ataaatgtga cgatgattgt atggctagta taaggaacaa tacttatgat    1500
cacagcaaat acagagaaga agcgatgcaa aatagaatac aaattgaccc agtcaaattg    1560
agtagtggct acaaagatgt gatactttgg tttagcttcg ggcatcatg ctttttgctt     1620
cttgccattg caatgggcct tgttttcata tctgtgaaga acggaaacat gcggtccact    1680
atttctatac tccggcctga agctccgcgg gcccgggatc caccggtcgc caccatggtg    1740
agcaagggcg aggagctgtt caccggggtg gtgcccatcc tggtcgagct ggacggcgac    1800
gtaaacggcc acaagttcag cgtgtccggc gagggcgagg gcgatgccac ctacggcaag    1860
ctgaccctga agttcatctg caccaccggc aagctgcccg tgccctggcc caccctcgtg    1920
accaccttcg gctacggcct gcagtgcttc gcccgctacc ccgaccacat gaagcagcac    1980
gacttcttca agtccgccat gcccgaaggc tacgtccagg agcgcaccat cttcttcaag    2040
gacgacggca actacaagac ccgcgccgag gtgaagttcg agggcgacac cctggtgaac    2100
cgcatcgagc tgaagggcat cgacttcaag gaggacggca acatcctggg gcacaagctg    2160
gagtacaact acaacagcca caacgtctat atcatggccg acaagcagaa gaacggcatc    2220
aaggtgaact tcaagatccg ccacaacatc gaggacggca gcgtgcagct cgccgaccac    2280
taccagcaga acacccccat cggcgacggc cccgtgctgc tgcccgacaa ccactacctg    2340
```

agctaccagt ccaaactgag caaagacccc aacgagaagc gcgatcacat ggtcctgctg 2400

gagttcgtga ccgccgccgg gatcactctc ggcatggacg agctgtacaa gtaa 2454

<210> 24
<211> 817
<212> PRT
<213> Artificial

<220>
<223> M3-HA-mYFP

<400> 24

Met Asn Thr Gln Ile Leu Val Phe Ala Leu Val Ala Val Ile Pro Thr
1 5 10 15

Asn Ala Asp Lys Ile Cys Leu Gly His His Ala Val Ser Asn Gly Thr
20 25 30

Lys Val Asn Thr Leu Thr Glu Arg Gly Val Glu Val Val Asn Ala Thr
35 40 45

Glu Thr Val Glu Arg Thr Asn Ile Pro Lys Ile Cys Ser Lys Gly Lys
50 55 60

Arg Thr Thr Asp Leu Gly Gln Cys Gly Leu Leu Gly Thr Ile Thr Gly
65 70 75 80

Pro Pro Gln Cys Asp Gln Phe Leu Glu Phe Ser Ala Asp Leu Ile Ile
85 90 95

Glu Arg Arg Glu Gly Asn Asp Val Cys Tyr Pro Gly Lys Phe Val Asn
100 105 110

Glu Glu Ala Leu Arg Gln Ile Leu Arg Gly Ser Gly Gly Ile Asp Lys
115 120 125

Glu Thr Met Gly Phe Thr Tyr Ser Gly Ile Arg Thr Asn Gly Thr Thr
130 135 140

Ser Ala Cys Arg Arg Ser Gly Ser Ser Phe Tyr Ala Glu Met Glu Trp
145 150 155 160

Leu Leu Ser Asn Thr Asp Asn Ala Ser Phe Pro Gln Met Thr Lys Ser
165 170 175

Tyr Lys Asn Thr Arg Arg Glu Ser Ala Leu Ile Val Trp Gly Ile His
180 185 190

```
His Ser Gly Ser Thr Thr Glu Gln Thr Lys Leu Tyr Gly Ser Gly Asn
        195                 200                 205

Lys Leu Ile Thr Val Gly Ser Ser Lys Tyr His Gln Ser Phe Val Pro
    210                 215                 220

Ser Pro Gly Thr Arg Pro Gln Ile Asn Gly Gln Ser Gly Arg Ile Asp
225                 230                 235                 240

Phe His Trp Leu Ile Leu Asp Pro Asn Asp Thr Val Thr Phe Ser Phe
                245                 250                 255

Asn Gly Ala Phe Ile Ala Pro Asn Arg Ala Ser Phe Leu Arg Gly Lys
            260                 265                 270

Ser Met Gly Ile Gln Ser Asp Val Gln Val Asp Ala Asn Cys Glu Gly
        275                 280                 285

Glu Cys Tyr His Ser Gly Gly Thr Ile Thr Ser Arg Leu Pro Phe Gln
    290                 295                 300

Asn Ile Asn Ser Arg Ala Val Gly Lys Cys Pro Arg Tyr Val Lys Gln
305                 310                 315                 320

Glu Ser Leu Leu Leu Ala Thr Gly Met Lys Asn Val Pro Glu Pro Ser
                325                 330                 335

Lys Lys Arg Lys Lys Arg Gly Leu Phe Gly Ala Ile Ala Gly Phe Ile
            340                 345                 350

Glu Asn Gly Trp Glu Gly Leu Val Asp Gly Trp Tyr Gly Phe Arg His
        355                 360                 365

Gln Asn Ala Gln Gly Glu Gly Thr Ala Ala Asp Tyr Lys Ser Thr Gln
    370                 375                 380

Ser Ala Ile Asp Gln Ile Thr Gly Lys Leu Asn Arg Leu Ile Glu Lys
385                 390                 395                 400

Thr Asn Gln Gln Phe Glu Leu Ile Asp Asn Glu Phe Thr Glu Val Glu
                405                 410                 415

Lys Gln Ile Gly Asn Leu Ile Asn Trp Thr Lys Asp Ser Ile Thr Glu
            420                 425                 430

Val Trp Ser Tyr Asn Ala Glu Leu Ile Val Ala Met Glu Asn Gln His
        435                 440                 445
```

```
Thr Ile Asp Leu Ala Asp Ser Glu Met Asn Arg Leu Tyr Glu Arg Val
    450                 455                 460

Arg Lys Gln Leu Arg Glu Asn Ala Glu Glu Asp Gly Thr Gly Cys Phe
465                 470                 475                 480

Glu Ile Phe His Lys Cys Asp Asp Asp Cys Met Ala Ser Ile Arg Asn
                485                 490                 495

Asn Thr Tyr Asp His Ser Lys Tyr Arg Glu Glu Ala Met Gln Asn Arg
            500                 505                 510

Ile Gln Ile Asp Pro Val Lys Leu Ser Ser Gly Tyr Lys Asp Val Ile
        515                 520                 525

Leu Trp Phe Ser Phe Gly Ala Ser Cys Phe Leu Leu Leu Ala Ile Ala
    530                 535                 540

Met Gly Leu Val Phe Ile Ser Val Lys Asn Gly Asn Met Arg Ser Thr
545                 550                 555                 560

Ile Ser Ile Leu Arg Pro Glu Ala Pro Arg Ala Arg Asp Pro Pro Val
                565                 570                 575

Ala Thr Met Val Ser Lys Gly Glu Glu Leu Phe Thr Gly Val Val Pro
            580                 585                 590

Ile Leu Val Glu Leu Asp Gly Asp Val Asn Gly His Lys Phe Ser Val
        595                 600                 605

Ser Gly Glu Gly Glu Gly Asp Ala Thr Tyr Gly Lys Leu Thr Leu Lys
    610                 615                 620

Phe Ile Cys Thr Thr Gly Lys Leu Pro Val Pro Trp Pro Thr Leu Val
625                 630                 635                 640

Thr Thr Phe Gly Tyr Gly Leu Gln Cys Phe Ala Arg Tyr Pro Asp His
                645                 650                 655

Met Lys Gln His Asp Phe Phe Lys Ser Ala Met Pro Glu Gly Tyr Val
            660                 665                 670

Gln Glu Arg Thr Ile Phe Phe Lys Asp Asp Gly Asn Tyr Lys Thr Arg
        675                 680                 685

Ala Glu Val Lys Phe Glu Gly Asp Thr Leu Val Asn Arg Ile Glu Leu
```

690 695 700

Lys Gly Ile Asp Phe Lys Glu Asp Gly Asn Ile Leu Gly His Lys Leu
705 710 715 720

Glu Tyr Asn Tyr Asn Ser His Asn Val Tyr Ile Met Ala Asp Lys Gln
725 730 735

Lys Asn Gly Ile Lys Val Asn Phe Lys Ile Arg His Asn Ile Glu Asp
740 745 750

Gly Ser Val Gln Leu Ala Asp His Tyr Gln Gln Asn Thr Pro Ile Gly
755 760 765

Asp Gly Pro Val Leu Leu Pro Asp Asn His Tyr Leu Ser Tyr Gln Ser
770 775 780

Ala Leu Ser Lys Asp Pro Asn Glu Lys Arg Asp His Met Val Leu Leu
785 790 795 800

Glu Phe Val Thr Ala Ala Gly Ile Thr Leu Gly Met Asp Glu Leu Tyr
805 810 815

Lys

<210> 25
<211> 780
<212> DNA
<213> Artificial

<220>
<223> Lyn-mCer

<400> 25
atgggatgta ttaaatcaaa aaggaaagac aatctcaatg acgatgaacc ggtcgccacc 60

atggtgagca agggcgagga gctgttcacc ggggtggtgc ccatcctggt cgagctggac 120

ggcgacgtaa acggccacaa gttcagcgtg tccggcgagg gcgagggcga tgccacctac 180

ggcaagctga ccctgaagtt catctgcacc accggcaagc tgcccgtgcc ctggcccacc 240

ctcgtgacca ccctgacctg gggcgtgcag tgcttcgccc gctaccccga ccacatgaag 300

cagcacgact tcttcaagtc cgccatgccc gaaggctacg tccaggagcg caccatcttc 360

ttcaaggacg acggcaacta caagacccgc gccgaggtga agttcgaggg cgacaccctg 420

gtgaaccgca tcgagctgaa gggcatcgac ttcaaggagg acggcaacat cctggggcac 480

aagctggagt acaacgccat cagcgacaac gtctatatca ccgccgacaa gcagaagaac 540

ggcatcaagg ccaacttcaa gatccgccac aacatcgagg acggcagcgt gcagctcgcc 600

gaccactacc agcagaacac ccccatcggc gacggccccg tgctgctgcc cgacaaccac 660

tacctgagca cccagtccaa actgagcaaa gaccccaacg agaagcgcga tcacatggtc 720

ctgctggagt tcgtgaccgc cgccgggatc actctcggca tggacgagct gtacaagtaa 780

<210> 26
<211> 259
<212> PRT
<213> Artificial

<220>
<223> Lyn-mCer

<400> 26

Met Gly Cys Ile Lys Ser Lys Arg Lys Asp Asn Leu Asn Asp Asp Glu
1 5 10 15

Pro Val Ala Thr Met Val Ser Lys Gly Glu Glu Leu Phe Thr Gly Val
20 25 30

Val Pro Ile Leu Val Glu Leu Asp Gly Asp Val Asn Gly His Lys Phe
35 40 45

Ser Val Ser Gly Glu Gly Glu Gly Asp Ala Thr Tyr Gly Lys Leu Thr
50 55 60

Leu Lys Phe Ile Cys Thr Thr Gly Lys Leu Pro Val Pro Trp Pro Thr
65 70 75 80

Leu Val Thr Thr Leu Thr Trp Gly Val Gln Cys Phe Ala Arg Tyr Pro
85 90 95

Asp His Met Lys Gln His Asp Phe Phe Lys Ser Ala Met Pro Glu Gly
100 105 110

Tyr Val Gln Glu Arg Thr Ile Phe Phe Lys Asp Asp Gly Asn Tyr Lys
115 120 125

Thr Arg Ala Glu Val Lys Phe Glu Gly Asp Thr Leu Val Asn Arg Ile
130 135 140

Glu Leu Lys Gly Ile Asp Phe Lys Glu Asp Gly Asn Ile Leu Gly His
145 150 155 160

Lys Leu Glu Tyr Asn Ala Ile Ser Asp Asn Val Tyr Ile Thr Ala Asp
165 170 175

Lys Gln Lys Asn Gly Ile Lys Ala Asn Phe Lys Ile Arg His Asn Ile

180 185 190

Glu Asp Gly Ser Val Gln Leu Ala Asp His Tyr Gln Gln Asn Thr Pro
195 200 205

Ile Gly Asp Gly Pro Val Leu Leu Pro Asp Asn His Tyr Leu Ser Thr
210 215 220

Gln Ser Ala Leu Ser Lys Asp Pro Asn Glu Lys Arg Asp His Met Val
225 230 235 240

Leu Leu Glu Phe Val Thr Ala Ala Gly Ile Thr Leu Gly Met Asp Glu
245 250 255

Leu Tyr Lys

<210> 27
<211> 780
<212> DNA
<213> Artificial

<220>
<223> Lyn-mYFP

<400> 27
atgggatgta ttaaatcaaa aaggaaagac aatctcaatg acgatgaacc ggtcgccacc 60
atggtgagca agggcgagga gctgttcacc ggggtggtgc ccatcctggt cgagctggac 120
ggcgacgtaa acggccacaa gttcagcgtg tccggcgagg gcgagggcga tgccacctac 180
ggcaagctga ccctgaagtt catctgcacc accggcaagc tgcccgtgcc ctggcccacc 240
ctcgtgacca ccttcggcta cggcctgcag tgcttcgccc gctaccccga ccacatgaag 300
cagcacgact tcttcaagtc cgccatgccc gaaggctacg tccaggagcg caccatcttc 360
ttcaaggacg acggcaacta caagacccgc gccgaggtga agttcgaggg cgacaccctg 420
gtgaaccgca tcgagctgaa gggcatcgac ttcaaggagg acggcaacat cctggggcac 480
aagctggagt acaactacaa cagccacaac gtctatatca tggccgacaa gcagaagaac 540
ggcatcaagg tgaacttcaa gatccgccac aacatcgagg acggcagcgt gcagctcgcc 600
gaccactacc agcagaacac ccccatcggc gacggccccg tgctgctgcc cgacaaccac 660
tacctgagct accagtccaa actgagcaaa gaccccaacg agaagcgcga tcacatggtc 720
ctgctggagt tcgtgaccgc cgccgggatc actctcggca tggacgagct gtacaagtaa 780

<210> 28
<211> 259
<212> PRT
<213> Artificial

<220>
<223> Lyn-mYFP

<400> 28

```
Met Gly Cys Ile Lys Ser Lys Arg Lys Asp Asn Leu Asn Asp Asp Glu
1               5                   10                  15

Pro Val Ala Thr Met Val Ser Lys Gly Glu Glu Leu Phe Thr Gly Val
            20                  25                  30

Val Pro Ile Leu Val Glu Leu Asp Gly Asp Val Asn Gly His Lys Phe
        35                  40                  45

Ser Val Ser Gly Glu Gly Glu Gly Asp Ala Thr Tyr Gly Lys Leu Thr
    50                  55                  60

Leu Lys Phe Ile Cys Thr Thr Gly Lys Leu Pro Val Pro Trp Pro Thr
65                  70                  75                  80

Leu Val Thr Thr Phe Gly Tyr Gly Leu Gln Cys Phe Ala Arg Tyr Pro
                85                  90                  95

Asp His Met Lys Gln His Asp Phe Phe Lys Ser Ala Met Pro Glu Gly
            100                 105                 110

Tyr Val Gln Glu Arg Thr Ile Phe Phe Lys Asp Asp Gly Asn Tyr Lys
        115                 120                 125

Thr Arg Ala Glu Val Lys Phe Glu Gly Asp Thr Leu Val Asn Arg Ile
    130                 135                 140

Glu Leu Lys Gly Ile Asp Phe Lys Glu Asp Gly Asn Ile Leu Gly His
145                 150                 155                 160

Lys Leu Glu Tyr Asn Tyr Asn Ser His Asn Val Tyr Ile Met Ala Asp
                165                 170                 175

Lys Gln Lys Asn Gly Ile Lys Val Asn Phe Lys Ile Arg His Asn Ile
            180                 185                 190

Glu Asp Gly Ser Val Gln Leu Ala Asp His Tyr Gln Gln Asn Thr Pro
        195                 200                 205

Ile Gly Asp Gly Pro Val Leu Leu Pro Asp Asn His Tyr Leu Ser Tyr
    210                 215                 220

Gln Ser Ala Leu Ser Lys Asp Pro Asn Glu Lys Arg Asp His Met Val
```

```
225                 230                 235                 240

Leu Leu Glu Phe Val Thr Ala Ala Gly Ile Thr Leu Gly Met Asp Glu
                245                 250                 255

Leu Tyr Lys


<210>  29
<211>  786
<212>  DNA
<213>  Artificial

<220>
<223>  Mem-mCer

<400>  29
atgctgtgct gtatgagaag aaccaaacag gttgaaaaga atgatgagga ccaaccggtc      60

gccaccatgg tgagcaaggg cgaggagctg ttcaccgggg tggtgcccat cctggtcgag     120

ctggacggcg acgtaaacgg ccacaagttc agcgtgtccg gcgagggcga gggcgatgcc     180

acctacggca agctgaccct gaagttcatc tgcaccaccg gcaagctgcc cgtgccctgg     240

cccaccctcg tgaccaccct gacctggggc gtgcagtgct tcgcccgcta ccccgaccac     300

atgaagcagc acgacttctt caagtccgcc atgcccgaag gctacgtcca ggagcgcacc     360

atcttcttca aggacgacgg caactacaag acccgcgccg aggtgaagtt cgagggcgac     420

accctggtga accgcatcga gctgaagggc atcgacttca aggaggacgg caacatcctg     480

gggcacaagc tggagtacaa cgccatcagc gacaacgtct atatcaccgc cgacaagcag     540

aagaacggca tcaaggccaa cttcaagatc cgccacaaca tcgaggacgg cagcgtgcag     600

ctcgccgacc actaccagca gaacaccccc atcggcgacg gccccgtgct gctgcccgac     660

aaccactacc tgagcaccca gtccaaactg agcaaagacc ccaacgagaa gcgcgatcac     720

atggtcctgc tggagttcgt gaccgccgcc gggatcactc tcggcatgga cgagctgtac     780

aagtaa                                                                 786


<210>  30
<211>  261
<212>  PRT
<213>  Artificial

<220>
<223>  Mem-mCer

<400>  30

Met Leu Cys Cys Met Arg Arg Thr Lys Gln Val Glu Lys Asn Asp Glu
1               5                   10                  15
```

```
Asp Gln Pro Val Ala Thr Met Val Ser Lys Gly Glu Glu Leu Phe Thr
            20                  25                  30

Gly Val Val Pro Ile Leu Val Glu Leu Asp Gly Asp Val Asn Gly His
        35                  40                  45

Lys Phe Ser Val Ser Gly Glu Gly Glu Gly Asp Ala Thr Tyr Gly Lys
    50                  55                  60

Leu Thr Leu Lys Phe Ile Cys Thr Thr Gly Lys Leu Pro Val Pro Trp
65                  70                  75                  80

Pro Thr Leu Val Thr Thr Leu Thr Trp Gly Val Gln Cys Phe Ala Arg
                85                  90                  95

Tyr Pro Asp His Met Lys Gln His Asp Phe Phe Lys Ser Ala Met Pro
            100                 105                 110

Glu Gly Tyr Val Gln Glu Arg Thr Ile Phe Phe Lys Asp Asp Gly Asn
        115                 120                 125

Tyr Lys Thr Arg Ala Glu Val Lys Phe Glu Gly Asp Thr Leu Val Asn
    130                 135                 140

Arg Ile Glu Leu Lys Gly Ile Asp Phe Lys Glu Asp Gly Asn Ile Leu
145                 150                 155                 160

Gly His Lys Leu Glu Tyr Asn Ala Ile Ser Asp Asn Val Tyr Ile Thr
                165                 170                 175

Ala Asp Lys Gln Lys Asn Gly Ile Lys Ala Asn Phe Lys Ile Arg His
            180                 185                 190

Asn Ile Glu Asp Gly Ser Val Gln Leu Ala Asp His Tyr Gln Gln Asn
        195                 200                 205

Thr Pro Ile Gly Asp Gly Pro Val Leu Leu Pro Asp Asn His Tyr Leu
    210                 215                 220

Ser Thr Gln Ser Ala Leu Ser Lys Asp Pro Asn Glu Lys Arg Asp His
225                 230                 235                 240

Met Val Leu Leu Glu Phe Val Thr Ala Ala Gly Ile Thr Leu Gly Met
                245                 250                 255

Asp Glu Leu Tyr Lys
            260
```

<210> 31
<211> 786
<212> DNA
<213> Artificial

<220>
<223> Mem-mYFP

<400> 31
atgctgtgct gtatgagaag aaccaaacag gttgaaaaga atgatgagga ccaaccggtc 60

gccaccatgg tgagcaaggg cgaggagctg ttcaccgggg tggtgcccat cctggtcgag 120

ctggacggcg acgtaaacgg ccacaagttc agcgtgtccg gcgagggcga gggcgatgcc 180

acctacggca agctgaccct gaagttcatc tgcaccaccg gcaagctgcc cgtgccctgg 240

cccaccctcg tgaccacctt cggctacggc ctgcagtgct tcgcccgcta ccccgaccac 300

atgaagcagc acgacttctt caagtccgcc atgcccgaag gctacgtcca ggagcgcacc 360

atcttcttca aggacgacgg caactacaag acccgcgccg aggtgaagtt cgagggcgac 420

accctggtga accgcatcga gctgaagggc atcgacttca aggaggacgg caacatcctg 480

gggcacaagc tggagtacaa ctacaacagc cacaacgtct atatcatggc cgacaagcag 540

aagaacggca tcaaggtgaa cttcaagatc cgccacaaca tcgaggacgg cagcgtgcag 600

ctcgccgacc actaccagca gaacaccccc atcggcgacg gccccgtgct gctgcccgac 660

aaccactacc tgagctacca gtccaaactg agcaaagacc ccaacgagaa gcgcgatcac 720

atggtcctgc tggagttcgt gaccgccgcc gggatcactc tcggcatgga cgagctgtac 780

aagtaa 786

<210> 32
<211> 261
<212> PRT
<213> Artificial

<220>
<223> Mem-mYFP

<400> 32

Met Leu Cys Cys Met Arg Arg Thr Lys Gln Val Glu Lys Asn Asp Glu
1               5                   10                  15

Asp Gln Pro Val Ala Thr Met Val Ser Lys Gly Glu Glu Leu Phe Thr
            20                  25                  30

Gly Val Val Pro Ile Leu Val Glu Leu Asp Gly Asp Val Asn Gly His
        35                  40                  45

Lys Phe Ser Val Ser Gly Glu Gly Glu Gly Asp Ala Thr Tyr Gly Lys
    50                  55                  60

```
Leu Thr Leu Lys Phe Ile Cys Thr Thr Gly Lys Leu Pro Val Pro Trp
65                  70                  75                  80

Pro Thr Leu Val Thr Thr Phe Gly Tyr Gly Leu Gln Cys Phe Ala Arg
                85                  90                  95

Tyr Pro Asp His Met Lys Gln His Asp Phe Phe Lys Ser Ala Met Pro
            100                 105                 110

Glu Gly Tyr Val Gln Glu Arg Thr Ile Phe Phe Lys Asp Asp Gly Asn
        115                 120                 125

Tyr Lys Thr Arg Ala Glu Val Lys Phe Glu Gly Asp Thr Leu Val Asn
    130                 135                 140

Arg Ile Glu Leu Lys Gly Ile Asp Phe Lys Glu Asp Gly Asn Ile Leu
145                 150                 155                 160

Gly His Lys Leu Glu Tyr Asn Tyr Asn Ser His Asn Val Tyr Ile Met
                165                 170                 175

Ala Asp Lys Gln Lys Asn Gly Ile Lys Val Asn Phe Lys Ile Arg His
            180                 185                 190

Asn Ile Glu Asp Gly Ser Val Gln Leu Ala Asp His Tyr Gln Gln Asn
        195                 200                 205

Thr Pro Ile Gly Asp Gly Pro Val Leu Leu Pro Asp Asn His Tyr Leu
    210                 215                 220

Ser Tyr Gln Ser Ala Leu Ser Lys Asp Pro Asn Glu Lys Arg Asp His
225                 230                 235                 240

Met Val Leu Leu Glu Phe Val Thr Ala Ala Gly Ile Thr Leu Gly Met
                245                 250                 255

Asp Glu Leu Tyr Lys
            260


<210>  33
<211>  105
<212>  DNA
<213>  Artificial

<220>
<223>  linker signal peptide-fluorophore for constructs 3 and 4

<400>  33
```

atgaacactc aaatcctggt tttcgccctt gtggcagtca ttcccacaaa tgcagacaaa 60

atttgtcttc cgcgggcccg ggatccaccg gtcgccacca tggtg 105

<210> 34
<211> 35
<212> PRT
<213> Artificial

<220>
<223> linker signal peptide-fluorophore for constructs 3 and 4

<400> 34

Met Asn Thr Gln Ile Leu Val Phe Ala Leu Val Ala Val Ile Pro Thr
1 5 10 15

Asn Ala Asp Lys Ile Cys Leu Pro Arg Ala Arg Asp Pro Pro Val Ala
20 25 30

Thr Met Val
35

<210> 35
<211> 234
<212> DNA
<213> Artificial

<220>
<223> linker fluorophore-HA TMD/CT for constructs 3, 4

<400> 35
gacgagctgt acaaggctag tataaggaac aatacttatg atcacagcaa atacagagaa 60

gaagcgatgc aaaatagaat acaaattgac ccagtcaaat tgagtagtgg ctacaaagat 120

gtgatacttt ggtttagctt cggggcatca tgctttttgc ttcttgccat tgcaatgggc 180

cttgttttca tatgtgtgaa gaacggaaac atgcggtgca ctatttgtat ataa 234

<210> 36
<211> 77
<212> PRT
<213> Artificial

<220>
<223> linker fluorophore-HA TMD/CT for constructs 3, 4

<400> 36

Asp Glu Leu Tyr Lys Ala Ser Ile Arg Asn Asn Thr Tyr Asp His Ser
1 5 10 15

Lys Tyr Arg Glu Glu Ala Met Gln Asn Arg Ile Gln Ile Asp Pro Val
20 25 30

```
Lys Leu Ser Ser Gly Tyr Lys Asp Val Ile Leu Trp Phe Ser Phe Gly
        35                  40                  45

Ala Ser Cys Phe Leu Leu Leu Ala Ile Ala Met Gly Leu Val Phe Ile
    50                  55                  60

Cys Val Lys Asn Gly Asn Met Arg Cys Thr Ile Cys Ile
65                  70                  75


<210>  37
<211>  79
<212>  DNA
<213>  Artificial

<220>
<223>  oligonucleotide for the generation of 1-6

<400>  37
gatctcatga acactcaaat cctggttttc gcccttgtgg cagtcattcc cacaaatgca      60

gacaaaattt gtcttccgc                                                   79


<210>  38
<211>  73
<212>  DNA
<213>  Artificial

<220>
<223>  antisense of 19

<400>  38
ggaagacaaa ttttgtctgc atttgtggga atgactgcca caagggcgaa aaccaggatt      60

tgagtgttca tga                                                         73


<210>  39
<211>  32
<212>  DNA
<213>  Artificial

<220>
<223>  forward primer for the generation of 3-6

<400>  39
ggctgtacaa ggctagtata aggaacaata ct                                    32


<210>  40
<211>  32
<212>  DNA
<213>  Artificial

<220>
<223>  reverse primer for 3, 4

<400>  40
gcgcggccgc ttatatacaa atagtgcacc gc                                    32
```

<210> 41
<211> 32
<212> DNA
<213> Artificial

<220>
<223> reverse primer for 5, 6

<400> 41
gcgcggccgc ttatatagaa atagtggacc gc 32

<210> 42
<211> 122
<212> DNA
<213> Artificial

<220>
<223> oligonucleotide GPI[DAF], for constructs 7, 8

<400> 42
gtacaagcca aataaaggaa gtggaaccac ttcaggtact acccgtcttc tatctgggca 60

cacgtgtttc acgttgacag gtttgcttgg gacgctagta accatgggct tgctgactta 120

gc 122

<210> 43
<211> 122
<212> DNA
<213> Artificial

<220>
<223> antisense of 24

<400> 43
ggccgctaag tcagcaagcc catggttact agcgtcccaa gcaaacctgt caacgtgaaa 60

cacgtgtgcc cagatagaag acgggtagta cctgaagtgg ttccacttcc tttatttggc 120

tt 122

<210> 44
<211> 72
<212> DNA
<213> Artificial

<220>
<223> linker for constructs 9, 10

<400> 44
tgcactattt gtatactccg gcctgaagct ccgcgggccc gggatccacc ggtcgccacc 60

atggtgagca ag 72

<210> 45
<211> 24
<212> PRT
<213> Artificial

<220>
<223>  linker for constructs 9, 10

<400>  45

Cys Thr Ile Cys Ile Leu Arg Pro Glu Ala Pro Arg Ala Arg Asp Pro
1               5                   10                  15

Pro Val Ala Thr Met Val Ser Lys
            20

<210>  46
<211>  75
<212>  DNA
<213>  Artificial

<220>
<223>  linker for Lyn-mYFP/-mCer, constructs 13, 14

<400>  46
ccgcggacca tgggatgtat taaatcaaaa aggaaagaca atctcaatga cgatgaaccg      60

gtcgccacca tggtg                                                        75

<210>  47
<211>  22
<212>  PRT
<213>  Artificial

<220>
<223>  linker for Lyn-mYFP/-mCer, constructs 13, 14

<400>  47

Met Gly Cys Ile Lys Ser Lys Arg Lys Asp Asn Leu Asn Asp Asp Glu
1               5                   10                  15

Pro Val Ala Thr Met Val
            20

<210>  48
<211>  75
<212>  DNA
<213>  Artificial

<220>
<223>  linker for Mem-mYFP/-mCer, 15, 16

<400>  48
accatgctgt gctgtatgag aagaaccaaa caggttgaaa agaatgatga ggaccaaccg      60

gtcgccacca tggtg                                                        75

<210>  49
<211>  24
<212>  PRT

<213> Artificial

<220>
<223> linker for Mem-mYFP/-mCer, 15, 16

<400> 49

Met Leu Cys Cys Met Arg Arg Thr Lys Gln Val Glu Lys Asn Asp Glu
1 5 10 15

Asp Gln Pro Val Ala Thr Met Val
20

<210> 50
<211> 33
<212> DNA
<213> Artificial

<220>
<223> primer for A206K mutation in YFP to generate mYFP, 10, 12, 14, 16

<400> 50
ggggtctttg ctcagtttgg actggtagct cag 33

<210> 51
<211> 33
<212> DNA
<213> Artificial

<220>
<223> primer for A206K mutation in YFP to generate mYFP, 10, 12, 14, 16

<400> 51
ctgagctacc agtccaaact gagcaaagac ccc 33

<210> 52
<211> 34
<212> DNA
<213> Artificial

<220>
<223> primer for generation of HA, 9-12

<400> 52
ctcagatctc atgaacactc aaatcctggt tttc 34

<210> 53
<211> 42
<212> DNA
<213> Artificial

<220>
<223> reverse primer for HA, 9, 10

<400> 53
gacccgcgga gcttcaggcc ggagtataca aatagtgcac cg 42

<210> 54
<211> 42
<212> DNA
<213> Artificial

<220>
<223> reverse primer for M3-HA, 11, 12

<400> 54
gacccgcgga gcttcaggcc ggagtataga aatagtggac cg 42

<210> 55
<211> 72
<212> DNA
<213> Artificial

<220>
<223> Mem oligonucleotide, 15, 16

<400> 55
gggccgcgga tgctgtgctg tatgagaaga accaaacagg ttgaaaagaa tgatgaggac 60

caaccggtca aa 72

<210> 56
<211> 72
<212> DNA
<213> Artificial

<220>
<223> antisense of 34

<400> 56
tttgaccggt tggtcctcat cattcttttc aacctgtttg gttcttctca tacagcacag 60

catccgcggc cc 72

<210> 57
<211> 69
<212> DNA
<213> Artificial

<220>
<223> Lyn oligonucleotide, 13, 14

<400> 57
gggccgcgga ccatgggatg tattaaatca aaaaggaaag acaatctcaa tgacgatgaa 60

ccggtcccc 69

<210> 58
<211> 69
<212> DNA
<213> Artificial

<220>
<223> antisense of 36

<400> 58

ggggaccggt tcatcgtcat tgagattgtc tttccttttt gatttaatac atcccatggt 60

ccgcggccc 69

<210> 59
<211> 33
<212> DNA
<213> Artificial

<220>
<223> linker CFP-M1 in construct 1

<400> 59
tacaagtccg gactcagatc tcgagctatg agt 33

<210> 60
<211> 11
<212> PRT
<213> Artificial

<220>
<223> linker CFP-M1 in construct 1

<400> 60

Tyr Lys Ser Gly Leu Arg Ser Arg Ala Met Ser
1 5 10

<210> 61
<211> 11
<212> PRT
<213> Artificial

<220>
<223> nuclear export signal derived from Rev

<400> 61

Leu Gln Leu Pro Pro Leu Glu Arg Leu Thr Leu
1 5 10

<210> 62
<211> 42
<212> DNA
<213> Artificial

<220>
<223> NES in construct 1

<400> 62
ttcaagcttc agctaccacc gcttgagaga cttactcttt ga 42

<210> 63
<211> 13
<212> PRT
<213> Artificial

<220>

<223> NES in construct 1

<400> 63

Phe Lys Leu Gln Leu Pro Pro Leu Glu Arg Leu Thr Leu
1 5 10

<210> 64
<211> 33
<212> DNA
<213> Artificial

<220>
<223> primer for NES construction

<400> 64
gcttacctcg agctatgagt cttctaaccg agg 33

<210> 65
<211> 61
<212> DNA
<213> Artificial

<220>
<223> reverse primer for NES construction

<400> 65
gtctcaggta cctcaaagag taagtctctc aagcggtggt agctgaagct tgaatcgttg 60

c 61

<210> 66
<211> 30
<212> DNA
<213> Artificial

<220>
<223> Myristoylation signal in construct 2

<400> 66
atgggatcaa tcaaatcaaa gatgagtctt 30

<210> 67
<211> 10
<212> PRT
<213> Artificial

<220>
<223> Myristoylation signal in construct 2

<400> 67

Met Gly Ser Ile Lys Ser Lys Met Ser Leu
1 5 10

<210> 68
<211> 45
<212> DNA

<213> Artificial

<220>
<223> linker M1-CFP in construct 2

<400> 68
ttcaagccgc gggcccggga tccaccggtc gccaccatgg tgagc 45

<210> 69
<211> 15
<212> PRT
<213> Artificial

<220>
<223> linker M1-CFP in construct 2

<400> 69

Phe Lys Pro Arg Ala Arg Asp Pro Pro Val Ala Thr Met Val Ser
1 5 10 15

<210> 70
<211> 48
<212> DNA
<213> Artificial

<220>
<223> primer for Myr-M1-CFP

<400> 70
gcttacctcg agatgggatc aatcaaatca aagatgagtc ttctaacc 48

<210> 71
<211> 33
<212> DNA
<213> Artificial

<220>
<223> reverse primer for Myr-M1-CFP

<400> 71
gtcttaccgc ggcttgaatc gttgcatctg cac 33